(19) Europäisches Patentamt
European Patent Office
Office européen des brevets

(11) EP 2 760 885 B1

(12) **EUROPEAN PATENT SPECIFICATION**

(45) Date of publication and mention of the grant of the patent:
**26.07.2017 Bulletin 2017/30**

(21) Application number: **12839164.6**

(22) Date of filing: **28.09.2012**

(51) Int Cl.:
***C07K 14/435*** *(2006.01)*

(86) International application number:
**PCT/US2012/057893**

(87) International publication number:
**WO 2013/089889 (20.06.2013 Gazette 2013/25)**

# (54) CHIMERIC POLYPEPTIDES HAVING BETA-GLUCOSIDASE ACTIVITY AND POLYNUCLEOTIDES ENCODING SAME

CHIMÄRE POLYPEPTIDE MIT BETA-GLUCOSIDASE-AKTIVITÄT UND DAFÜR KODIERENDE POLYNUKLEOTIDE

POLYPEPTIDES CHIMÉRIQUES PRÉSENTANT UNE ACTIVITÉ BÊTA-GLUCOSIDASE ET POLYNUCLÉOTIDES CODANT CEUX-CI

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.09.2011 US 201161541456 P**

(43) Date of publication of application:
**06.08.2014 Bulletin 2014/32**

(73) Proprietor: **Novozymes, Inc.**
**Davis, CA 95618 (US)**

(72) Inventors:
- **WOGULIS, Mark**
  **Davis, California 95616 (US)**
- **OSBORN, David**
  **Sacramento, California 95816 (US)**
- **HEU, Tia**
  **Elk Grove, California 95624 (US)**

(74) Representative: **Shenker, Paul Dhan**
**Novozymes A/S**
**Patents**
**Krogshoejvej 36**
**2880 Bagsværd (DK)**

(56) References cited:
- **DATABASE Geneseq [Online] 23 June 2011 (2011-06-23), "Aspergillus aculeatus GH3 beta-glucosidase protein SEQ ID:178.", XP002698459, retrieved from EBI accession no. GSP:AZI05014 Database accession no. AZI05014 -& DATABASE Geneseq [Online] 23 June 2011 (2011-06-23), "Aspergillus aculeatus GH3 beta-glucosidase gene SEQ ID:177.", XP002698460, retrieved from EBI accession no. GSN:AZI05013 Database accession no. AZI05013 & WO 2011/057140 A1 (NOVOZYMES INC [US]; MCBRAYER BRETT [US]; SHAGHASI TARANA [US]; VLASENK) 12 May 2011 (2011-05-12)**
- **KAWAGUCHI T ET AL: "Cloning and sequencing of the cDNA encoding beta-glucosidase 1 from Aspergillus aculeatus", GENE, ELSEVIER, AMSTERDAM, NL, vol. 173, no. 2, 16 September 1996 (1996-09-16), pages 287-288, XP004043232, ISSN: 0378-1119, DOI: 10.1016/0378-1119(96)00179-5**
- **DATABASE UniProt [Online] 3 May 2011 (2011-05-03), "SubName: Full=Extracellular beta-glucosidase/cellulase BGL3; Flags: Precursor;", XP002698461, retrieved from EBI accession no. UNIPROT:F1DGF3 Database accession no. F1DGF3 -& LIU DONGYANG ET AL: "Characterization of a thermostable [beta]-glucosidase from Aspergillus fumigatus Z5, and its functional expression in Pichia pastoris X33.", MICROBIAL CELL FACTORIES 2012, vol. 11, 2012, page 25, XP002698462, ISSN: 1475-2859**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 760 885 B1

- DATABASE Geneseq [Online] 20 October 2005 (2005-10-20), "Family GH3A beta-glucosidase SEQ ID NO 76.", XP002698463, retrieved from EBI accession no. GSP:AEB90591 Database accession no. AEB90591
- MARIMUTHU JEYA ET AL: "Characterization of Î-glucosidase from a strain of Penicillium purpurogenum KJS506", APPLIED MICROBIOLOGY AND BIOTECHNOLOGY, SPRINGER, BERLIN, DE, vol. 86, no. 5, 31 December 2009 (2009-12-31), pages 1473-1484, XP019799929, ISSN: 1432-0614
- KIM KEE-HONG ET AL: "A proteomics strategy to discover beta-glucosidases from Aspergillus fumigatus with two-dimensional page in-gel activity assay and tandem mass spectrometry", JOURNAL OF PROTEOME RESEARCH, vol. 6, no. 12, December 2007 (2007-12), pages 4749-4757, XP002698464, ISSN: 1535-3893

SEQUENCE LISTING

<110> Novozymes, Inc.
Wogulis, Mark
Osborn, David
Heu, Tia

<120> Chimeric polypeptides having beta-glucosidase activity and polynucleotides encoding same

<130> 12291-WO-PCT

<150> US 61/541,456
<151> 2011-09-30

<160> 18

<170> PatentIn version 3.5

<210> 1
<211> 2940
<212> DNA
<213> Aspergillus aculeatus

<400> 1

atgaagctca gttggcttga ggcggctgcc ttgacggctg cttcagtcgt cagcgctgta 60

tgttggcttt ttttgacctc ctcgctgctt ctagatattt ggtgtgaggc tgacaattcg 120

tgctctacag gatgaactgg cgttctctcc tcctttctac ccctctccgt gggccaatgg 180

ccagggagag tgggcggaag cctaccagcg tgcagtggcc attgtatccc agatgactct 240

ggatgagaag gtcaacctga ccaccggaac tgggtaatga cactacagct gctgcgagat 300

gaatcgcctg ctaacgagct tctagatggg agctggagaa gtgcgtcggt cagactggtg 360

gtgtcccaag gtaggacccc cggataaaaa catgtgttca gttggctaac cgacgatcgc 420

tgtctagact gaacatcggt ggcatgtgtc ttcaggacag tcccttggga attcgtgata 480

gtaagtcttg atacaactgg agctcggccg ttgacactct tgctcacaat gtgttctgca 540

ggtgactaca attcggcttt ccctgctggt gtcaacgttg ctgcgacatg ggacaagaac 600

cttgcttatc tacgtggtca ggctatgggt caagagttca gtgacaaagg aattgatgtt 660

caattgggac cggccgcggg tcccctcggc aggagccctg atggaggtcg caactgggaa 720

ggtttctctc cagacccggc tcttactggt gtgctctttg cggagacgat taagggtatt 780

caagacgctg gtgtcgtggc gacagccaag cattacattc tcaatgagca agagcatttc 840

cgccaggtcg cagaggctgc gggctacgga ttcaatatct ccgacacgat cagctctaac 900

gttgatgaca agaccattca tgaaatgtac ctctggccct tcgcggatgc cgttcgcgcc 960

ggcgttggcg ccatcatgtg ttcctacaac cagatcaaca acagctacgg ttgccagaac 1020

agttacactc tgaacaagct tctgaaggcc gagctcggct tccagggctt tgtgatgtct 1080

gactggggtg ctcaccacag tggtgttggt tctgctttgg ccggcttgga tatgtcaatg 1140

cctggcgata tcaccttcga ttctgccact agtttctggg gtaccaacct gaccattgct 1200
gtgctcaacg gtaccgtccc gcagtggcgc gttgacgaca tggctgtccg tatcatggct 1260
gcctactaca aggttggccg cgaccgcctg taccagccgc ctaacttcag ctcctggact 1320
cgcgatgaat acggcttcaa gtatttctac ccccaggaag ggccctatga gaaggtcaat 1380
cactttgtca atgtgcagcg caaccacagc gaggttattc gcaagttggg agcagacagt 1440
actgttctac tgaagaacaa caatgccctg ccgctgaccg gaaaggagcg caaagttgcg 1500
atcctgggtg aagatgctgg atccaactcg tacggtgcca atggctgctc tgaccgtggc 1560
tgtgacaacg gtactcttgc tatggcttgg ggtagcggca ctgccgaatt cccatatctc 1620
gtgacccctg agcaggctat tcaagccgag gtgctcaagc ataagggcag cgtctacgcc 1680
atcacggaca actgggcgct gagccaggtg gagaccctcg ctaaacaagc caggtaagtt 1740
ctgtcgtcca tacattggag gtatatgttt tcagtgaact gacaagtgtc tccgtagtgt 1800
ctctcttgta tttgtcaact cggacgcggg agagggctat atctccgtgg acggaaacga 1860
gggcgaccgc aacaacctca ccctctggaa gaacggcgac aacctcatca aggctgctgc 1920
aaacaactgc aacaacacca tcgttgtcat ccactccgtt ggacctgttt tggttgacga 1980
gtggtatgac caccccaacg ttactgccat cctctgggcg ggcttgccag gccaggagtc 2040
tggcaactcc ttggctgacg tgctctacgg ccgcgtcaac ccgggcgcca aatctccatt 2100
cacctggggc aagacgaggg aggcgtacgg ggattacctt gtccgtgagc tcaacaacgg 2160
caacggagct ccccaagatg atttctcgga aggtgttttc attgactacc gcggattcga 2220
caagcgcaat gagaccccga tctacgagtt cggacatggt ctgagctaca ccactttcaa 2280
ctactctggc cttcacatcc aggttctcaa cgcttcctcc aacgctcaag tagccactga 2340
gactggcgcc gctcccacct tcggacaagt cggcaatgcc tctgactacg tgtaccctga 2400
gggattgacc agaatcagca agttcatcta tccctggctt aattccacag acctgaaggc 2460
ctcatctggc gacccgtact atggagtcga caccgcggag cacgtgcccg agggtgctac 2520
tgatggctct ccgcagcccg ttctgcctgc cggtggtggc ttcggtggta acccgcgcct 2580
ctacgatgag ttgatccgtg tttcggtgac agtcaagaac actggtcgtg ttgccggtga 2640
tgctgtgcct caattggtaa ttagatcctc gtgcagtatt ggttccagat gctaaccgct 2700
tgctagtatg tttcccttgg tggacccaat gagcccaagg ttgtgttgcg caaattcgac 2760
cgcctcaccc tcaagccctc cgaggagacg gtgtggacga ctaccctgac ccgccgcgat 2820
ctgtctaact gggacgttgc ggctcaggac tgggtcatca cttcttaccc gaagaaggtc 2880
catgttggta gctcttcgcg tcagctgccc cttcacgcgg cgctcccgaa ggtgcaataa 2940

<210> 2

<211> 860
<212> PRT
<213> Aspergillus aculeatus

<400> 2

```
Met Lys Leu Ser Trp Leu Glu Ala Ala Ala Leu Thr Ala Ala Ser Val
1               5                   10                  15

Val Ser Ala Asp Glu Leu Ala Phe Ser Pro Pro Phe Tyr Pro Ser Pro
            20                  25                  30

Trp Ala Asn Gly Gln Gly Glu Trp Ala Glu Ala Tyr Gln Arg Ala Val
        35                  40                  45

Ala Ile Val Ser Gln Met Thr Leu Asp Glu Lys Val Asn Leu Thr Thr
    50                  55                  60

Gly Thr Gly Trp Glu Leu Glu Lys Cys Val Gly Gln Thr Gly Gly Val
65                  70                  75                  80

Pro Arg Leu Asn Ile Gly Gly Met Cys Leu Gln Asp Ser Pro Leu Gly
                85                  90                  95

Ile Arg Asp Ser Asp Tyr Asn Ser Ala Phe Pro Ala Gly Val Asn Val
            100                 105                 110

Ala Ala Thr Trp Asp Lys Asn Leu Ala Tyr Leu Arg Gly Gln Ala Met
        115                 120                 125

Gly Gln Glu Phe Ser Asp Lys Gly Ile Asp Val Gln Leu Gly Pro Ala
    130                 135                 140

Ala Gly Pro Leu Gly Arg Ser Pro Asp Gly Gly Arg Asn Trp Glu Gly
145                 150                 155                 160

Phe Ser Pro Asp Pro Ala Leu Thr Gly Val Leu Phe Ala Glu Thr Ile
                165                 170                 175

Lys Gly Ile Gln Asp Ala Gly Val Val Ala Thr Ala Lys His Tyr Ile
            180                 185                 190

Leu Asn Glu Gln Glu His Phe Arg Gln Val Ala Glu Ala Ala Gly Tyr
        195                 200                 205

Gly Phe Asn Ile Ser Asp Thr Ile Ser Ser Asn Val Asp Asp Lys Thr
    210                 215                 220

Ile His Glu Met Tyr Leu Trp Pro Phe Ala Asp Ala Val Arg Ala Gly
225                 230                 235                 240
```

```
Val Gly Ala Ile Met Cys Ser Tyr Asn Gln Ile Asn Asn Ser Tyr Gly
                245                 250                 255

Cys Gln Asn Ser Tyr Thr Leu Asn Lys Leu Leu Lys Ala Glu Leu Gly
            260                 265                 270

Phe Gln Gly Phe Val Met Ser Asp Trp Gly Ala His His Ser Gly Val
        275                 280                 285

Gly Ser Ala Leu Ala Gly Leu Asp Met Ser Met Pro Gly Asp Ile Thr
    290                 295                 300

Phe Asp Ser Ala Thr Ser Phe Trp Gly Thr Asn Leu Thr Ile Ala Val
305                 310                 315                 320

Leu Asn Gly Thr Val Pro Gln Trp Arg Val Asp Asp Met Ala Val Arg
                325                 330                 335

Ile Met Ala Ala Tyr Tyr Lys Val Gly Arg Asp Arg Leu Tyr Gln Pro
            340                 345                 350

Pro Asn Phe Ser Ser Trp Thr Arg Asp Glu Tyr Gly Phe Lys Tyr Phe
        355                 360                 365

Tyr Pro Gln Glu Gly Pro Tyr Glu Lys Val Asn His Phe Val Asn Val
    370                 375                 380

Gln Arg Asn His Ser Glu Val Ile Arg Lys Leu Gly Ala Asp Ser Thr
385                 390                 395                 400

Val Leu Leu Lys Asn Asn Asn Ala Leu Pro Leu Thr Gly Lys Glu Arg
                405                 410                 415

Lys Val Ala Ile Leu Gly Glu Asp Ala Gly Ser Asn Ser Tyr Gly Ala
            420                 425                 430

Asn Gly Cys Ser Asp Arg Gly Cys Asp Asn Gly Thr Leu Ala Met Ala
        435                 440                 445

Trp Gly Ser Gly Thr Ala Glu Phe Pro Tyr Leu Val Thr Pro Glu Gln
    450                 455                 460

Ala Ile Gln Ala Glu Val Leu Lys His Lys Gly Ser Val Tyr Ala Ile
465                 470                 475                 480

Thr Asp Asn Trp Ala Leu Ser Gln Val Glu Thr Leu Ala Lys Gln Ala
```

```
                485                 490                 495

Ser Val Ser Leu Val Phe Val Asn Ser Asp Ala Gly Glu Gly Tyr Ile
            500                 505                 510

Ser Val Asp Gly Asn Glu Gly Asp Arg Asn Asn Leu Thr Leu Trp Lys
        515                 520                 525

Asn Gly Asp Asn Leu Ile Lys Ala Ala Ala Asn Asn Cys Asn Asn Thr
    530                 535                 540

Ile Val Val Ile His Ser Val Gly Pro Val Leu Val Asp Glu Trp Tyr
545                 550                 555                 560

Asp His Pro Asn Val Thr Ala Ile Leu Trp Ala Gly Leu Pro Gly Gln
                565                 570                 575

Glu Ser Gly Asn Ser Leu Ala Asp Val Leu Tyr Gly Arg Val Asn Pro
            580                 585                 590

Gly Ala Lys Ser Pro Phe Thr Trp Gly Lys Thr Arg Glu Ala Tyr Gly
        595                 600                 605

Asp Tyr Leu Val Arg Glu Leu Asn Asn Gly Asn Gly Ala Pro Gln Asp
    610                 615                 620

Asp Phe Ser Glu Gly Val Phe Ile Asp Tyr Arg Gly Phe Asp Lys Arg
625                 630                 635                 640

Asn Glu Thr Pro Ile Tyr Glu Phe Gly His Gly Leu Ser Tyr Thr Thr
                645                 650                 655

Phe Asn Tyr Ser Gly Leu His Ile Gln Val Leu Asn Ala Ser Ser Asn
            660                 665                 670

Ala Gln Val Ala Thr Glu Thr Gly Ala Ala Pro Thr Phe Gly Gln Val
        675                 680                 685

Gly Asn Ala Ser Asp Tyr Val Tyr Pro Glu Gly Leu Thr Arg Ile Ser
    690                 695                 700

Lys Phe Ile Tyr Pro Trp Leu Asn Ser Thr Asp Leu Lys Ala Ser Ser
705                 710                 715                 720

Gly Asp Pro Tyr Tyr Gly Val Asp Thr Ala Glu His Val Pro Glu Gly
                725                 730                 735
```

```
Ala Thr Asp Gly Ser Pro Gln Pro Val Leu Pro Ala Gly Gly Gly Phe
            740                 745                 750

Gly Gly Asn Pro Arg Leu Tyr Asp Glu Leu Ile Arg Val Ser Val Thr
        755                 760                 765

Val Lys Asn Thr Gly Arg Val Ala Gly Asp Ala Val Pro Gln Leu Tyr
    770                 775                 780

Val Ser Leu Gly Gly Pro Asn Glu Pro Lys Val Val Leu Arg Lys Phe
785                 790                 795                 800

Asp Arg Leu Thr Leu Lys Pro Ser Glu Glu Thr Val Trp Thr Thr Thr
                805                 810                 815

Leu Thr Arg Arg Asp Leu Ser Asn Trp Asp Val Ala Ala Gln Asp Trp
            820                 825                 830

Val Ile Thr Ser Tyr Pro Lys Lys Val His Val Gly Ser Ser Ser Arg
        835                 840                 845

Gln Leu Pro Leu His Ala Ala Leu Pro Lys Val Gln
    850                 855                 860
```

<210> 3
<211> 3060
<212> DNA
<213> Aspergillus fumigatus

<400> 3

```
atgagattcg gttggctcga ggtggccgct ctgacggccg cttctgtagc caatgcccag      60
gtttgtgatg ctttcccgtc attgtttcgg atatagttga caatagtcat ggaaataatc     120
aggaattggc tttctctcca ccattctacc cttcgccttg ggctgatggc cagggagagt     180
gggcagatgc ccatcgacgc gccgtcgaga tcgtttctca gatgacactg gcggagaagg     240
ttaaccttac aacgggtact gggtgggttg cgactttttt gttgacagtg agctttcttc     300
actgaccatc tacacagatg ggaaatggac cgatgcgtcg gtcaaaccgg cagcgttccc     360
aggtaagctt gcaattctgc aacaacgtgc aagtgtagtt gctaaaacgc ggtggtgcag     420
acttggtatc aactggggtc tttgtggcca ggattcccct ttgggtatcc gtttctgtga     480
gctatacccg cggagtcttt cagtccttgt attatgtgct gatgattgtc tctgtatagc     540
tgacctcaac tccgccttcc ctgctggtac taatgtcgcc gcgacatggg acaagacact     600
cgcctacctt cgtggcaagg ccatgggtga ggaattcaac gacaagggcg tggacatttt     660
gctggggcct gctgctggtc ctctcggcaa atacccggac ggcggcagaa tctgggaagg     720
cttctctcct gatccggttc tcactggtgt acttttcgcc gaaactatca agggtatcca     780
```

agacgcgggt gtgattgcta ctgccaagca ttacattctg aatgaacagg agcatttccg 840
acaggttggc gaggcccagg gatatggtta caacatcacg gagacgatca gctccaacgt 900
ggatgacaag accatgcacg agttgtacct ttggtgagta gttgacactg caaatgagga 960
ccttgattga tttgactgac ctggaatgca ggccctttgc agatgctgtg cgcggtaaga 1020
ttttccgtag acttgacctc gcgacgaaga aatcgctgac gaaccatcgt agctggcgtt 1080
ggcgctgtca tgtgttccta caatcaaatc aacaacagct acggttgtca aaacagtcaa 1140
actctcaaca agctcctcaa ggctgagctg ggcttccaag gcttcgtcat gagtgactgg 1200
agcgctcacc acagcggtgt cggcgctgcc ctcgctgggt tggatatgtc gatgcctgga 1260
gacatttcct tcgacgacgg actctccttc tggggcacga acctaactgt cagtgttctt 1320
aacggcaccg ttccagcctg gcgtgtcgat gacatggctg ttcgtatcat gaccgcgtac 1380
tacaaggttg gtcgtgaccg tcttcgtatt ccccctaact tcagctcctg gacccgggat 1440
gagtacggct gggagcattc tgctgtctcc gagggagcct ggaccaaggt gaacgacttc 1500
gtcaatgtgc agcgcagtca ctctcagatc atccgtgaga ttggtgccgc tagtacagtg 1560
ctcttgaaga acacgggtgc tcttcctttg accggcaagg aggttaaagt gggtgttctc 1620
ggtgaagacg ctggttccaa cccgtggggt gctaacggct gccccgaccg cggctgtgat 1680
aacggcactc ttgctatggc ctggggtagt ggtactgcca acttccctta ccttgtcacc 1740
cccgagcagg ctatccagcg agaggtcatc agcaacggcg gcaatgtctt tgctgtgact 1800
gataacgggg ctctcagcca gatggcagat gttgcatctc aatccaggtg agtgcgggct 1860
cttagaaaaa gaacgttctc tgaatgaagt tttttaacca ttgcgaacag cgtgtctttg 1920
gtgtttgtca acgccgactc tggagagggt ttcatcagtg tcgacggcaa cgagggtgac 1980
cgcaaaaatc tcactctgtg gaagaacggc gaggccgtca ttgacactgt tgtcagccac 2040
tgcaacaaca cgattgtggt tattcacagt gttgggcccg tcttgatcga ccggtggtat 2100
gataacccca acgtcactgc catcatctgg gccggcttgc ccggtcagga gagtggcaac 2160
tccctggtcg acgtgctcta tggccgcgtc aaccccagcg ccaagacccc gttcacctgg 2220
ggcaagactc gggagtctta cggggctccc ttgctcaccg agcctaacaa tggcaatggt 2280
gctccccagg atgatttcaa cgagggcgtc ttcattgact accgtcactt tgacaagcgc 2340
aatgagaccc ccatttatga gtttggccat ggcttgagct acaccacctt tggttactct 2400
caccttcggg ttcaggccct caatagttcg agttcggcat atgtcccgac tagcggagag 2460
accaagcctg cgccaaccta tggtgagatc ggtagtgccg ccgactacct gtatcccgag 2520
ggtctcaaaa gaattaccaa gtttatttac ccttggctca actcgaccga cctcgaggat 2580
tcttctgacg acccgaacta cggctgggag gactcggagt acattcccga aggcgctagg 2640

gatgggtctc ctcaacccct cctgaaggct ggcggcgctc ctggtggtaa ccctaccctt 2700

tatcaggatc ttgttagggt gtcggccacc ataaccaaca ctggtaacgt cgccggttat 2760

gaagtccctc aattggtgag tgacccgcat gttccttgcg ttgcaatttg gctaactcgc 2820

ttctagtatg tttcactggg cggaccgaac gagcctcggg tcgttctgcg caagttcgac 2880

cgaatcttcc tggctcctgg ggagcaaaag gtttggacca cgactcttaa ccgtcgtgat 2940

ctcgccaatt gggatgtgga ggctcaggac tgggtcatca caaagtaccc caagaaagtg 3000

cacgtcggca gctcctcgcg taagctgcct ctgagagcgc ctctgccccg tgtctactag 3060

<210> 4
<211> 863
<212> PRT
<213> Aspergillus fumigatus

<400> 4

```
Met Arg Phe Gly Trp Leu Glu Val Ala Ala Leu Thr Ala Ala Ser Val
1               5                   10                  15

Ala Asn Ala Gln Glu Leu Ala Phe Ser Pro Pro Phe Tyr Pro Ser Pro
            20                  25                  30

Trp Ala Asp Gly Gln Gly Glu Trp Ala Asp Ala His Arg Arg Ala Val
        35                  40                  45

Glu Ile Val Ser Gln Met Thr Leu Ala Glu Lys Val Asn Leu Thr Thr
    50                  55                  60

Gly Thr Gly Trp Glu Met Asp Arg Cys Val Gly Gln Thr Gly Ser Val
65                  70                  75                  80

Pro Arg Leu Gly Ile Asn Trp Gly Leu Cys Gly Gln Asp Ser Pro Leu
                85                  90                  95

Gly Ile Arg Phe Ser Asp Leu Asn Ser Ala Phe Pro Ala Gly Thr Asn
            100                 105                 110

Val Ala Ala Thr Trp Asp Lys Thr Leu Ala Tyr Leu Arg Gly Lys Ala
        115                 120                 125

Met Gly Glu Glu Phe Asn Asp Lys Gly Val Asp Ile Leu Leu Gly Pro
    130                 135                 140

Ala Ala Gly Pro Leu Gly Lys Tyr Pro Asp Gly Gly Arg Ile Trp Glu
145                 150                 155                 160

Gly Phe Ser Pro Asp Pro Val Leu Thr Gly Val Leu Phe Ala Glu Thr
```

```
                165                 170                 175

Ile Lys Gly Ile Gln Asp Ala Gly Val Ile Ala Thr Ala Lys His Tyr
            180                 185                 190

Ile Leu Asn Glu Gln Glu His Phe Arg Gln Val Gly Glu Ala Gln Gly
        195                 200                 205

Tyr Gly Tyr Asn Ile Thr Glu Thr Ile Ser Ser Asn Val Asp Asp Lys
    210                 215                 220

Thr Met His Glu Leu Tyr Leu Trp Pro Phe Ala Asp Ala Val Arg Ala
225                 230                 235                 240

Gly Val Gly Ala Val Met Cys Ser Tyr Asn Gln Ile Asn Asn Ser Tyr
                245                 250                 255

Gly Cys Gln Asn Ser Gln Thr Leu Asn Lys Leu Leu Lys Ala Glu Leu
            260                 265                 270

Gly Phe Gln Gly Phe Val Met Ser Asp Trp Ser Ala His His Ser Gly
        275                 280                 285

Val Gly Ala Ala Leu Ala Gly Leu Asp Met Ser Met Pro Gly Asp Ile
    290                 295                 300

Ser Phe Asp Asp Gly Leu Ser Phe Trp Gly Thr Asn Leu Thr Val Ser
305                 310                 315                 320

Val Leu Asn Gly Thr Val Pro Ala Trp Arg Val Asp Asp Met Ala Val
                325                 330                 335

Arg Ile Met Thr Ala Tyr Tyr Lys Val Gly Arg Asp Arg Leu Arg Ile
            340                 345                 350

Pro Pro Asn Phe Ser Ser Trp Thr Arg Asp Glu Tyr Gly Trp Glu His
        355                 360                 365

Ser Ala Val Ser Glu Gly Ala Trp Thr Lys Val Asn Asp Phe Val Asn
    370                 375                 380

Val Gln Arg Ser His Ser Gln Ile Ile Arg Glu Ile Gly Ala Ala Ser
385                 390                 395                 400

Thr Val Leu Leu Lys Asn Thr Gly Ala Leu Pro Leu Thr Gly Lys Glu
                405                 410                 415
```

```
Val Lys Val Gly Val Leu Gly Glu Asp Ala Gly Ser Asn Pro Trp Gly
            420                 425                 430

Ala Asn Gly Cys Pro Asp Arg Gly Cys Asp Asn Gly Thr Leu Ala Met
        435                 440                 445

Ala Trp Gly Ser Gly Thr Ala Asn Phe Pro Tyr Leu Val Thr Pro Glu
    450                 455                 460

Gln Ala Ile Gln Arg Glu Val Ile Ser Asn Gly Gly Asn Val Phe Ala
465                 470                 475                 480

Val Thr Asp Asn Gly Ala Leu Ser Gln Met Ala Asp Val Ala Ser Gln
                485                 490                 495

Ser Ser Val Ser Leu Val Phe Val Asn Ala Asp Ser Gly Glu Gly Phe
            500                 505                 510

Ile Ser Val Asp Gly Asn Glu Gly Asp Arg Lys Asn Leu Thr Leu Trp
        515                 520                 525

Lys Asn Gly Glu Ala Val Ile Asp Thr Val Val Ser His Cys Asn Asn
    530                 535                 540

Thr Ile Val Val Ile His Ser Val Gly Pro Val Leu Ile Asp Arg Trp
545                 550                 555                 560

Tyr Asp Asn Pro Asn Val Thr Ala Ile Ile Trp Ala Gly Leu Pro Gly
                565                 570                 575

Gln Glu Ser Gly Asn Ser Leu Val Asp Val Leu Tyr Gly Arg Val Asn
            580                 585                 590

Pro Ser Ala Lys Thr Pro Phe Thr Trp Gly Lys Thr Arg Glu Ser Tyr
        595                 600                 605

Gly Ala Pro Leu Leu Thr Glu Pro Asn Asn Gly Asn Gly Ala Pro Gln
    610                 615                 620

Asp Asp Phe Asn Glu Gly Val Phe Ile Asp Tyr Arg His Phe Asp Lys
625                 630                 635                 640

Arg Asn Glu Thr Pro Ile Tyr Glu Phe Gly His Gly Leu Ser Tyr Thr
                645                 650                 655

Thr Phe Gly Tyr Ser His Leu Arg Val Gln Ala Leu Asn Ser Ser Ser
            660                 665                 670
```

Ser Ala Tyr Val Pro Thr Ser Gly Glu Thr Lys Pro Ala Pro Thr Tyr
675 680 685

Gly Glu Ile Gly Ser Ala Ala Asp Tyr Leu Tyr Pro Glu Gly Leu Lys
690 695 700

Arg Ile Thr Lys Phe Ile Tyr Pro Trp Leu Asn Ser Thr Asp Leu Glu
705 710 715 720

Asp Ser Ser Asp Asp Pro Asn Tyr Gly Trp Glu Asp Ser Glu Tyr Ile
725 730 735

Pro Glu Gly Ala Arg Asp Gly Ser Pro Gln Pro Leu Leu Lys Ala Gly
740 745 750

Gly Ala Pro Gly Gly Asn Pro Thr Leu Tyr Gln Asp Leu Val Arg Val
755 760 765

Ser Ala Thr Ile Thr Asn Thr Gly Asn Val Ala Gly Tyr Glu Val Pro
770 775 780

Gln Leu Tyr Val Ser Leu Gly Gly Pro Asn Glu Pro Arg Val Val Leu
785 790 795 800

Arg Lys Phe Asp Arg Ile Phe Leu Ala Pro Gly Glu Gln Lys Val Trp
805 810 815

Thr Thr Thr Leu Asn Arg Arg Asp Leu Ala Asn Trp Asp Val Glu Ala
820 825 830

Gln Asp Trp Val Ile Thr Lys Tyr Pro Lys Lys Val His Val Gly Ser
835 840 845

Ser Ser Arg Lys Leu Pro Leu Arg Ala Pro Leu Pro Arg Val Tyr
850 855 860

<210> 5
<211> 2939
<212> DNA
<213> Artificial Sequence

<220>
<223> Artificial DNA Sequence

<400> 5

```
atgaagctca gttggcttga ggcggctgcc ttgacggctg cttcagtcgt cagcgctgta        60
tgttggcttt ttttgacctc ctcgctgctt ctagatattt ggtgtgaggc tgacaattcg       120
tgctctacag gatgaactgg cgttctctcc tcctttctac ccctctccgt gggccaatgg       180
```

ccagggagag tgggcggaag cctaccagcg tgcagtggcc attgtatccc agatgactct 240
ggatgagaag gtcaacctga ccaccggaac tgggtaatga cactacagct gctgcgagat 300
gaatcgcctg ctaacgagct tctagatggg agctggagaa gtgcgtcggt cagactggtg 360
gtgtcccaag gtaggacccc cggataaaaa catgtgttca gttggctaac cgacgatcgc 420
tgtctagact gaacatcggt ggcatgtgtc ttcaggacag tcccttggga attcgtgata 480
gtaagtcttg atacaactgg agctcggccg ttgacactct tgctcacaat gtgttctgca 540
ggtgactaca attcggcttt ccctgctggt gtcaacgttg ctgcgacatg ggacaagaac 600
cttgcttatc tacgtggtca ggctatgggt caagagttca gtgacaaagg aattgatgtt 660
caattgggac cggccgcggg tcccctcggc aggagccctg atggaggtcg caactgggaa 720
ggtttctctc cagacccggc tcttactggt gtgctctttg cggagacgat taagggtatt 780
caagacgctg gtgtcgtggc gacagccaag cattacattc tcaatgagca agagcatttc 840
cgccaggtcg cagaggctgc gggctacgga ttcaatatct ccgacacgat cagctctaac 900
gttgatgaca agaccattca tgaaatgtac ctctggccct tcgcggatgc cgttcgcgcc 960
ggcgttggcg ccatcatgtg ttcctacaac cagatcaaca acagctacgg ttgccagaac 1020
agttacactc tgaacaagct tctgaaggcc gagctcggct tccagggctt tgtgatgtct 1080
gactggggtg ctcaccacag tggtgttggt tctgctttgg ccggcttgga tatgtcaatg 1140
cctggcgata tcaccttcga ttctgccact agtttctggg gtaccaacct gaccattgct 1200
gtgctcaacg gtaccgtccc gcagtggcgc gttgacgaca tggctgtccg tatcatggct 1260
gcctactaca aggttggccg cgaccgcctg taccagccgc ctaacttcag ctcctggact 1320
cgcgatgaat acggcttcaa gtatttctac ccccaggaag ggccctatga gaaggtcaat 1380
cactttgtca atgtgcagcg caaccacagc gaggttattc gcaagttggg agcagacagt 1440
actgttctac tgaagaacac gggtgctctt cctttgaccg gcaaggaggt taaagtgggt 1500
gttctcggtg aagacgctgg ttccaacccg tggggtgcta acggctgccc cgaccgcggc 1560
tgtgataacg gcactcttgc tatggcctgg ggtagtggta ctgccaactt cccttacctt 1620
gtcacccccg agcaggctat ccagcgagag gtcatcagca acggcggcaa tgtctttgct 1680
gtgactgata acggggctct cagccagatg gcagatgttg catctcaatc caggtgagtg 1740
cgggctctta gaaaaagaac gttctctgaa tgaagttttt taaccattgc gaacagcgtg 1800
tctttggtgt ttgtcaacgc cgactctgga gagggtttca tcagtgtcga cggcaacgag 1860
ggtgaccgca aaaatctcac tctgtggaag aacggcgagg ccgtcattga cactgttgtc 1920
agccactgca acaacacgat tgtggttatt cacagtgttg ggcccgtctt gatcgaccgg 1980
tggtatgata accccaacgt cactgccatc atctgggccg gcttgcccgg tcaggagagt 2040

```
ggcaactccc tggtcgacgt gctctatggc cgcgtcaacc cgggcgccaa atctccattc     2100
acctggggca agacgaggga ggcgtacggg gattaccttg tccgtgagct caacaacggc     2160
aacggagctc cccaagatga tttctcggaa ggtgttttca ttgactaccg cggattcgac     2220
aagcgcaatg agaccccgat ctacgagttc ggacatggtc tgagctacac cactttcaac     2280
tactctggcc ttcacatcca ggttctcaac gcttcctcca acgctcaagt agccactgag     2340
actggcgccg ctcccacctt cggacaagtc ggcaatgcct ctgactacgt gtaccctgag     2400
ggattgacca gaatcagcaa gttcatctat ccctggctta attccacaga cctgaaggcc     2460
tcatctggcg acccgtacta tggagtcgac accgcggagc acgtgcccga gggtgctact     2520
gatggctctc cgcagcccgt tctgcctgcc ggtggtggct tcggtggtaa cccgcgcctc     2580
tacgatgagt tgatccgtgt ttcggtgaca gtcaagaaca ctggtcgtgt tgccggtgat     2640
gctgtgcctc aattggtaat tagatcctcg tgcagtattg gttccagatg ctaaccgctt     2700
gctagtatgt ttcccttggt ggacccaatg agcccaaggt tgtgttgcgc aaattcgacc     2760
gcctcaccct caagccctcc gaggagacgg tgtggacgac taccctgacc cgccgcgatc     2820
tgtctaactg ggacgttgcg gctcaggact gggtcatcac ttcttacccg aagaaggtcc     2880
atgttggtag ctcttcgcgt cagctgcccc ttcacgcggc gctcccgaag gtgcaataa      2939
```

<210> 6
<211> 860
<212> PRT
<213> Artificial Sequence

<220>
<223> Artificial Amino Acid Sequence

<400> 6

```
Met Lys Leu Ser Trp Leu Glu Ala Ala Ala Leu Thr Ala Ala Ser Val
1               5                   10                  15

Val Ser Ala Asp Glu Leu Ala Phe Ser Pro Pro Phe Tyr Pro Ser Pro
            20                  25                  30

Trp Ala Asn Gly Gln Gly Glu Trp Ala Glu Ala Tyr Gln Arg Ala Val
        35                  40                  45

Ala Ile Val Ser Gln Met Thr Leu Asp Glu Lys Val Asn Leu Thr Thr
    50                  55                  60

Gly Thr Gly Trp Glu Leu Glu Lys Cys Val Gly Gln Thr Gly Gly Val
65                  70                  75                  80

Pro Arg Leu Asn Ile Gly Gly Met Cys Leu Gln Asp Ser Pro Leu Gly
                85                  90                  95
```

```
Ile Arg Asp Ser Asp Tyr Asn Ser Ala Phe Pro Ala Gly Val Asn Val
            100                 105                 110

Ala Ala Thr Trp Asp Lys Asn Leu Ala Tyr Leu Arg Gly Gln Ala Met
        115                 120                 125

Gly Gln Glu Phe Ser Asp Lys Gly Ile Asp Val Gln Leu Gly Pro Ala
    130                 135                 140

Ala Gly Pro Leu Gly Arg Ser Pro Asp Gly Gly Arg Asn Trp Glu Gly
145                 150                 155                 160

Phe Ser Pro Asp Pro Ala Leu Thr Gly Val Leu Phe Ala Glu Thr Ile
                165                 170                 175

Lys Gly Ile Gln Asp Ala Gly Val Val Ala Thr Ala Lys His Tyr Ile
            180                 185                 190

Leu Asn Glu Gln Glu His Phe Arg Gln Val Ala Glu Ala Ala Gly Tyr
        195                 200                 205

Gly Phe Asn Ile Ser Asp Thr Ile Ser Ser Asn Val Asp Asp Lys Thr
    210                 215                 220

Ile His Glu Met Tyr Leu Trp Pro Phe Ala Asp Ala Val Arg Ala Gly
225                 230                 235                 240

Val Gly Ala Ile Met Cys Ser Tyr Asn Gln Ile Asn Asn Ser Tyr Gly
                245                 250                 255

Cys Gln Asn Ser Tyr Thr Leu Asn Lys Leu Leu Lys Ala Glu Leu Gly
            260                 265                 270

Phe Gln Gly Phe Val Met Ser Asp Trp Gly Ala His His Ser Gly Val
        275                 280                 285

Gly Ser Ala Leu Ala Gly Leu Asp Met Ser Met Pro Gly Asp Ile Thr
    290                 295                 300

Phe Asp Ser Ala Thr Ser Phe Trp Gly Thr Asn Leu Thr Ile Ala Val
305                 310                 315                 320

Leu Asn Gly Thr Val Pro Gln Trp Arg Val Asp Asp Met Ala Val Arg
                325                 330                 335

Ile Met Ala Ala Tyr Tyr Lys Val Gly Arg Asp Arg Leu Tyr Gln Pro
```

```
            340                 345                 350

Pro Asn Phe Ser Ser Trp Thr Arg Asp Glu Tyr Gly Phe Lys Tyr Phe
        355                 360                 365

Tyr Pro Gln Glu Gly Pro Tyr Glu Lys Val Asn His Phe Val Asn Val
    370                 375                 380

Gln Arg Asn His Ser Glu Val Ile Arg Lys Leu Gly Ala Asp Ser Thr
385                 390                 395                 400

Val Leu Leu Lys Asn Thr Gly Ala Leu Pro Leu Thr Gly Lys Glu Val
                405                 410                 415

Lys Val Gly Val Leu Gly Glu Asp Ala Gly Ser Asn Pro Trp Gly Ala
            420                 425                 430

Asn Gly Cys Pro Asp Arg Gly Cys Asp Asn Gly Thr Leu Ala Met Ala
        435                 440                 445

Trp Gly Ser Gly Thr Ala Asn Phe Pro Tyr Leu Val Thr Pro Glu Gln
    450                 455                 460

Ala Ile Gln Arg Glu Val Ile Ser Asn Gly Gly Asn Val Phe Ala Val
465                 470                 475                 480

Thr Asp Asn Gly Ala Leu Ser Gln Met Ala Asp Val Ala Ser Gln Ser
                485                 490                 495

Ser Val Ser Leu Val Phe Val Asn Ala Asp Ser Gly Glu Gly Phe Ile
            500                 505                 510

Ser Val Asp Gly Asn Glu Gly Asp Arg Lys Asn Leu Thr Leu Trp Lys
        515                 520                 525

Asn Gly Glu Ala Val Ile Asp Thr Val Val Ser His Cys Asn Asn Thr
    530                 535                 540

Ile Val Val Ile His Ser Val Gly Pro Val Leu Ile Asp Arg Trp Tyr
545                 550                 555                 560

Asp Asn Pro Asn Val Thr Ala Ile Ile Trp Ala Gly Leu Pro Gly Gln
                565                 570                 575

Glu Ser Gly Asn Ser Leu Val Asp Val Leu Tyr Gly Arg Val Asn Pro
            580                 585                 590
```

```
Gly Ala Lys Ser Pro Phe Thr Trp Gly Lys Thr Arg Glu Ala Tyr Gly
        595                 600                 605

Asp Tyr Leu Val Arg Glu Leu Asn Asn Gly Asn Gly Ala Pro Gln Asp
    610                 615                 620

Asp Phe Ser Glu Gly Val Phe Ile Asp Tyr Arg Gly Phe Asp Lys Arg
625                 630                 635                 640

Asn Glu Thr Pro Ile Tyr Glu Phe Gly His Gly Leu Ser Tyr Thr Thr
                645                 650                 655

Phe Asn Tyr Ser Gly Leu His Ile Gln Val Leu Asn Ala Ser Ser Asn
            660                 665                 670

Ala Gln Val Ala Thr Glu Thr Gly Ala Ala Pro Thr Phe Gly Gln Val
        675                 680                 685

Gly Asn Ala Ser Asp Tyr Val Tyr Pro Glu Gly Leu Thr Arg Ile Ser
    690                 695                 700

Lys Phe Ile Tyr Pro Trp Leu Asn Ser Thr Asp Leu Lys Ala Ser Ser
705                 710                 715                 720

Gly Asp Pro Tyr Tyr Gly Val Asp Thr Ala Glu His Val Pro Glu Gly
                725                 730                 735

Ala Thr Asp Gly Ser Pro Gln Pro Val Leu Pro Ala Gly Gly Gly Phe
            740                 745                 750

Gly Gly Asn Pro Arg Leu Tyr Asp Glu Leu Ile Arg Val Ser Val Thr
        755                 760                 765

Val Lys Asn Thr Gly Arg Val Ala Gly Asp Ala Val Pro Gln Leu Tyr
    770                 775                 780

Val Ser Leu Gly Gly Pro Asn Glu Pro Lys Val Val Leu Arg Lys Phe
785                 790                 795                 800

Asp Arg Leu Thr Leu Lys Pro Ser Glu Glu Thr Val Trp Thr Thr Thr
                805                 810                 815

Leu Thr Arg Arg Asp Leu Ser Asn Trp Asp Val Ala Ala Gln Asp Trp
            820                 825                 830

Val Ile Thr Ser Tyr Pro Lys Lys Val His Val Gly Ser Ser Ser Arg
        835                 840                 845
```

Gln Leu Pro Leu His Ala Ala Leu Pro Lys Val Gln
850 855 860

<210> 7
<211> 2939
<212> DNA
<213> Artificial Sequence

<220>
<223> Artificial DNA Sequence

<400> 7

```
atgaagctca gttggcttga ggcggctgcc ttgacggctg cttcagtcgt cagcgctgta      60
tgttggcttt ttttgacctc ctcgctgctt ctagatattt ggtgtgaggc tgacaattcg     120
tgctctacag gatgaactgg cgttctctcc tcctttctac ccctctccgt gggccaatgg     180
ccagggagag tgggcggaag cctaccagcg tgcagtggcc attgtatccc agatgactct     240
ggatgagaag gtcaacctga ccaccggaac tgggtaatga cactacagct gctgcgagat     300
gaatcgcctg ctaacgagct tctagatggg agctggagaa gtgcgtcggt cagactggtg     360
gtgtcccaag gtaggacccc cggataaaaa catgtgttca gttggctaac cgacgatcgc     420
tgtctagact gaacatcggt ggcatgtgtc ttcaggacag tcccttggga attcgtgata     480
gtaagtcttg atacaactgg agctcggccg ttgacactct tgctcacaat gtgttctgca     540
ggtgactaca attcggcttt ccctgctggt gtcaacgttg ctgcgacatg ggacaagaac     600
cttgcttatc tacgtggtca ggctatgggt caagagttca gtgacaaagg aattgatgtt     660
caattgggac cggccgcggg tcccctcggc aggagccctg atggaggtcg caactgggaa     720
ggtttctctc cagacccggc tcttactggt gtgctctttg cggagacgat taagggtatt     780
caagacgctg gtgtcgtggc gacagccaag cattacattc tcaatgagca agagcatttc     840
cgccaggtcg cagaggctgc gggctacgga ttcaatatct ccgacacgat cagctctaac     900
gttgatgaca agaccattca tgaaatgtac ctctggccct tcgcggatgc cgttcgcgcc     960
ggcgttggcg ccatcatgtg ttcctacaac cagatcaaca acagctacgg ttgccagaac    1020
agttacactc tgaacaagct tctgaaggcc gagctcggct tccagggctt tgtgatgtct    1080
gactggggtg ctcaccacag tggtgttggt tctgctttgg ccggcttgga tatgtcaatg    1140
cctggcgata tcaccttcga ttctgccact agtttctggg gtaccaacct gaccattgct    1200
gtgctcaacg gtaccgtccc gcagtggcgc gttgacgaca tggctgtccg tatcatggct    1260
gcctactaca aggttggccg cgaccgcctg taccagccgc ctaacttcag ctcctggact    1320
cgcgatgaat acggcttcaa gtatttctac ccccaggaag ggccctatga gaaggtcaat    1380
cactttgtca atgtgcagcg caaccacagc gaggttattc gcaagttggg agcagacagt    1440
actgttctac tgaagaacac gggtgctctt cctttgaccg gcaaggaggt taaagtgggt    1500
```

gttctcggtg aagacgctgg ttccaacccg tggggtgcta acggctgccc cgaccgcggc 1560

tgtgataacg gcactcttgc tatggcctgg ggtagtggta ctgccgagtt cccttacctt 1620

gtcacccccg agcaggctat ccagcgagag gtcatcagca acggcggcaa tgtctttgct 1680

gtgactgata acggggctct cagccagatg gcagatgttg catctcaatc caggtgagtg 1740

cgggctctta gaaaaagaac gttctctgaa tgaagttttt taaccattgc gaacagcgtg 1800

tctttggtgt ttgtcaacgc cgactctgga gagggttaca tcagtgtcga cggcaacgag 1860

ggtgaccgca aaaatctcac tctgtggaag aacggcgagg ccgtcattga cactgttgtc 1920

agccactgca acaacacgat tgtggttatt cacagtgttg ggcccgtctt gatcgaccgg 1980

tggtatgata accccaacgt cactgccatc atctgggccg gcttgcccgg tcaggagagt 2040

ggcaactccc tggtcgacgt gctctatggc cgcgtcaacc cgggcgccaa atctccattc 2100

acctggggca agacgaggga ggcgtacggg gattaccttg tccgtgagct caacaacggc 2160

aacggagctc cccaagatga tttctcggaa ggtgttttca ttgactaccg cggattcgac 2220

aagcgcaatg agaccccgat ctacgagttc ggacatggtc tgagctacac cactttcaac 2280

tactctggcc ttcacatcca ggttctcaac gcttcctcca acgctcaagt agccactgag 2340

actggcgccg ctcccacctt cggacaagtc ggcaatgcct ctgactacgt gtaccctgag 2400

ggattgacca gaatcagcaa gttcatctat ccctggctta attccacaga cctgaaggcc 2460

tcatctggcg acccgtacta tggagtcgac accgcggagc acgtgcccga gggtgctact 2520

gatggctctc cgcagcccgt tctgcctgcc ggtggtggct tcggtggtaa cccgcgcctc 2580

tacgatgagt tgatccgtgt ttcggtgaca gtcaagaaca ctggtcgtgt tgccggtgat 2640

gctgtgcctc aattggtaat tagatcctcg tgcagtattg gttccagatg ctaaccgctt 2700

gctagtatgt ttcccttggt ggacccaatg agcccaaggt tgtgttgcgc aaattcgacc 2760

gcctcaccct caagccctcc gaggagacgg tgtggacgac taccctgacc cgccgcgatc 2820

tgtctaactg ggacgttgcg gctcaggact gggtcatcac ttcttacccg aagaaggtcc 2880

atgttggtag ctcttcgcgt cagctgcccc ttcacgcggc gctcccgaag gtgcaataa 2939

<210> 8
<211> 860
<212> PRT
<213> Artificial Sequence

<220>
<223> Artificial Amino Acid Sequence

<400> 8

Met Lys Leu Ser Trp Leu Glu Ala Ala Ala Leu Thr Ala Ala Ser Val
1 5 10 15

```
Val Ser Ala Asp Glu Leu Ala Phe Ser Pro Pro Phe Tyr Pro Ser Pro
            20                  25                  30

Trp Ala Asn Gly Gln Gly Glu Trp Ala Glu Ala Tyr Gln Arg Ala Val
        35                  40                  45

Ala Ile Val Ser Gln Met Thr Leu Asp Glu Lys Val Asn Leu Thr Thr
    50                  55                  60

Gly Thr Gly Trp Glu Leu Glu Lys Cys Val Gly Gln Thr Gly Gly Val
65                  70                  75                  80

Pro Arg Leu Asn Ile Gly Gly Met Cys Leu Gln Asp Ser Pro Leu Gly
                85                  90                  95

Ile Arg Asp Ser Asp Tyr Asn Ser Ala Phe Pro Ala Gly Val Asn Val
            100                 105                 110

Ala Ala Thr Trp Asp Lys Asn Leu Ala Tyr Leu Arg Gly Gln Ala Met
        115                 120                 125

Gly Gln Glu Phe Ser Asp Lys Gly Ile Asp Val Gln Leu Gly Pro Ala
    130                 135                 140

Ala Gly Pro Leu Gly Arg Ser Pro Asp Gly Gly Arg Asn Trp Glu Gly
145                 150                 155                 160

Phe Ser Pro Asp Pro Ala Leu Thr Gly Val Leu Phe Ala Glu Thr Ile
                165                 170                 175

Lys Gly Ile Gln Asp Ala Gly Val Val Ala Thr Ala Lys His Tyr Ile
            180                 185                 190

Leu Asn Glu Gln Glu His Phe Arg Gln Val Ala Glu Ala Ala Gly Tyr
        195                 200                 205

Gly Phe Asn Ile Ser Asp Thr Ile Ser Ser Asn Val Asp Asp Lys Thr
    210                 215                 220

Ile His Glu Met Tyr Leu Trp Pro Phe Ala Asp Ala Val Arg Ala Gly
225                 230                 235                 240

Val Gly Ala Ile Met Cys Ser Tyr Asn Gln Ile Asn Asn Ser Tyr Gly
                245                 250                 255

Cys Gln Asn Ser Tyr Thr Leu Asn Lys Leu Leu Lys Ala Glu Leu Gly
            260                 265                 270
```

```
Phe Gln Gly Phe Val Met Ser Asp Trp Gly Ala His His Ser Gly Val
        275                 280                 285

Gly Ser Ala Leu Ala Gly Leu Asp Met Ser Met Pro Gly Asp Ile Thr
    290                 295                 300

Phe Asp Ser Ala Thr Ser Phe Trp Gly Thr Asn Leu Thr Ile Ala Val
305                 310                 315                 320

Leu Asn Gly Thr Val Pro Gln Trp Arg Val Asp Asp Met Ala Val Arg
                325                 330                 335

Ile Met Ala Ala Tyr Tyr Lys Val Gly Arg Asp Arg Leu Tyr Gln Pro
            340                 345                 350

Pro Asn Phe Ser Ser Trp Thr Arg Asp Glu Tyr Gly Phe Lys Tyr Phe
        355                 360                 365

Tyr Pro Gln Glu Gly Pro Tyr Glu Lys Val Asn His Phe Val Asn Val
    370                 375                 380

Gln Arg Asn His Ser Glu Val Ile Arg Lys Leu Gly Ala Asp Ser Thr
385                 390                 395                 400

Val Leu Leu Lys Asn Thr Gly Ala Leu Pro Leu Thr Gly Lys Glu Val
                405                 410                 415

Lys Val Gly Val Leu Gly Glu Asp Ala Gly Ser Asn Pro Trp Gly Ala
            420                 425                 430

Asn Gly Cys Pro Asp Arg Gly Cys Asp Asn Gly Thr Leu Ala Met Ala
        435                 440                 445

Trp Gly Ser Gly Thr Ala Glu Phe Pro Tyr Leu Val Thr Pro Glu Gln
    450                 455                 460

Ala Ile Gln Arg Glu Val Ile Ser Asn Gly Gly Asn Val Phe Ala Val
465                 470                 475                 480

Thr Asp Asn Gly Ala Leu Ser Gln Met Ala Asp Val Ala Ser Gln Ser
                485                 490                 495

Ser Val Ser Leu Val Phe Val Asn Ala Asp Ser Gly Glu Gly Tyr Ile
            500                 505                 510

Ser Val Asp Gly Asn Glu Gly Asp Arg Lys Asn Leu Thr Leu Trp Lys
```

515 520 525

Asn Gly Glu Ala Val Ile Asp Thr Val Val Ser His Cys Asn Asn Thr
530 535 540

Ile Val Val Ile His Ser Val Gly Pro Val Leu Ile Asp Arg Trp Tyr
545 550 555 560

Asp Asn Pro Asn Val Thr Ala Ile Ile Trp Ala Gly Leu Pro Gly Gln
565 570 575

Glu Ser Gly Asn Ser Leu Val Asp Val Leu Tyr Gly Arg Val Asn Pro
580 585 590

Gly Ala Lys Ser Pro Phe Thr Trp Gly Lys Thr Arg Glu Ala Tyr Gly
595 600 605

Asp Tyr Leu Val Arg Glu Leu Asn Asn Gly Asn Gly Ala Pro Gln Asp
610 615 620

Asp Phe Ser Glu Gly Val Phe Ile Asp Tyr Arg Gly Phe Asp Lys Arg
625 630 635 640

Asn Glu Thr Pro Ile Tyr Glu Phe Gly His Gly Leu Ser Tyr Thr Thr
645 650 655

Phe Asn Tyr Ser Gly Leu His Ile Gln Val Leu Asn Ala Ser Ser Asn
660 665 670

Ala Gln Val Ala Thr Glu Thr Gly Ala Ala Pro Thr Phe Gly Gln Val
675 680 685

Gly Asn Ala Ser Asp Tyr Val Tyr Pro Glu Gly Leu Thr Arg Ile Ser
690 695 700

Lys Phe Ile Tyr Pro Trp Leu Asn Ser Thr Asp Leu Lys Ala Ser Ser
705 710 715 720

Gly Asp Pro Tyr Tyr Gly Val Asp Thr Ala Glu His Val Pro Glu Gly
725 730 735

Ala Thr Asp Gly Ser Pro Gln Pro Val Leu Pro Ala Gly Gly Gly Phe
740 745 750

Gly Gly Asn Pro Arg Leu Tyr Asp Glu Leu Ile Arg Val Ser Val Thr
755 760 765

```
Val Lys Asn Thr Gly Arg Val Ala Gly Asp Ala Val Pro Gln Leu Tyr
    770                 775                 780

Val Ser Leu Gly Gly Pro Asn Glu Pro Lys Val Val Leu Arg Lys Phe
785                 790                 795                 800

Asp Arg Leu Thr Leu Lys Pro Ser Glu Glu Thr Val Trp Thr Thr Thr
                805                 810                 815

Leu Thr Arg Arg Asp Leu Ser Asn Trp Asp Val Ala Ala Gln Asp Trp
            820                 825                 830

Val Ile Thr Ser Tyr Pro Lys Lys Val His Val Gly Ser Ser Ser Arg
        835                 840                 845

Gln Leu Pro Leu His Ala Ala Leu Pro Lys Val Gln
    850                 855                 860
```

<210> 9
<211> 11939
<212> DNA
<213> Artificial Sequence

<220>
<223> VECTOR SEQUENCE

<400> 9

gcgcccaata cgcaaaccgc ctctccccgc gcgttggccg attcattaat gcagctggca 60
cgacaggttt cccgactgga aagcgggcag tgagcgcaac gcaattaatg tgagttagct 120
cactcattag gcaccccagg ctttacactt tatgcttccg gctcgtatgt tgtgtggaat 180
tgtgagcgga taacaatttc acacaggaaa cagctatgac catgattacg ccaagcttgg 240
ctgcaggtcg accatgccgt ttatggttag cgcctcccaa aaaggaatgg ccgacttaga 300
gttacctctt gacgactttt tctttcctcc cccttacatt tcgttaccac aacacattcc 360
tatatccaaa ctccaggtac ataactagtc gaaatctctt taaatctagt caagaactag 420
atacttaacc ttcatactgc ttagagatag cctttgaagc tcttatttcg ctctttgtct 480
cactctcacc ttcctcctcc tcctccaatc ctttttgctc gggactagtc caagactagt 540
caacagttag tatgccgagc attcgcgata acgataacga tctccgaaag tccccagagt 600
actgtcacta tctcgaggca gttaaggacg gggagcttac gctgccggat ttcaagatag 660
taagccgacc cgactagctt taagcctagt tactgactag tccacaggac gacaatggcg 720
tgcctgatat ccatccatat gaagtctact gccgagtgaa gggatgcctc aagcgtacag 780
tgagtctact gctatcatag tttctgacta gttattaact agttgacagg ttccctctgc 840
caacagaaac atattggtca agcacttgaa ggacaagaac tcccacggca tggagtttac 900

attgcacaat ggtcctccca ctatgaagga actgatggag gccaaaggca agtcctatct 960
agattacttg gtgactagtc cctgactagt caagtagcat ggtatgaagg cttgtttgaa 1020
ggcactgttc tcccaacccc gactcctacc aagaagcgca agcgagctgc gtaagtttct 1080
gtgagtctaa ctagtgtatt agctaatata tagcagcacc aagtccaagg accacaatac 1140
taagggagtc gagaattcgt gagtttcttc tcccatttca actagtcctt gactagtcac 1200
taactacttt gcagcaacga gggtgaagct ggaaatgatc aggacaatgg cgagggcccg 1260
taagtacagc cattcaatgc agactagttg ctaactaatc tgtgactagt tcaagtggtc 1320
cgtacgccgt gcatacccct gtgactggta ggaatttgag caagcctgtc ttgccgcgcg 1380
atgagaaagg aaaggcaagt tacattcagc cccgtaccta ggatcagtgc taatttataa 1440
cctctagcca ctctttatgg cagatccgcc gtgagggtag caaggcagct aaatcagctg 1500
gtgagaaagg aaccataccc tgcaagacct gtcgcaacgc aaagggcaaa ggtaagctat 1560
ccaagctagt ttgggactag attctaacta gtctcagcac cgtgtggttc aaagccatat 1620
tgcgagtttt ggcgcttttt ctcatcgatt gacgaggcaa agggagcgag tatgcaacct 1680
cgtaagtcag actcagacaa agaccaacta gttattgacc agtcttctag aaggctctgt 1740
tgtggatctt gaggccctgg agagttcctc caacaatccg gagacaagca agtcctcgtc 1800
ggactagtca ctaactagac tctaactagt tgcagacatg gataatgcaa aagagacaag 1860
caatgaagaa agtggtaaga catttctcct ttgtggttct ggactagtct ttgactagtc 1920
acagtcttaa acaaggaaaa tgagcatgaa aatgaggagg aaaaggctgc tgagcccgag 1980
gaagtgcagg gtgatggcag acatggtagg ttaatacctt gttagttatt gctagtcact 2040
gactagtcaa taactagtct ctgaacacct tgcaatcact ccgtttgcgc agctgaacag 2100
tggtgaggat aatagtagta agttattcta gcttcagagt tataggagac tagatactaa 2160
ctagtattag ttgcaactaa cctggatctc agagactttg gcctcaatct agaatctatc 2220
tagttgtcaa ctagactgtg gtatcattgt cttttatttt cctagtcctg gaactagctt 2280
ctaactagtc tccctaatat gtggctgtct tgtttttttt ttttgtttcc ctacccggat 2340
atctagtccc cttctaggtt ctgttaacct ctcgggctct gatttagttt aacgcaaacc 2400
tgagattagt ttctaactag tctctaggtt ttctatccac ctttaattgt aataataaat 2460
acaagcaacg tttatacgtc aaaagcattt ataaactttt accctaaagt agcttgcttg 2520
tgtgtttagt ttataattag tctcttatta atttgatgta ggtaagcccg ccacaaatat 2580
atatttttac aagataccgt ggaaaaactt cgtgctatca caaaacagta tacaaaaaat 2640
aagcttgcat gcacaactgc acctcagaag aaaaggatga tcaataccgt acgggagatt 2700
gtacgaacag atggcccgag aggactattc cgagggtgtg ctatcaacac gtttatggcc 2760
gctgtcggat caggattata ctttggtctc tacgagagca ccaaatggtg gctaggctct 2820

gactcgatgg ataattgtgc cgtgttagag taggggggtgg tgggaaatct tgtatataat 2880

tgtgattgtt tgtacgatag tgaccgactg tacattagtg ataccccact ctaagaaaat 2940

agaccaatct ccagctgcac cttcagacac tccggtacaa attctcgtct atgttggaga 3000

ttgttgtgac tttgaaacat gacccttgac cctgattttg aatttgtcca tatatcgagg 3060

caggtgtctt attcgtacgg agagggtatc tgtcgtagac acatagtagt agtcatttcg 3120

agtgctgaat ttataaatcg catcatactt gcgacatact gccataaaag gagtacgtat 3180

ccaccactac ttattgcgca ccaacacgct tcaggtatgc atcccatccc tccttctggt 3240

actgcttcgc cgcctccacg ggatcaggag cagcataaat tccacggcca gcaataataa 3300

agtcggcacc gcgtccaaca gccgactcag gagtttggta ctgctgtccc agcttgtcac 3360

ccttcgagga gaggttgaca cctgtcgtga agacgacaaa atcttcctcc tccgaaggcg 3420

agctaacttc agactgaacc tcgccaaggt gacgtgtcga gacgaatccc atcacaaact 3480

tcttatactt ccgagcatag tcaacagaag aagtagtata ttgaccggta gccaaagatc 3540

ccttggaggt catctccgca aggatcaaaa ggcccctctc ggagccgtag gggaagtcct 3600

cggccgaagc agtctgggcc agagcctcga cgataccctc accgggcaga atactgcagt 3660

tgatgatgtg ggcccactca gagatacgca gagtgccgcc atggtactgc ttttggacgt 3720

tgtttccgat atcgatgaac ttgcgatctt cgaagatgag gaaattgtgc ttctctgcaa 3780

gggccttcag accggtgatg gtttcttcgc tgaaatcgga gaggatatcg atgtgagttt 3840

tgatcacggc aatgtacgga ccgagtcctg ttatataatc caccattaac cattactaga 3900

tcacatgtaa gtggcatccc cggtgcgcat acggtcagcc aaatccagca gctctttggt 3960

ggttgtcacg tcggcggaaa cggtgacatt ggttttcttg gcctcggcaa cctcgaagag 4020

cttctttacg agcgcattgg ggtgcttgct agcgcgtgcg ctgtaggtca attgcgactt 4080

ggaagacata aaaccgatgg aggggtagcg cggggttctg caaatattgt ataaatgagc 4140

acttagtggt tgaaactggc ttattagtag gttagtactt cgagttttca gtaattagac 4200

aaaataatca ggatgtccaa ctactaactc ttgatatatg gaatgaaatg tagatacaaa 4260

ctgcacgaca attgccgcga aaaattaaat tgaatctatg gaggggactg tcatgcacta 4320

gccacacgtt tctccgcctg tggggtgagc cacatgcctc attttgacca aacacatcga 4380

tgcagtcaca tgtagataag attagggcct atccttaggg taccgtccac gcggggatta 4440

tgcctggctt tttgcctgct tttgatatcc tttcagggac atagcaataa gcccaaccct 4500

atcggccata ataatgtcaa ttccagcagc ggcttgggtc tagaatattt aatcagctac 4560

cgagcaactt ctacatcaca gtttgaaagc actaaggatc aatatagaag cacttacctt 4620

cgcattttct ggtatattgt tctgagatcc ataggcctga ttaatgatta catacgcctc 4680

cgggtagtag accgagcagc cgagccagtt cagcgcctaa aacgccttat acaattaagc 4740
agttaaagaa gttagaatct acgcttaaaa agctacttaa aaatcgatct cgcagtcccg 4800
attcgcctat caaaaccagt ttaaatcaac tgattaaagg tgccgaacga gctataaatg 4860
atataacaat attaaagcat taattagagc aatatcaggc cgcgcacgaa aggcaactta 4920
aaaagcgaaa gcgctctact aaacagatta cttttgaaaa aggcacatca gtatttaaag 4980
cccgaatcct tattaagcgc cgaaatcagg cagataaagc catacaggca gatagacctc 5040
tacctattaa atcggcttct aggcgcgcaa tacatggtgt tttgatcatt ttaaattttt 5100
atatggcggg tggtgggcaa ctcgcttgcg cgggcaactc gcttaccgat tacgttaggg 5160
ctgatattta cgtaaaaatc gtcaagggat gcaagaccaa accgttaaat ttccggagtc 5220
aacagcatcc aagcccaagt ccttcacgga gaaaccccag cgtccacatc acgagcgaag 5280
gaccacctct aggcatcgga cgcaccatcc aattagaagc agcaaagcga aacagcccaa 5340
gaaaaaggtc ggcccgtcgg ccttttctgc aacgctgatc acgggcagcg atccaaccaa 5400
caccctccag agtgactagg ggcggaaatt tatcgggatt aatttccact caaccacaaa 5460
tcacagtcgt ccccggtatt gtcctgcaga cggcaattta acggcttctg cgaatcgctt 5520
ggattccccg cccctggccg tagagcttaa agtatgtccc ttgacaatgc gatgtatcac 5580
atgatataat tactagcaag ggaagccgtg cttggagttt ccaactcaat ttacctctat 5640
ccacacttct cttccttcct caatcctcta tatacacaac tggggatcca tgagattcgg 5700
ttggctcgag gtggccgctc tgacggccgc ttctgtagcc aatgcccagg tttgtgatgc 5760
tttcccgtca ttgtttcgga tatagttgac aatagtcatg gaaataatca ggaattggct 5820
ttctctccac cattctaccc ttcgccttgg gctgatggcc agggagagtg ggcagatgcc 5880
catcgacgcg ccgtcgagat cgtttctcag atgacactgg cggagaaggt taaccttaca 5940
acgggtactg ggtgggttgc gactttttttg ttgacagtga gctttcttca ctgaccatct 6000
acacagatgg gaaatggacc gatgcgtcgg tcaaaccggc agcgttccca ggtaagcttg 6060
caattctgca acaacgtgca agtgtagttg ctaaaacgcg gtggtgcaga cttggtatca 6120
actggggtct ttgtggccag gattcccctt tgggtatccg tttctgtgag ctatacccgc 6180
ggagtctttc agtccttgta ttatgtgctg atgattgtct ctgtatagct gacctcaact 6240
ccgccttccc tgctggtact aatgtcgccg cgacatggga caagacactc gcctaccttc 6300
gtggcaaggc catgggtgag gaattcaacg acaagggcgt ggacattttg ctggggcctg 6360
ctgctggtcc tctcggcaaa tacccggacg gcggcagaat ctgggaaggc ttctctcctg 6420
atccggttct cactggtgta cttttcgccg aaactatcaa gggtatccaa gacgcgggtg 6480
tgattgctac tgccaagcat tacattctga atgaacagga gcatttccga caggttggcg 6540
aggcccaggg atatggttac aacatcacgg agacgatcag ctccaacgtg gatgacaaga 6600

ccatgcacga gttgtacctt tggtgagtag ttgacactgc aaatgaggac cttgattgat 6660

ttgactgacc tggaatgcag gccctttgca gatgctgtgc gcggtaagat tttccgtaga 6720

cttgacctcg cgacgaagaa atcgctgacg aaccatcgta gctggcgttg gcgctgtcat 6780

gtgttcctac aatcaaatca acaacagcta cggttgtcaa aacagtcaaa ctctcaacaa 6840

gctcctcaag gctgagctgg gcttccaagg cttcgtcatg agtgactgga gcgctcacca 6900

cagcggtgtc ggcgctgccc tcgctgggtt ggatatgtcg atgcctggag acatttcctt 6960

cgacgacgga ctctccttct ggggcacgaa cctaactgtc agtgttctta acggcaccgt 7020

tccagcctgg cgtgtcgatg acatggctgt tcgtatcatg accgcgtact acaaggttgg 7080

tcgtgaccgt cttcgtattc cccctaactt cagctcctgg acccgggatg agtacggctg 7140

ggagcattct gctgtctccg agggagcctg gaccaaggtg aacgacttcg tcaatgtgca 7200

gcgcagtcac tctcagatca tccgtgagat tggtgccgct agtacagtgc tcttgaagaa 7260

cacgggtgct cttcctttga ccggcaagga ggttaaagtg ggtgttctcg gtgaagacgc 7320

tggttccaac ccgtggggtg ctaacggctg ccccgaccgc ggctgtgata acggcactct 7380

tgctatggcc tggggtagtg gtactgccaa cttcccttac cttgtcaccc ccgagcaggc 7440

tatccagcga gaggtcatca gcaacggcgg caatgtcttt gctgtgactg ataacggggc 7500

tctcagccag atggcagatg ttgcatctca atccaggtga gtgcgggctc ttagaaaaag 7560

aacgttctct gaatgaagtt ttttaaccat tgcgaacagc gtgtctttgg tgtttgtcaa 7620

cgccgactct ggagagggtt tcatcagtgt cgacggcaac gagggtgacc gcaaaaatct 7680

cactctgtgg aagaacggcg aggccgtcat tgacactgtt gtcagccact gcaacaacac 7740

gattgtggtt attcacagtg ttgggcccgt cttgatcgac cggtggtatg ataaccccaa 7800

cgtcactgcc atcatctggg ccggcttgcc cggtcaggag agtggcaact ccctggtcga 7860

cgtgctctat ggccgcgtca accccagcgc caagaccccg ttcacctggg gcaagactcg 7920

ggagtcttac ggggctccct tgctcaccga gcctaacaat ggcaatggtg ctccccagga 7980

tgatttcaac gagggcgtct tcattgacta ccgtcacttt gacaagcgca atgagacccc 8040

catttatgag tttggccatg gcttgagcta caccaccttt ggttactctc accttcgggt 8100

tcaggccctc aatagttcga gttcggcata tgtcccgact agcggagaga ccaagcctgc 8160

gccaacctat ggtgagatcg gtagtgccgc cgactacctg tatcccgagg gtctcaaaag 8220

aattaccaag tttatttacc cttggctcaa ctcgaccgac ctcgaggatt cttctgacga 8280

cccgaactac ggctgggagg actcggagta cattcccgaa ggcgctaggg atgggtctcc 8340

tcaacccctc ctgaaggctg gcggcgctcc tggtggtaac cctacccttt atcaggatct 8400

tgttagggtg tcggccacca taaccaacac tggtaacgtc gccggttatg aagtccctca 8460

attggtgagt gacccgcatg ttccttgcgt tgcaatttgg ctaactcgct tctagtatgt 8520
ttcactgggc ggaccgaacg agcctcgggt cgttctgcgc aagttcgacc gaatcttcct 8580
ggctcctggg gagcaaaagg tttggaccac gactcttaac cgtcgtgatc tcgccaattg 8640
ggatgtggag gctcaggact gggtcatcac aaagtacccc aagaaagtgc acgtcggcag 8700
ctcctcgcgt aagctgcctc tgagagcgcc tctgccccgt gtctactagg cggccgcgga 8760
ggccaccggt gaccgagctc gagcttaaga cctggtacat gtgccggcgc gcccgggaga 8820
tctagagggt gactgacacc tggcggtaga caatcaatcc atttcgctat agttaaagga 8880
tggggatgag ggcaattggt tatatgatca tgtatgtagt gggtgtgcat aatagtagtg 8940
aaatggaagc caagtcatgt gattgtaatc gaccgacgga attgaggata tccggaaata 9000
cagacaccgt gaaagccatg gtctttcctt cgtgtagaag accagacaga cagtccctga 9060
tttacccttg cacaaagcac tagaaaatta gcattccatc cttctctgct tgctctgctg 9120
atatcactgt cattcaatgc atagccatga gctcatctta gatccaagca cgtaattcca 9180
tagccgaggt ccacagtgga gcagcaacat tccccatcat tgctttcccc aggggcctcc 9240
caacgactaa atcaagagta tatctctacc gtccaataga tcgtcttcgc ttcaaaatct 9300
ttgacaattc caagagggtc cccatccatc aaacccagtt caataatagc cgagatgcat 9360
ggtggagtca attaggcagt attgctggaa tgtcgggcca gttggcccgg gtggtcattg 9420
gccgcctgtg atgccatctg ccactaaatc cgatcattga tccaccgccc acgaggcgcg 9480
tctttgcttt ttgcgcggcg tccaggttca actctctcgc tctagatatc gatgaattca 9540
ctggccgtcg ttttacaacg tcgtgactgg gaaaaccctg gcgttaccca acttaatcgc 9600
cttgcagcac atcccccttt cgccagctgg cgtaatagcg aagaggcccg caccgatcgc 9660
ccttcccaac agttgcgcag cctgaatggc gaatggcgcc tgatgcggta ttttctcctt 9720
acgcatctgt gcggtatttc acaccgcata tggtgcactc tcagtacaat ctgctctgat 9780
gccgcatagt taagccagcc ccgacacccg ccaacacccg ctgacgcgcc ctgacgggct 9840
tgtctgctcc cggcatccgc ttacagacaa gctgtgaccg tctccgggag ctgcatgtgt 9900
cagaggtttt caccgtcatc accgaaacgc gcgagacgaa agggcctcgt gatacgccta 9960
tttttatagg ttaatgtcat gataataatg gtttcttaga cgtcaggtgg cacttttcgg 10020
ggaaatgtgc gcggaacccc tatttgttta tttttctaaa tacattcaaa tatgtatccg 10080
ctcatgagac aataaccctg ataaatgctt caataatatt gaaaaaggaa gagtatgagt 10140
attcaacatt tccgtgtcgc ccttattccc ttttttgcgg cattttgcct tcctgttttt 10200
gctcacccag aaacgctggt gaaagtaaaa gatgctgaag atcagttggg tgcacgagtg 10260
ggttacatcg aactggatct caacagcggt aagatccttg agagttttcg ccccgaagaa 10320
cgttttccaa tgatgagcac ttttaaagtt ctgctatgtg gcgcggtatt atcccgtatt 10380

gacgccgggc aagagcaact cggtcgccgc atacactatt ctcagaatga cttggttgac 10440

gcgtcaccag tcacagaaaa gcatcttacg gatggcatga cagtaagaga attatgcagt 10500

gctgccataa ccatgagtga taacactgcg gccaacttac ttctgacaac gatcggagga 10560

ccgaaggagc taaccgcttt tttgcacaac atgggggatc atgtaactcg ccttgatcgt 10620

tgggaaccgg agctgaatga agccatacca aacgacgagc gtgacaccac gatgcctgta 10680

gcaatggcaa caacgttgcg caaactatta actggcgaac tacttactct agcttcccgg 10740

caacaattaa tagactggat ggaggcggat aaagttgcag gaccacttct gcgctcggcc 10800

cttccggctg gctggtttat tgctgataaa tctggagccg gtgagcgtgg gtctcgcggt 10860

atcattgcag cactggggcc agatggtaag ccctcccgta tcgtagttat ctacacgacg 10920

gggagtcagg caactatgga tgaacgaaat agacagatcg ctgagatagg tgcctcactg 10980

attaagcatt ggtaactgtc agaccaagtt tactcatata tactttagat tgatttaaaa 11040

cttcattttt aatttaaaag gatctaggtg aagatccttt ttgataatct catgaccaaa 11100

atcccttaac gtgagttttc gttccactga gcgtcagacc ccgtagaaaa gatcaaagga 11160

tcttcttgag atcctttttt tctgcgcgta atctgctgct tgcaaacaaa aaaaccaccg 11220

ctaccagcgg tggtttgttt gccggatcaa gagctaccaa ctctttttcc gaaggtaact 11280

ggcttcagca gagcgcagat accaaatact gtccttctag tgtagccgta gttaggccac 11340

cacttcaaga actctgtagc accgcctaca tacctcgctc tgctaatcct gttaccagtg 11400

gctgctgcca gtggcgataa gtcgtgtctt accgggttgg actcaagacg atagttaccg 11460

gataaggcgc agcggtcggg ctgaacgggg ggttcgtgca cacagcccag cttggagcga 11520

acgacctaca ccgaactgag atacctacag cgtgagctat gagaaagcgc cacgcttccc 11580

gaagggagaa aggcggacag gtatccggta agcggcaggg tcggaacagg agagcgcacg 11640

agggagcttc cagggggaaa cgcctggtat ctttatagtc ctgtcgggtt tcgccacctc 11700

tgacttgagc gtcgattttt gtgatgctcg tcaggggggc ggagcctatg gaaaaacgcc 11760

agcaacgcgg cctttttacg gttcctggcc ttttgctggc cttttgctca catgttcttt 11820

cctgcgttat cccctgattc tgtggataac cgtattaccg cctttgagtg agctgatacc 11880

gctcgccgca gccgaacgac cgagcgcagc gagtcagtga gcgaggaagc ggaagagag 11939

<210> 10
<211> 8134
<212> DNA
<213> Artificial Sequence

<220>
<223> Vector sequence

<400> 10

acctagatcc ttttaaatta aaaatgaagt tttaaatcaa tctaaagtat atatgagtaa 60
acttggtctg acagttacca atgcttaatc agtgaggcac ctatctcagc gatctgtcta 120
tttcgttcat ccatagttgc ctgactcccc gtcgtgtaga taactacgat acgggagcgc 180
ttaccatctg gccccagtgc tgcaatgata ccgcgagacc cacgctcacc ggctccagat 240
ttatcagcaa taaaccagcc agccggaagg gccgagcgca gaagtggtcc tgcaacttta 300
tccgcctcca ttcagtctat taattgttgc cgggaagcta gagtaagtag ttcgccagtt 360
aatagtttgc gcaacgttgt tggcattgct acaggcatcg tggtgtcact ctcgtcgttt 420
ggtatggctt cattcagctc cggttcccaa cgatcaaggc gagttacatg atcccccatg 480
ttgtgcaaaa aagcggttag ctccttcggt cctccgatcg ttgtcagaag taagttggcc 540
gcagtgttat cactcatggt tatggcagca ctgcataatt ctcttactgt catgccatcc 600
gtaagatgct tttctgtgac tggtgagtac tcaaccaagt cattctgaga atagtgtatg 660
cggcgaccga gttgctcttg cccggcgtca atacgggata atagtgtatc acatagcaga 720
actttaaaag tgctcatcat tggaaaacgt tcttcggggc gaaaactctc aaggatctta 780
ccgctgttga gatccagttc gatgtaaccc actcgtgcac ccaactgatc ttcagcatct 840
tttactttca ccagcgtttc tgggtgagca aaaacaggaa ggcaaaatgc cgcaaaaaag 900
ggaataaggg cgacacggaa atgttgaata ctcatactct tcctttttca atgggtaata 960
actgatataa ttaaattgaa gctctaattt gtgagtttag tatacatgca tttacttata 1020
atacagtttt ttagttttgc tggccgcatc ttctcaaata tgcttcccag cctgcttttc 1080
tgtaacgttc accctctacc ttagcatccc ttccctttgc aaatagtcct cttccaacaa 1140
taataatgtc agatcctgta gagaccacat catccacggt tctatactgt tgacccaatg 1200
cgtctccctt gtcatctaaa cccacaccgg gtgtcataat caaccaatcg taaccttcat 1260
ctcttccacc catgtctctt tgagcaataa agccgataac aaaatctttg tcgctcttcg 1320
caatgtcaac agtaccctta gtatattctc cagtagatag ggagcccttg catgacaatt 1380
ctgctaacat caaaaggcct ctaggttcct ttgttacttc ttctgccgcc tgcttcaaac 1440
cgctaacaat acctgggccc accacaccgt gtgcattcgt aatgtctgcc cattctgcta 1500
ttctgtatac acccgcagag tactgcaatt tgactgtatt accaatgtca gcaaattttc 1560
tgtcttcgaa gagtaaaaaa ttgtacttgg cggataatgc ctttagcggc ttaactgtgc 1620
cctccatgga aaaatcagtc aagatatcca catgtgtttt tagtaaacaa attttgggac 1680
ctaatgcttc aactaactcc agtaattcct tggtggtacg aacatccaat gaagcacaca 1740
agtttgtttg cttttcgtgc atgatattaa atagcttggc agcaacagga ctaggatgag 1800
tagcagcacg ttccttatat gtagctttcg acatgattta tcttcgtttc ctcgagcttc 1860
tcaatgatat tcgaatacgc tttgaggaga tacagcctaa tatccgacaa actgttttac 1920

agatttacga tcgtacttgt tacccatcat tgaattttga acatccgaac ctgggagttt 1980
tccctgaaac agatagtata tttgaacctg tataataata tatagtctag cgctttacgg 2040
aagacaatgt atgtatttcg gttcctggag aaactattgc atctattgca taggtaatct 2100
tgcacgtcgc atccccggtt cattttctgc gtttccatct tgcacttcaa tagcatatct 2160
ttgttaacga agcatctgtg cttcattttg tagaacaaaa atgcaacgcg agagcgctaa 2220
tttttcaaac aaagaatctg agctgcattt ttacagaaca gaaatgcaac gcgaaagcgc 2280
tattttacca acgaagaatc tgtgcttcat tttgtaaaa caaaaatgca acgcgacgag 2340
agcgctaatt tttcaaacaa agaatctgag ctgcattttt acagaacaga aatgcaacgc 2400
gagagcgcta ttttaccaac aaagaatcta tacttctttt ttgttctaca aaaatgcatc 2460
ccgagagcgc tatttttcta acaaagcatc ttagattact ttttttctcc tttgtgcgct 2520
ctataatgca gtctcttgat aactttttgc actgtaggtc cgttaaggtt agaagaaggc 2580
tactttggtg tctattttct cttccataaa aaaagcctga ctccacttcc cgcgtttact 2640
gattactagc gaagctgcgg gtgcattttt tcaagataaa ggcatccccg attatattct 2700
ataccgatgt ggattgcgca tactttgtga acagaaagtg atagcgttga tgattcttca 2760
ttggtcagaa aattatgaac ggtttcttct attttgtctc tatatactac gtataggaaa 2820
tgtttacatt ttcgtattgt tttcgattca ctctatgaat agttcttact acaatttttt 2880
tgtctaaaga gtaatactag agataaacat aaaaaatgta gaggtcgagt ttagatgcaa 2940
gttcaaggag cgaaaggtgg atgggtaggt tatataggga tatagcacag agatatatag 3000
caaagagata cttttgagca atgtttgtgg aagcggtatt cgcaatggga agctccaccc 3060
cggttgataa tcagaaaagc cccaaaaaca ggaagattgt ataagcaaat atttaaattg 3120
taaacgttaa tattttgtta aaattcgcgt taaatttttg ttaaatcagc tcatttttta 3180
acgaatagcc cgaaatcggc aaaatccctt ataaatcaaa agaatagacc gagatagggt 3240
tgagtgttgt tccagtttcc aacaagagtc cactattaaa gaacgtggac tccaacgtca 3300
aagggcgaaa aagggtctat cagggcgatg gcccactacg tgaaccatca ccctaatcaa 3360
gtttttttggg gtcgaggtgc cgtaaagcag taaatcggaa gggtaaacgg atgcccccat 3420
ttagagcttg acggggaaag ccggcgaacg tggcgagaaa ggaagggaag aaagcgaaag 3480
gagcgggggc tagggcggtg ggaagtgtag gggtcacgct gggcgtaacc accacacccg 3540
ccgcgcttaa tggggcgcta cagggcgcgt ggggatgatc cactagtacg gattagaagc 3600
cgccgagcgg gtgacagccc tccgaaggaa gactctcctc cgtgcgtcct cgtcttcacc 3660
ggtgcatgcc tgcaggagct cctagttaga aaaagacatt tttgctgtca gtcactgtca 3720
agagattctt ttgctggcat ttcttctaga agcaaaaaga gcgatgcgtc ttttccgctg 3780

aaccgttcca gcaaaaaaga ctaccaacgc aatatggatt gtcagaatcg tataaaagag 3840
aagcaaataa ctccttgtct tgtatcaatt gcattatagt atcttcttgt tagtgcaata 3900
tcatatagaa gtcatcgact agaattccat tcaagaatag ttcaaacaag aagattacaa 3960
actatcaatt tcatacacaa tataaacgat taaaagaaag cttcaccatg aagctcagtt 4020
ggcttgaggc ggctgccttg acggctgctt cagtcgtcag cgctgtatgt tggctttttt 4080
tgacctcctc gctgcttcta gatatttggt gtgaggctga caattcgtgc tctacaggat 4140
gaactggcgt tctctcctcc tttctacccc tctccgtggg ccaatggcca gggagagtgg 4200
gcggaagcct accagcgtgc agtggccatt gtatcccaga tgactctgga tgagaaggtc 4260
aacctgacca ccggaactgg gtaatgacac tacagctgct gcgagatgaa tcgcctgcta 4320
acgagcttct agatgggagc tggagaagtg cgtcggtcag actggtggtg tcccaaggta 4380
ggacccccgg ataaaaacat gtgttcagtt ggctaaccga cgatcgctgt ctagactgaa 4440
catcggtggc atgtgtcttc aggacagtcc cttgggaatt cgtgatagta agtcttgata 4500
caactggagc tcggccgttg acactcttgc tcacaatgtg ttctgcaggt gactacaatt 4560
cggctttccc tgctggtgtc aacgttgctg cgacatggga caagaacctt gcttatctac 4620
gtggtcaggc tatgggtcaa gagttcagtg acaaaggaat tgatgttcaa ttgggaccgg 4680
ccgcgggtcc cctcggcagg agccctgatg gaggtcgcaa ctgggaaggt ttctctccag 4740
acccggctct tactggtgtg ctctttgcgg agacgattaa gggtattcaa gacgctggtg 4800
tcgtggcgac agccaagcat tacattctca atgagcaaga gcatttccgc caggtcgcag 4860
aggctgcggg ctacggattc aatatctccg acacgatcag ctctaacgtt gatgacaaga 4920
ccattcatga aatgtacctc tggcccttcg cggatgccgt tcgcgccggc gttggcgcca 4980
tcatgtgttc ctacaaccag atcaacaaca gctacggttg ccagaacagt tacactctga 5040
acaagcttct gaaggccgag ctcggcttcc agggctttgt gatgtctgac tggggtgctc 5100
accacagtgg tgttggttct gctttggccg gcttggatat gtcaatgcct ggcgatatca 5160
ccttcgattc tgccactagt ttctggggta ccaacctgac cattgctgtg ctcaacggta 5220
ccgtcccgca gtggcgcgtt gacgacatgg ctgtccgtat catggctgcc tactacaagg 5280
ttggccgcga ccgcctgtac cagccgccta acttcagctc ctggactcgc gatgaatacg 5340
gcttcaagta tttctacccc caggaagggc cctatgagaa ggtcaatcac tttgtcaatg 5400
tgcagcgcaa ccacagcgag gttattcgca agttgggagc agacagtact gttctactga 5460
agaacaacaa tgccctgccg ctgaccggaa aggagcgcaa agttgcgatc ctgggtgaag 5520
atgctggatc caactcgtac ggtgccaatg gctgctctga ccgtggctgt gacaacggta 5580
ctcttgctat ggcttggggt agcggcactg ccgaattccc atatctcgtg acccctgagc 5640
aggctattca agccgaggtg ctcaagcata agggcagcgt ctacgccatc acggacaact 5700

gggcgctgag ccaggtggag accctcgcta aacaagccag gtaagttctg tcgtccatac 5760
attggaggta tatgttttca gtgaactgac aagtgtctcc gtagtgtctc tcttgtattt 5820
gtcaactcgg acgcgggaga gggctatatc tccgtggacg gaaacgaggg cgaccgcaac 5880
aacctcaccc tctggaagaa cggcgacaac ctcatcaagg ctgctgcaaa caactgcaac 5940
aacaccatcg ttgtcatcca ctccgttgga cctgttttgg ttgacgagtg gtatgaccac 6000
cccaacgtta ctgccatcct ctgggcgggc ttgccaggcc aggagtctgg caactccttg 6060
gctgacgtgc tctacggccg cgtcaacccg ggcgccaaat ctccattcac ctggggcaag 6120
acgagggagg cgtacgggga ttaccttgtc cgtgagctca acaacggcaa cggagctccc 6180
caagatgatt tctcggaagg tgttttcatt gactaccgcg gattcgacaa gcgcaatgag 6240
accccgatct acgagttcgg acatggtctg agctacacca ctttcaacta ctctggcctt 6300
cacatccagg ttctcaacgc ttcctccaac gctcaagtag ccactgagac tggcgccgct 6360
cccaccttcg gacaagtcgg caatgcctct gactacgtgt accctgaggg attgaccaga 6420
atcagcaagt tcatctatcc ctggcttaat tccacagacc tgaaggcctc atctggcgac 6480
ccgtactatg gagtcgacac cgcggagcac gtgcccgagg gtgctactga tggctctccg 6540
cagcccgttc tgcctgccgg tggtggcttc ggtggtaacc cgcgcctcta cgatgagttg 6600
atccgtgttt cggtgacagt caagaacact ggtcgtgttg ccggtgatgc tgtgcctcaa 6660
ttggtaatta gatcctcgtg cagtattggt tccagatgct aaccgcttgc tagtatgttt 6720
cccttggtgg acccaatgag cccaaggttg tgttgcgcaa attcgaccgc ctcaccctca 6780
agccctccga ggagacggtg tggacgacta ccctgacccg ccgcgatctg tctaactggg 6840
acgttgcggc tcaggactgg gtcatcactt cttacccgaa gaaggtccat gttggtagct 6900
cttcgcgtca gctgcccctt cacgcggcgc tcccgaaggt gcaataagct agcgggccgc 6960
atcatgtaat tagttatgtc acgcttacat tcacgccctc cccccacatc cgctctaacc 7020
gaaaaggaag gagttagaca acctgaagtc taggtcccta tttatttttt tatagttatg 7080
ttagtattaa gaacgttatt tatatttcaa atttttcttt tttttctgta cagacgcgtg 7140
tacgcatgta acattatact gaaaaccttg cttgagaagg ttttgggacg ctcgaaggct 7200
ttaatttgcg gccctgcatt aatgaatcgg ccaacgcgcg gggagaggcg gtttgcgtat 7260
tgggcgctct tccgcttcct cgctcactga ctcgctgcgc tcggtcgttc ggctgcggcg 7320
agcggtatca gctcactcaa aggcggtaat acggttatcc acagaatcag gggataacgc 7380
aggaaagaac atgtgagcaa aaggccagca aaagcccagg aaccgtaaaa aggccgcgtt 7440
gctggcgttt ttccataggc tccgcccccc tgacgagcat cacaaaaatc gacgctcaag 7500
tcagaggtgg cgaaacccga caggactata aagataccag gcgtttcccc ctggaagctc 7560

cctcgtgcgc tctcctgttc cgaccctgcc gcttaccgga tacctgtccg cctttctccc 7620
ttcgggaagc gtggcgcttt ctcatagctc acgctgtagg tatctcagtt cggtgtaggt 7680
cgttcgctcc aagctgggct gtgtgcacga accccccgtt cagcccgacc gctgcgcctt 7740
atccggtaac tatcgtcttg agtccaaccc ggtaagacac gacttatcgc cactggcagc 7800
agccactggt aacaggatta gcagagcgag gtatgtaggc ggtgctacag agttcttgaa 7860
gtggtggcct aactacggct acactagaag gacagtattt ggtatctgcg ctctgctgaa 7920
gccagttacc ttcggaaaaa gagttggtag ctcttgatcc ggcaaacaaa ccaccgctgg 7980
tagcggtggt ttttttgttt gcaagcagca gattacgcgc agaaaaaaag gatctcaaga 8040
agatcctttg atcttttcta cggggtctga cgctcagtgg aacgaaaact cacgttaagg 8100
gattttggtc atgagattat caaaaaggat cttc 8134

<210> 11
<211> 41
<212> DNA
<213> Aspergillus fumigatus

<400> 11
atacacaact ggatttacat gaagctcagt tggcttgagg c 41

<210> 12
<211> 39
<212> DNA
<213> Aspergillus fumigatus

<400> 12
aagagcaccc gtgttcttca gtagaacagt actgtctgc 39

<210> 13
<211> 39
<212> DNA
<213> Aspergillus fumigatus

<400> 13
actgttctac tgaagaacac gggtgctctt cctttgacc 39

<210> 14
<211> 39
<212> DNA
<213> Aspergillus fumigatus

<400> 14
gcccgggttg acgcggccat agagcacgtc gaccaggga 39

<210> 15
<211> 39
<212> DNA
<213> Aspergillus fumigatus

<400> 15
gacgtgctct atggccgcgt caacccgggc gccaaatct 39

<210> 16
<211> 50
<212> DNA
<213> Aspergillus fumigatus

<400> 16
gtcagtcacc tctagttaat taattattgc accttcggga gcgccgcgtg 50

<210> 17
<211> 36
<212> DNA
<213> Aspergillus fumigatus

<400> 17
gggtagtggt actgccgagt tcccttacct tgtcac 36

<210> 18
<211> 39
<212> DNA
<213> Aspergillus fumigatus

<400> 18
gccgactctg gagagggtta catcagtgtc gacggcaac 39

## Description

### Reference to a Sequence Listing

This application contains a Sequence Listing in computer readable form.

### Background of the Invention

### Field of the Invention

The present invention relates to chimeric polypeptides having beta-glucosidase activity, polynucleotides encoding the chimeric polypeptides, methods of producing the chimeric polypeptides, and methods of using the chimeric polypeptides.

### Description of the Related Art

Cellulose is a polymer of the simple sugar glucose covalently linked by beta-1,4-bonds. Many microorganisms produce enzymes that hydrolyze beta-linked glucans. These enzymes include endoglucanases, cellobiohydrolases, and beta-glucosidases. Endoglucanases digest the cellulose polymer at random locations, opening it to attack by cellobiohydrolases. Cellobiohydrolases sequentially release molecules of cellobiose from the ends of the cellulose polymer. Cellobiose is a water-soluble beta-1,4-linked dimer of glucose. Beta-glucosidases hydrolyze cellobiose to glucose.

The conversion of lignocellulosic feedstocks into ethanol has the advantages of the ready availability of large amounts of feedstock, the desirability of avoiding burning or land filling the materials, and the cleanliness of the ethanol fuel. Wood, agricultural residues, herbaceous crops, and municipal solid wastes have been considered as feedstocks for ethanol production. These materials primarily consist of cellulose, hemicellulose, and lignin. Once the lignocellulose is converted to fermentable sugars, *e.g.,* glucose, the fermentable sugars can easily be fermented by yeast into ethanol.

U.S Patent No. 7,244,605 discloses an *Aspergillus fumigatus* GH3A beta-glucosidase and the polynucleotide thereof. Kawaguchi et al., 1996, Gene 173:287-288, discloses an *Aspergillus aculeatus* GH3A beta-glucosidase and the polynucleotide thereof.

It would be advantageous in the art to improve the ability of beta-glucosidases to degrade lignocellulosic feedstocks.

The present invention provides chimeric polypeptides having beta-glucosidase activity.

### Summary of the Invention

The present invention relates to isolated chimeric polypeptides having beta-glucosidase activity, comprising:

(a) a first polypeptide fragment at the N-terminal end of the chimeric polypeptide selected from the group consisting of (i) a polypeptide fragment having at least 90% identity to amino acids 20 to 404 of SEQ ID NO: 2; and (ii) a polypeptide fragment comprising or consisting of amino acids 20 to 404 of SEQ ID NO: 2;

(b) a second polypeptide fragment at the C-terminal end of the first polypeptide fragment selected from the group consisting of (i) a polypeptide fragment having at least 90% identity to amino acids 406 to 589 of SEQ ID NO: 4; and (ii) a polypeptide fragment comprising or consisting of amino acids 406 to 589 of SEQ ID NO: 4; and

(c) a third polypeptide fragment at the C-terminal end of the second polypeptide fragment selected from the group consisting of (i) a polypeptide fragment having at least 90% identity to amino acids 589 to 860 of SEQ ID NO: 2; and (ii) a polypeptide fragment comprising or consisting of amino acids 589 to 860 of SEQ ID NO: 2, wherein said chimeric polypeptide has increased specific activity compared to both the mature polypeptide of SEQ ID NO: 2 and the mature polypeptide of SEQ ID NO: 4.

The present invention also relates to isolated polynucleotides encoding the chimeric polypeptides; nucleic acid constructs, vectors, and host cells comprising the polynucleotides; and methods of producing the chimeric polypeptides.

The present invention also relates to methods for degrading or converting a cellulosic material, comprising: treating the cellulosic material with an enzyme composition in the presence of a chimeric polypeptide having beta-glucosidase activity the present invention.

The present invention also relates to methods for producing a fermentation product, comprising: (a) saccharifying a cellulosic material with an enzyme composition in the presence of a chimeric polypeptide having beta-glucosidase activity the present invention; (b) fermenting the saccharified cellulosic material with one or more (e.g., several) fermenting microorganisms to produce the fermentation product; and (c) recovering the fermentation product from the fermentation.

The present invention also relates to methods of fermenting a cellulosic material, comprising: fermenting the cellulosic material with one or more (*e.g.,* several) fermenting microorganisms, wherein the cellulosic material is saccharified with an enzyme composition in the presence of a chimeric polypeptide having beta-glucosidase activity of the present invention.

**Brief Description of the Figures**

Figures 1A and 1B show the genomic DNA sequence and the deduced amino acid sequence of the *Aspergillus aculeatus* GH3A beta-glucosidase gene (SEQ ID NOs: 1 and 2, respectively). The predicted signal peptide is underlined and predicted introns are italicized.

Figures 2A and 2B show the genomic DNA sequence and the deduced amino acid sequence of the *Aspergillus fumigatus* GH3a beta-glucosidase gene (SEQ ID NOs: 3 and 4, respectively). The predicted signal peptide is underlined and predicted introns are italicized.

Figure 3 shows a restriction map of pAG114.

Figure 4 shows a restriction map of pDFng116.

Figure 5 shows the effect of the wild-type *Aspergillus fumigatus* GH3A beta-glucosidase, the wild-type *Aspergillus aculeatus* GH3A beta-glucosidase, and the chimeric beta-glucosidase on hydrolysis of pretreated corn stover (PCS) by a *Trichoderma reesei* cellulolytic protein composition. Circles = chimeric beta-glucosidase. Diamonds = wild-type *Aspergillus aculeatus* GH3A beta-glucosidase. Squares = wild-type *Aspergillus fumigatus* GH3A beta-glucosidase.

Figure 6 shows the effect of the wild-type *Aspergillus fumigatus* GH3A beta-glucosidase, the wild-type *Aspergillus aculeatus* GH3A beta-glucosidase, and the chimeric beta-glucosidase variant on hydrolysis of pretreated corn stover (PCS) by a *Trichoderma reesei* cellulolytic protein composition. Circles = chimeric beta-glucosidase variant. Diamonds = wild-type *Aspergillus aculeatus* GH3A beta-glucosidase. Squares = wild-type *Aspergillus fumigatus* GH3A beta-glucosidase.

**Definitions**

**Acetylxylan esterase:** The term "acetylxylan esterase" means a carboxylesterase (EC 3.1.1.72) that catalyzes the hydrolysis of acetyl groups from polymeric xylan, acetylated xylose, acetylated glucose, alpha-napthyl acetate, and *p*-nitrophenyl acetate. For purposes of the present invention, acetylxylan esterase activity is determined using 0.5 mM *p*-nitrophenylacetate as substrate in 50 mM sodium acetate pH 5.0 containing 0.01% TWEE™ 20 (polyoxyethylene sorbitan monolaurate). One unit of acetylxylan esterase is defined as the amount of enzyme capable of releasing 1 μmole of *p*-nitrophenolate anion per minute at pH 5, 25°C.

**Allelic variant:** The term "allelic variant" means any of two or more alternative forms of a gene occupying the same chromosomal locus. Allelic variation arises naturally through mutation, and may result in polymorphism within populations. Gene mutations can be silent (no change in the encoded polypeptide) or may encode polypeptides having altered amino acid sequences. An allelic variant of a polypeptide is a polypeptide encoded by an allelic variant of a gene.

**Alpha-L-arabinofuranosidase:** The term "alpha-L-arabinofuranosidase" means an alpha-L-arabinofuranoside arabinofuranohydrolase (EC 3.2.1.55) that catalyzes the hydrolysis of terminal non-reducing alpha-L-arabinofuranoside residues in alpha-L-arabinosides. The enzyme acts on alpha-L-arabinofuranosides, alpha-L-arabinans containing (1,3)- and/or (1,5)-linkages, arabinoxylans, and arabinogalactans. Alpha-L-arabinofuranosidase is also known as arabinosidase, alpha-arabinosidase, alpha-L-arabinosidase, alpha-arabinofuranosidase, polysaccharide alpha-L-arabinofuranosidase, alpha-L-arabinofuranoside hydrolase, L-arabinosidase, or alpha-L-arabinanase. For purposes of the present invention, alpha-L-arabinofuranosidase activity is determined using 5 mg of medium viscosity wheat arabinoxylan (Megazyme International Ireland, Ltd., Bray, Co. Wicklow, Ireland) per ml of 100 mM sodium acetate pH 5 in a total volume of 200 μl for 30 minutes at 40°C followed by arabinose analysis by AMINEX® HPX-87H column chromatography (Bio-Rad Laboratories, Inc., Hercules, CA, USA).

**Alpha-glucuronidase:** The term "alpha-glucuronidase" means an alpha-D-glucosiduronate glucuronohydrolase (EC 3.2.1.139) that catalyzes the hydrolysis of an alpha-D-glucuronoside to D-glucuronate and an alcohol. For purposes of the present invention, alpha-glucuronidase activity is determined according to de Vries, 1998, J. Bacteriol. 180: 243-249. One unit of alpha-glucuronidase equals the amount of enzyme capable of releasing 1 μmole of glucuronic or 4-O-methylglucuronic acid per minute at pH 5, 40°C.

**Beta-glucosidase:** The term "beta-glucosidase" means a beta-D-glucoside glucohydrolase (E.C. 3.2.1.21) that catalyzes the hydrolysis of terminal non-reducing beta-D-glucose residues with the release of beta-D-glucose. For purposes of the present invention, beta-glucosidase activity is determined using *p*-nitrophenyl-beta-D-glucopyranoside as substrate according to the procedure of Venturi et al., 2002, Extracellular beta-D-glucosidase from Chaetomium thermophilum var. coprophilum: production, purification and some biochemical properties, J. Basic Microbiol. 42: 55-66. One unit of beta-glucosidase is defined as 1.0 μmole of *p*-nitrophenolate anion produced per minute at 25°C, from 1 mM *p*-nitrophenyl-beta-D-glucopyranoside as substrate in 50 mM sodium citrate pH 4.8 containing 0.01% TWEEN® 20 or in 50 mM sodium acetate pH 5.0 containing 0.01% TWEEN® 20.

**Beta-xylosidase:** The term "beta-xylosidase" means a beta-D-xyloside xylohydrolase (E.C. 3.2.1.37) that catalyzes the exo-hydrolysis of short beta (1→4)-xylooligosaccharides, to remove successive D-xylose residues from non-reducing termini. For purposes of the present invention, one unit of beta-xylosidase is defined as 1.0 μmole of *p*-nitrophenolate anion produced per minute at 40°C, pH 5 from 1 mM *p*-nitrophenyl-beta-D-xyloside as substrate in 100 mM sodium citrate containing 0.01% TWEEN® 20.

**cDNA:** The term "cDNA" means a DNA molecule that can be prepared by reverse transcription from a mature, spliced, mRNA molecule obtained from a eukaryotic or prokaryotic cell. cDNA lacks intron sequences that may be present in the corresponding genomic DNA. The initial, primary RNA transcript is a precursor to mRNA that is processed through a series of steps, including splicing, before appearing as mature spliced mRNA.

**Cellobiohydrolase:** The term "cellobiohydrolase" means a 1,4-beta-D-glucan cellobiohydrolase (E.C. 3.2.1.91 and E.C. 3.2.1.176) that catalyzes the hydrolysis of 1,4-beta-D-glucosidic linkages in cellulose, cellooligosaccharides, or any beta-1,4-linked glucose containing polymer, releasing cellobiose from the reducing or non-reducing ends of the chain (Teeri, 1997, Crystalline cellulose degradation: New insight into the function of cellobiohydrolases, Trends in Biotechnology 15: 160-167; Teeri et al., 1998, Trichoderma reesei cellobiohydrolases: why so efficient on crystalline cellulose?, Biochem. Soc. Trans. 26: 173-178). Cellobiohydrolase activity is determined according to the procedures described by Lever et al., 1972, Anal. Biochem. 47: 273-279; van Tilbeurgh et al., 1982, FEBS Letters, 149: 152-156; van Tilbeurgh and Claeyssens, 1985, FEBS Letters, 187: 283-288; and Tomme et al., 1988, Eur. J. Biochem. 170: 575-581. In the present invention, the Tomme *et al.* method can be used to determine cellobiohydrolase activity.

**Cellulolytic enzyme or cellulase:** The term "cellulolytic enzyme" or "cellulase" means one or more (*e.g.,* several) enzymes that hydrolyze a cellulosic material. Such enzymes include endoglucanase(s), cellobiohydrolase(s), beta-glucosidase(s), or combinations thereof. The two basic approaches for measuring cellulolytic activity include: (1) measuring the total cellulolytic activity, and (2) measuring the individual cellulolytic activities (endoglucanases, cellobiohydrolases, and beta-glucosidases) as reviewed in Zhang et al., Outlook for cellulase improvement: Screening and selection strategies, 2006, Biotechnology Advances 24: 452-481. Total cellulolytic activity is usually measured using insoluble substrates, including Whatman №1 filter paper, microcrystalline cellulose, bacterial cellulose, algal cellulose, cotton, pretreated lignocellulose, *etc.* The most common total cellulolytic activity assay is the filter paper assay using Whatman №1 filter paper as the substrate. The assay was established by the International Union of Pure and Applied Chemistry (IUPAC) (Ghose, 1987, Measurement of cellulase activities, Pure Appl. Chem. 59: 257-68).

For purposes of the present invention, cellulolytic enzyme activity is determined by measuring the increase in hydrolysis of a cellulosic material by cellulolytic enzyme(s) under the following conditions: 1-50 mg of cellulolytic enzyme protein/g of cellulose in PCS (or other pretreated cellulosic material) for 3-7 days at a suitable temperature, *e.g.,* 50°C, 55°C, or 60°C, and a suitable pH, *e.g.,* 4-9, compared to a control hydrolysis without addition of cellulolytic enzyme protein. Typical conditions are 1 ml reactions, washed or unwashed PCS, 5% insoluble solids, 50 mM sodium acetate pH 5, 1 mM $MnSO_4$, 50°C, 55°C, or 60°C, 72 hours, sugar analysis by AMINEX® HPX-87H column (Bio-Rad Laboratories, Inc., Hercules, CA, USA).

**Cellulosic material:** The term "cellulosic material" means any material containing cellulose. The predominant polysaccharide in the primary cell wall of biomass is cellulose, the second most abundant is hemicellulose, and the third is pectin. The secondary cell wall, produced after the cell has stopped growing, also contains polysaccharides and is strengthened by polymeric lignin covalently cross-linked to hemicellulose. Cellulose is a homopolymer of anhydrocellobiose and thus a linear beta-(1-4)-D-glucan, while hemicelluloses include a variety of compounds, such as xylans, xyloglucans, arabinoxylans, and mannans in complex branched structures with a spectrum of substituents. Although generally polymorphous, cellulose is found in plant tissue primarily as an insoluble crystalline matrix of parallel glucan chains. Hemicelluloses usually hydrogen bond to cellulose, as well as to other hemicelluloses, which help stabilize the cell wall matrix.

Cellulose is generally found, for example, in the stems, leaves, hulls, husks, and cobs of plants or leaves, branches, and wood of trees. The cellulosic material can be, but is not limited to, agricultural residue, herbaceous material (including energy crops), municipal solid waste, pulp and paper mill residue, waste paper, and wood (including forestry residue) (see, for example, Wiselogel et al., 1995, in Handbook on Bioethanol (Charles E. Wyman, editor), pp.105-118, Taylor & Francis, Washington D.C.; Wyman, 1994, Bioresource Technology 50: 3-16; Lynd, 1990, Applied Biochemistry and Biotechnology 24/25: 695-719; Mosier et al., 1999, Recent Progress in Bioconversion of Lignocellulosics, in Advances in Biochemical Engineering/Biotechnology, T. Scheper, managing editor, Volume 65, pp.23-40, Springer-Verlag, New York). It is understood herein that the cellulose may be in the form of lignocellulose, a plant cell wall material containing lignin, cellulose, and hemicellulose in a mixed matrix. In a preferred aspect, the cellulosic material is any biomass material. In another preferred aspect, the cellulosic material is lignocellulose, which comprises cellulose, hemicelluloses, and lignin.

In one aspect, the cellulosic material is agricultural residue. In another aspect, the cellulosic material is herbaceous material (including energy crops). In another aspect, the cellulosic material is municipal solid waste. In another aspect, the cellulosic material is pulp and paper mill residue. In another aspect, the cellulosic material is waste paper. In another aspect, the cellulosic material is wood (including forestry residue).

In another aspect, the cellulosic material is arundo. In another aspect, the cellulosic material is bagasse. In another aspect, the cellulosic material is bamboo. In another aspect, the cellulosic material is corn cob. In another aspect, the cellulosic material is corn fiber. In another aspect, the cellulosic material is corn stover. In another aspect, the cellulosic material is miscanthus. In another aspect, the cellulosic material is orange peel. In another aspect, the cellulosic material is rice straw. In another aspect, the cellulosic material is switchgrass. In another aspect, the cellulosic material is wheat straw.

In another aspect, the cellulosic material is aspen. In another aspect, the cellulosic material is eucalyptus. In another aspect, the cellulosic material is fir. In another aspect, the cellulosic material is pine. In another aspect, the cellulosic material is poplar. In another aspect, the cellulosic material is spruce. In another aspect, the cellulosic material is willow.

In another aspect, the cellulosic material is algal cellulose. In another aspect, the cellulosic material is bacterial cellulose. In another aspect, the cellulosic material is cotton linter. In another aspect, the cellulosic material is filter paper. In another aspect, the cellulosic material is microcrystalline cellulose. In another aspect, the cellulosic material is phosphoric-acid treated cellulose.

In another aspect, the cellulosic material is an aquatic biomass. As used herein the term "aquatic biomass" means biomass produced in an aquatic environment by a photosynthesis process. The aquatic biomass can be algae, emergent plants, floating-leaf plants, or submerged plants.

The cellulosic material may be used as is or may be subjected to pretreatment, using conventional methods known in the art, as described herein. In a preferred aspect, the cellulosic material is pretreated.

**Chimeric polypeptide having beta-glucosidase activity:** The term "chimeric polypeptide having beta-glucosidase activity" means a polypeptide whose composition is generated by replacing one or more (*e.g.,* several) sequences of amino acids from one beta-glucosidase with those from homologous positions of one or more (*e.g.,* several) other beta-glucosidases, wherein the chimeric polypeptide has beta-glucosidase activity. The term "chimeric polypeptide having beta-glucosidase activity" is also referred to herein as "chimeric beta-glucosidase".

**Coding sequence:** The term "coding sequence" means a polynucleotide, which directly specifies the amino acid sequence of a polypeptide. The boundaries of the coding sequence are generally determined by an open reading frame, which begins with a start codon such as ATG, GTG or TTG and ends with a stop codon such as TAA, TAG, or TGA. The coding sequence may be a genomic DNA, cDNA, synthetic DNA, or a combination thereof.

**Control sequences:** The term "control sequences" means nucleic acid sequences necessary for expression of a polynucleotide encoding a polypeptide. Each control sequence may be native (*i.e.,* from the same gene) or foreign (*i.e.,* from a different gene) to the polynucleotide encoding the polypeptide or native or foreign to each other. Such control sequences include, but are not limited to, a leader, polyadenylation sequence, propeptide sequence, promoter, signal peptide sequence, and transcription terminator. At a minimum, the control sequences include a promoter, and transcriptional and translational stop signals. The control sequences may be provided with linkers for the purpose of introducing specific restriction sites facilitating ligation of the control sequences with the coding region of the polynucleotide encoding a polypeptide.

**Endoglucanase:** The term "endoglucanase" means an endo-1,4-(1,3;1,4)-beta-D-glucan 4-glucanohydrolase (E.C. 3.2.1.4) that catalyzes endohydrolysis of 1,4-beta-D-glycosidic linkages in cellulose, cellulose derivatives (such as carboxymethyl cellulose and hydroxyethyl cellulose), lichenin, beta-1,4 bonds in mixed beta-1,3 glucans such as cereal beta-D-glucans or xyloglucans, and other plant material containing cellulosic components. Endoglucanase activity can be determined by measuring reduction in substrate viscosity or increase in reducing ends determined by a reducing sugar assay (Zhang et al., 2006, Biotechnology Advances 24: 452-481). For purposes of the present invention, endoglucanase activity is determined using carboxymethyl cellulose (CMC) as substrate according to the procedure of Ghose, 1987, Pure and Appl. Chem. 59: 257-268, at pH 5, 40°C.

**Expression:** The term "expression" includes any step involved in the production of a polypeptide including, but not limited to, transcription, post-transcriptional modification, translation, post-translational modification, and secretion.

**Expression vector:** The term "expression vector" means a linear or circular DNA molecule that comprises a polynucleotide encoding a polypeptide and is operably linked to control sequences that provide for its expression.

**Family GH3 beta-glucosidase:** The term "Family GH3 beta-glucosidase" means a glycoside hydrolase of Family 3 according to Coutinho, P.M. and Henrissat, B., 1999, Carbohydrate-active enzymes: an integrated database approach, in "Recent Advances in Carbohydrate Bioengineering", H.J. Gilbert, G. Davies, B. Henrissat and B. Svensson eds., The Royal Society of Chemistry, Cambridge, pp. 3-12.

**Family 61 glycoside hydrolase:** The term "Family 61 glycoside hydrolase" or "Family GH61" or "GH61" means a polypeptide falling into the glycoside hydrolase Family 61 according to Henrissat B., 1991, A classification of glycosyl hydrolases based on amino-acid sequence similarities, Biochem. J. 280: 309-316, and Henrissat B., and Bairoch A., 1996, Updating the sequence-based classification of glycosyl hydrolases, Biochem. J. 316: 695-696. The enzymes in this family were originally classified as a glycoside hydrolase family based on measurement of very weak endo-1,4-beta-D-glucanase activity in one family member. The structure and mode of action of these enzymes are non-canonical and they cannot be considered as bona fide glycosidases. However, they are kept in the CAZy classification on the basis of their capacity to enhance the breakdown of lignocellulose when used in conjunction with a cellulase or a mixture of cellulases.

**Feruloyl esterase:** The term "feruloyl esterase" means a 4-hydroxy-3-methoxycinnamoyl-sugar hydrolase (EC 3.1.1.73) that catalyzes the hydrolysis of 4-hydroxy-3-methoxycinnamoyl (feruloyl) groups from esterified sugar, which is usually arabinose in natural biomass substrates, to produce ferulate (4-hydroxy-3-methoxycinnamate). Feruloyl esterase is also known as ferulic acid esterase, hydroxycinnamoyl esterase, FAE-III, cinnamoyl ester hydrolase, FAEA, cinnAE, FAE-I, or FAE-II. For purposes of the present invention, feruloyl esterase activity is determined using 0.5 mM *p*-nitrophenylferulate as substrate in 50 mM sodium acetate pH 5.0. One unit of feruloyl esterase equals the amount of enzyme capable of releasing 1 μmole of *p*-nitrophenolate anion per minute at pH 5, 25°C.

**Fragment:** The term "fragment" means a polypeptide having one or more (*e.g.,* several) amino acids absent from the amino and/or carboxyl terminus of a mature polypeptide; wherein the fragment has beta-glucosidase activity. In one aspect, a fragment contains at least 720 amino acid residues, *e.g.,* at least 760 amino acid residues or at least 800 amino acid residues of SEQ ID NO: 2. In another aspect, a fragment contains at least 720 amino acid residues, *e.g.,* at least 760 amino acid residues or at least 800 amino acid residues of SEQ ID NO: 4. In another aspect, a fragment contains at least 720 amino acid residues, *e.g.,* at least 760 amino acid residues or at least 800 amino acid residues of SEQ ID NO: 6 or SEO ID NO: 8.

**Hemicellulolytic enzyme or hemicellulase:** The term "hemicellulolytic enzyme" or "hemicellulase" means one or more (e.g., several) enzymes that hydrolyze a hemicellulosic material. See, for example, Shallom, D. and Shoham, Y. Microbial hemicellulases. Current Opinion In Microbiology, 2003, 6(3): 219-228). Hemicellulases are key components in the degradation of plant biomass. Examples of hemicellulases include, but are not limited to, an acetylmannan esterase, an acetylxylan esterase, an arabinanase, an arabinofuranosidase, a coumaric acid esterase, a feruloyl esterase, a galactosidase, a glucuronidase, a glucuronoyl esterase, a mannanase, a mannosidase, a xylanase, and a xylosidase. The substrates of these enzymes, the hemicelluloses, are a heterogeneous group of branched and linear polysaccharides that are bound via hydrogen bonds to the cellulose microfibrils in the plant cell wall, crosslinking them into a robust network. Hemicelluloses are also covalently attached to lignin, forming together with cellulose a highly complex structure. The variable structure and organization of hemicelluloses require the concerted action of many enzymes for its complete degradation. The catalytic modules of hemicellulases are either glycoside hydrolases (GHs) that hydrolyze glycosidic bonds, or carbohydrate esterases (CEs), which hydrolyze ester linkages of acetate or ferulic acid side groups. These catalytic modules, based on homology of their primary sequence, can be assigned into GH and CE families. Some families, with an overall similar fold, can be further grouped into clans, marked alphabetically (*e.g.,* GH-A). A most informative and updated classification of these and other carbohydrate active enzymes is available in the Carbohydrate-Active Enzymes (CAZy) database. Hemicellulolytic enzyme activities can be measured according to Ghose and Bisaria, 1987, Pure & Appl. Chem. 59: 1739-1752, at a suitable temperature, *e.g.,* 50°C, 55°C, or 60°C, and pH, *e.g.,* 5.0 or 5.5.

**High stringency conditions:** The term "high stringency conditions" means for probes of at least 100 nucleotides in length, prehybridization and hybridization at 42°C in 5X SSPE, 0.3% SDS, 200 micrograms/ml sheared and denatured salmon sperm DNA, and 50% formamide, following standard Southern blotting procedures for 12 to 24 hours. The carrier material is finally washed three times each for 15 minutes using 2X SSC, 0.2% SDS at 65°C.

**Host cell:** The term "host cell" means any cell type that is susceptible to transformation, transfection, transduction, or the like with a nucleic acid construct or expression vector comprising a polynucleotide of the present invention. The term "host cell" encompasses any progeny of a parent cell that is not identical to the parent cell due to mutations that occur during replication.

**Increased specific activity:** The term "increased specific activity" means a higher enzyme activity per molecule or μmole of a chimeric beta-glucosidase or a variant thereof compared to the enzyme activity per molecule or μmole of one or more (*e.g.,* several) parents of the chimeric beta-glucosidase or the variant thereof. The increased specific activity of the chimeric beta-glucosidase or the variant thereof relative to the one or more parents can be assessed, for example, under one or more (*e.g.,* several) conditions of pH, temperature, substrate concentration, and product inhibitor concentration, *e.g.,* glucose.

In one aspect, the chimeric beta-glucosidase or the variant thereof has an increased specific activity relative to one or more of the parent beta-glucosidases. In another aspect, the chimeric beta-glucosidase or the variant thereof has an increased specific activity relative to one of the parent beta-glucosidases. In another aspect, the chimeric beta-glucosidase or the variant thereof has an increased specific activity relative to each of the parent beta-glucosidases.

In one aspect, the condition is pH. For example, the pH can be any pH in the range of 3 to 7, *e.g.,* 3.0, 3.5, 4.0, 4.5, 5.0, 5.5, 6.0, 6.5, or 7.0 (or in between). Any suitable buffer for achieving the desired pH can be used.

In another aspect, the condition is temperature. For example, the temperature can be any temperature in the range of 25°C to 90°C, *e.g.,* 25, 30, 35, 40, 45, 50, 55, 60, 65, 70, 75, 80, 85, or 90°C (or in between).

In another aspect, the condition is substrate concentration. For example, the substrate concentration can be any substrate concentration in the range of 1 mM to 100 mM, *e.g.,* 1 mM, 10 mM, 25 mM, 50 mM, 75 mM, or 100 mM (or in between). Examples of a substrate include without limitation cellobiose, cellotriose, cellotetrose, beta-gentiobiose, laminaribose, or sophorose.

In another aspect, the condition is product inhibitor concentration. For example, the product inhibitor concentration can be any concentration in the range of 1 mM to 120 mM, *e.g.,* 1 mM, 10 mM, 25 mM, 50 mM, 75 mM, 100 mM, or 120 mM (or in between). Examples of a product include without limitation glucose, beta-D-mannose, beta-D-sorbose, or D-glucuronic acid.

In another aspect, a combination of two or more (*e.g.,* several) of the above conditions are used to determine the increased specific activity of the chimeric beta-glucosidase or the variant thereof relative to a parent, such as any temperature in the range of 25°C to 90°C, *e.g.,* 25, 30, 35, 40, 45, 50, 55, 60, 65, 70, 75, 80, 85, or 90°C (or in between) at a pH in the range of 3 to 7, *e.g.,* 3.0, 3.5, 4.0, 4.5, 5.0, 5.5, 6.0, 6.5, or 7.0 (or in between).

In one aspect, the specific activity of the chimeric beta-glucosidase or the variant thereof relative to a parent is determined at pH 4.0 and 25°C. In another aspect, the specific activity of the chimeric beta-glucosidase or the variant thereof relative to a parent is determined at pH 4.0 and 30°C. In another aspect, the specific activity of the chimeric beta-glucosidase or the variant thereof relative to a parent is determined at pH 4.0 and 35°C. In another aspect, the specific activity of the chimeric beta-glucosidase or the variant thereof relative to a parent is determined at pH 4.0 and 40°C. In another aspect, the specific activity of the chimeric beta-glucosidase or the variant thereof relative to a parent is determined at pH 4.0 and 45°C. In another aspect, the specific activity of the chimeric beta-glucosidase or the variant thereof relative to a parent is determined at pH 4.0 and 50°C. In another aspect, the specific activity of the chimeric beta-glucosidase or the variant thereof relative to a parent is determined at pH 4.0 and 55°C. In another aspect, the specific activity of the chimeric beta-glucosidase or the variant thereof relative to a parent is determined at pH 4.0 and 60°C. In another aspect, the specific activity of the chimeric beta-glucosidase or the variant thereof relative to a parent is determined at pH 4.0 and 65°C. In another aspect, the specific activity of the chimeric beta-glucosidase or the variant thereof relative to a parent is determined at pH 4.0 and 70°C. In another aspect, the specific activity of the chimeric beta-glucosidase or the variant thereof relative to a parent is determined at pH 4.0 and 75°C. In another aspect, the specific activity of the chimeric beta-glucosidase or the variant thereof relative to a parent is determined at pH 4.0 and 80°C. In another aspect, the specific activity of the chimeric beta-glucosidase or the variant thereof relative to a parent is determined at pH 4.0 and 85°C. In another aspect, the specific activity of the chimeric beta-glucosidase or the variant thereof relative to a parent is determined at pH 4.0 and 90°C.

In another aspect, the specific activity of the chimeric beta-glucosidase or the variant thereof relative to a parent is determined at pH 4.5 and 25°C. In another aspect, the specific activity of the chimeric beta-glucosidase or the variant thereof relative to a parent is determined at pH 4.5 and 30°C. In another aspect, the specific activity of the chimeric beta-glucosidase or the variant thereof relative to a parent is determined at pH 4.5 and 35°C. In another aspect, the specific activity of the chimeric beta-glucosidase or the variant thereof relative to a parent is determined at pH 4.5 and 40°C. In another aspect, the specific activity of the chimeric beta-glucosidase or the variant thereof relative to a parent is determined at pH 4.5 and 45°C. In another aspect, the specific activity of the chimeric beta-glucosidase or the variant thereof relative to a parent is determined at pH 4.5 and 50°C. In another aspect, the specific activity of the chimeric beta-glucosidase or the variant thereof relative to a parent is determined at pH 4.5 and 55°C. In another aspect, the specific activity of the chimeric beta-glucosidase or the variant thereof relative to a parent is determined at pH 4.5 and 60°C. In another aspect, the specific activity of the chimeric beta-glucosidase or the variant thereof relative to a parent is determined at pH 4.5 and 65°C. In another aspect, the specific activity of the chimeric beta-glucosidase or the variant thereof relative to a parent is determined at pH 4.5 and 70°C. In another aspect, the specific activity of the chimeric beta-glucosidase or the variant thereof relative to a parent is determined at pH 4.5 and 75°C. In another aspect, the specific activity of the chimeric beta-glucosidase or the variant thereof relative to a parent is determined at pH 4.5 and 80°C. In another aspect, the specific activity of the chimeric beta-glucosidase or the variant thereof relative to a parent is determined at pH 4.5 and 85°C. In another aspect, the specific activity of the chimeric beta-glucosidase or the variant thereof relative to a parent is determined at pH 4.5 and 90°C.

In another aspect, the specific activity of the chimeric beta-glucosidase or the variant thereof relative to a parent is determined at pH 5.0 and 25°C. In another aspect, the specific activity of the chimeric beta-glucosidase or the variant thereof relative to a parent is determined at pH 5.0 and 30°C. In another aspect, the specific activity of the chimeric beta-glucosidase or the variant thereof relative to a parent is determined at pH 5.0 and 35°C. In another aspect, the specific activity of the chimeric beta-glucosidase or the variant thereof relative to a parent is determined at pH 5.0 and 40°C. In another aspect, the specific activity of the chimeric beta-glucosidase or the variant thereof relative to a parent is determined at pH 5.0 and 45°C. In another aspect, the specific activity of the chimeric beta-glucosidase or the variant thereof relative to a parent is determined at pH 5.0 and 50°C. In another aspect, the specific activity of the chimeric beta-glucosidase or the variant thereof relative to a parent is determined at pH 5.0 and 55°C. In another aspect, the specific activity of the chimeric beta-glucosidase or the variant thereof relative to a parent is determined at pH 5.0 and 60°C. In another aspect, the specific activity of the chimeric beta-glucosidase or the variant thereof relative to a parent is determined at pH 5.0 and 65°C. In another aspect, the specific activity of the chimeric beta-glucosidase or the variant thereof relative to a parent is determined at pH 5.0 and 70°C. In another aspect, the specific activity of the chimeric beta-glucosidase or the variant thereof relative to a parent is determined at pH 5.0 and 75°C. In another aspect, the specific activity of the chimeric beta-glucosidase or the variant thereof relative to a parent is determined at pH 5.0 and 80°C. In another aspect, the specific activity of the chimeric beta-glucosidase or the variant thereof relative to a parent is determined at pH 5.0 and 85°C. In another aspect, the specific activity of the chimeric beta-glucosidase or the variant thereof relative to a parent is determined at pH 5.0 and 90°C.

In another aspect, the specific activity of the chimeric beta-glucosidase or the variant thereof relative to a parent is determined at pH 5.5 and 25°C. In another aspect, the specific activity of the chimeric beta-glucosidase or the variant thereof relative to a parent is determined at pH 5.5 and 30°C. In another aspect, the specific activity of the chimeric beta-glucosidase or the variant thereof relative to a parent is determined at pH 5.5 and 35°C. In another aspect, the specific activity of the chimeric beta-glucosidase or the variant thereof relative to a parent is determined at pH 5.5 and 40°C. In another aspect, the specific activity of the chimeric beta-glucosidase or the variant thereof relative to a parent is determined at pH 5.5 and 45°C. In another aspect, the specific activity of the chimeric beta-glucosidase or the variant thereof relative to a parent is determined at pH 5.5 and 50°C. In another aspect, the specific activity of the chimeric beta-glucosidase or the variant thereof relative to a parent is determined at pH 5.5 and 55°C. In another aspect, the specific activity of the chimeric beta-glucosidase or the variant thereof relative to a parent is determined at pH 5.5 and 60°C. In another aspect, the specific activity of the chimeric beta-glucosidase or the variant thereof relative to a parent is determined at pH 5.5 and 65°C. In another aspect, the specific activity of the chimeric beta-glucosidase or the variant thereof relative to a parent is determined at pH 5.5 and 70°C. In another aspect, the specific activity of the chimeric beta-glucosidase or the variant thereof relative to a parent is determined at pH 5.5 and 75°C. In another aspect, the specific activity of the chimeric beta-glucosidase or the variant thereof relative to a parent is determined at pH 5.5 and 80°C. In another aspect, the specific activity of the chimeric beta-glucosidase or the variant thereof relative to a parent is determined at pH 5.5 and 85°C. In another aspect, the specific activity of the chimeric beta-glucosidase or the variant thereof relative to a parent is determined at pH 5.5 and 90°C.

In another aspect, the specific activity of the chimeric beta-glucosidase or the variant thereof relative to a parent is determined at pH 6.0 and 25°C. In another aspect, the specific activity of the chimeric beta-glucosidase or the variant thereof relative to a parent is determined at pH 6.0 and 30°C. In another aspect, the specific activity of the chimeric beta-glucosidase or the variant thereof relative to a parent is determined at pH 6.0 and 35°C. In another aspect, the specific activity of the chimeric beta-glucosidase or the variant thereof relative to a parent is determined at pH 6.0 and 40°C. In another aspect, the specific activity of the chimeric beta-glucosidase or the variant thereof relative to a parent is determined at pH 6.0 and 45°C. In another aspect, the specific activity of the chimeric beta-glucosidase or the variant thereof relative to a parent is determined at pH 6.0 and 50°C. In another aspect, the specific activity of the chimeric beta-glucosidase or the variant thereof relative to a parent is determined at pH 6.0 and 55°C. In another aspect, the specific activity of the chimeric beta-glucosidase or the variant thereof relative to a parent is determined at pH 6.0 and 60°C. In another aspect, the specific activity of the chimeric beta-glucosidase or the variant thereof relative to a parent is determined at pH 6.0 and 65°C. In another aspect, the specific activity of the chimeric beta-glucosidase or the variant thereof relative to a parent is determined at pH 6.0 and 70°C. In another aspect, the specific activity of the chimeric beta-glucosidase or the variant thereof relative to a parent is determined at pH 6.0 and 75°C. In another aspect, the specific activity of the chimeric beta-glucosidase or the variant thereof relative to a parent is determined at pH 6.0 and 80°C. In another aspect, the specific activity of the chimeric beta-glucosidase or the variant thereof relative to a parent is determined at pH 6.0 and 85°C. In another aspect, the specific activity of the chimeric beta-glucosidase or the variant thereof relative to a parent is determined at pH 6.0 and 90°C.

In each of the aspects above, the specific activity of the chimeric beta-glucosidase or the variant thereof relative to a parent can be determined in the presence of glucose at a concentration of 10 mM. In each of the aspects above, the specific activity of the chimeric beta-glucosidase or the variant thereof relative to a parent can be determined in the presence of glucose at a concentration of 25 mM. In each of the aspects above, the specific activity of the chimeric beta-glucosidase or the variant thereof relative to a parent can be determined in the presence of glucose at a concentration of 50 mM. In each of the aspects above, the specific activity of the chimeric beta-glucosidase or the variant thereof relative to a parent can be determined in the presence of glucose at a concentration of 75 mM. In each of the aspects above, the specific activity of the chimeric beta-glucosidase or the variant thereof relative to a parent can be determined in the presence of glucose at a concentration of 100 mM. In each of the aspects above, the specific activity of the chimeric beta-glucosidase or the variant thereof relative to a parent can be determined in the presence of glucose at a concentration of 120 mM.

The increased specific activity of the chimeric beta-glucosidase or the variant thereof relative to a parent can be determined using any enzyme assay known in the art for beta-glucosidases. Such enzyme assays include without limitation cellobiose or *p*-nitrophenyl-beta-D-glucopyranoside as a substrate. Alternatively, the increased specific activity of the chimeric beta-glucosidase or the variant thereof relative to a parent can be determined using any application assay for the chimeric beta-glucosidase or the variant thereof where the performance of the chimeric beta-glucosidase or the variant thereof is compared to the parent. For example, the application assay described in Example 7 can be used.

In one aspect, the specific activity of the chimeric beta-glucosidase or the variant thereof is at least 1.01-fold, e.g., at least 1.05-fold, at least 1.1-fold, at least 1.5-fold, at least 1.8-fold, at least 2-fold, at least 5-fold, at least 10-fold, at least 15-fold, at least 20-fold, at least 25-fold, and at least 50-fold higher than the specific activity of a parent.

**Isolated:** The term "isolated" means a substance in a form or environment that does not occur in nature. Non-limiting examples of isolated substances include (1) any non-naturally occurring substance, (2) any substance including, but not limited to, any enzyme, variant, nucleic acid, protein, peptide or cofactor, that is at least partially removed from one or more or all of the naturally occurring constituents with which it is associated in nature; (3) any substance modified by the hand of man relative to that substance found in nature; or (4) any substance modified by increasing the amount of the substance relative to other components with which it is naturally associated (*e.g.,* recombinant production in a host cell; multiple copies of a gene encoding the substance; and use of a stronger promoter than the promoter naturally associated with the gene encoding the substance)

**Low stringency conditions:** The term "low stringency conditions" means for probes of at least 100 nucleotides in length, prehybridization and hybridization at 42°C in 5X SSPE, 0.3% SDS, 200 micrograms/ml sheared and denatured salmon sperm DNA, and 25% formamide, following standard Southern blotting procedures for 12 to 24 hours. The carrier material is finally washed three times each for 15 minutes using 2X SSC, 0.2% SDS at 50°C.

**Mature polypeptide:** The term "mature polypeptide" means a polypeptide in its final form following translation and any post-translational modifications, such as N-terminal processing, C-terminal truncation, glycosylation, phosphorylation, etc. In one aspect, the mature polypeptide is amino acids 20 to 860 of SEQ ID NO: 2 based on the SignalP program (Nielsen et al., 1997, Protein Engineering 10: 1-6) that predicts amino acids 1 to 19 of SEQ ID NO: 1 are a signal peptide. In another aspect, the mature polypeptide is amino acids 20 to 863 of SEQ ID NO: 4 based on the SignalP program that predicts amino acids 1 to 19 of SEQ ID NO: 4 are a signal peptide. In another aspect, the mature polypeptide is amino acids 20 to 860 of SEQ ID NO: 6 based on the SignalP program that predicts amino acids 1 to 19 of SEQ ID NO: 6 are a signal peptide. In another aspect, the mature polypeptide is amino acids 20 to 860 of SEQ ID NO: 8 based on the SignalP program that predicts amino acids 1 to 19 of SEQ ID NO: 8 are a signal peptide. It is known in the art that a host cell may produce a mixture of two of more different mature polypeptides (*i.e.*, with a different C-terminal and/or N-terminal amino acid) expressed by the same polynucleotide.

**Mature polypeptide coding sequence:** The term "mature polypeptide coding sequence" means a polynucleotide that encodes a mature polypeptide having beta-glucosidase activity. In one aspect, the mature polypeptide coding sequence is nucleotides 131 to 2937 of SEQ ID NO: 1 based on the SignalP program (Nielsen *et al.*, 1997, *supra)* that predicts nucleotides 1 to 57 of SEQ ID NO: 1 encode a signal peptide. In another aspect, the mature polypeptide coding sequence is the cDNA sequence contained in nucleotides 131 to 2937 of SEQ ID NO: 1. In another aspect, the mature polypeptide coding sequence is nucleotides 58 to 3057 of SEQ ID NO: 3 based on the SignalP program that predicts nucleotides 1 to 57 of SEQ ID NO: 3 encode a signal peptide. In another aspect, the mature polypeptide coding sequence is the cDNA sequence contained in nucleotides 58 to 3057 of SEQ ID NO: 3. In another aspect, the mature polypeptide coding sequence is nucleotides 131 to 2936 of SEQ ID NO: 5 based on the SignalP program that predicts nucleotides 1 to 57 of SEQ ID NO: 5 encode a signal peptide. In another aspect, the mature polypeptide coding sequence is the cDNA sequence contained in nucleotides 131 to 2936 of SEQ ID NO: 5. In another aspect, the mature polypeptide coding sequence is nucleotides 131 to 2936 of SEQ ID NO: 7 based on the SignalP program that predicts nucleotides 1 to 57 of SEQ ID NO: 7 encode a signal peptide. In another aspect, the mature polypeptide coding sequence is the cDNA sequence contained in nucleotides 131 to 2936 of SEQ ID NO: 7.

**Medium stringency conditions:** The term "medium stringency conditions" means for probes of at least 100 nucleotides in length, prehybridization and hybridization at 42°C in 5X SSPE, 0.3% SDS, 200 micrograms/ml sheared and denatured salmon sperm DNA, and 35% formamide, following standard Southern blotting procedures for 12 to 24 hours. The carrier material is finally washed three times each for 15 minutes using 2X SSC, 0.2% SDS at 55°C.

**Medium-high stringency conditions:** The term "medium-high stringency conditions" means for probes of at least 100 nucleotides in length, prehybridization and hybridization at 42°C in 5X SSPE, 0.3% SDS, 200 micrograms/ml sheared and denatured salmon sperm DNA, and 35% formamide, following standard Southern blotting procedures for 12 to 24 hours. The carrier material is finally washed three times each for 15 minutes using 2X SSC, 0.2% SDS at 60°C.

**Mutant:** The term "mutant" means a polynucleotide encoding a variant.

**Nucleic acid construct:** The term "nucleic acid construct" means a nucleic acid molecule, either single- or double-stranded, which is isolated from a naturally occurring gene or is modified to contain segments of nucleic acids in a manner that would not otherwise exist in nature or which is synthetic, which comprises one or more control sequences.

**Operably linked:** The term "operably linked" means a configuration in which a control sequence is placed at an appropriate position relative to the coding sequence of a polynucleotide such that the control sequence directs expression of the coding sequence.

**Parent or parent beta-glucosidase (chimeric beta-glucosidase):** For a chimeric beta-glucosidase, the term "parent" or "parent beta-glucosidase" means a beta-glucosidase, a portion of which is a component of a chimeric beta-glucosidase or a variant thereof.

**Parent or parent beta-glucosidase (variants):** For variants, the term "parent" or "parent beta-glucosidase" means a beta-glucosidase to which an alteration, *i.e*., a substitution, insertion, and/or deletion, at one or more (*e.g.,* several) positions, is made to produce variants of the present invention. The parent may be a naturally occurring (wild-type) polypeptide, a chimeric polypeptide, fusion polypeptide; or a variant thereof; or a fragment thereof.

**Polypeptide having cellulolytic enhancing activity:** The term "polypeptide having cellulolytic enhancing activity" means a GH61 polypeptide that catalyzes the enhancement of the hydrolysis of a cellulosic material by enzyme having cellulolytic activity. For purposes of the present invention, cellulolytic enhancing activity is determined by measuring the increase in reducing sugars or the increase of the total of cellobiose and glucose from the hydrolysis of a cellulosic material by cellulolytic enzyme under the following conditions: 1-50 mg of total protein/g of cellulose in pretreated corn stover (PCS), wherein total protein is comprised of 50-99.5% w/w cellulolytic enzyme protein and 0.5-50% w/w protein of a GH61 polypeptide having cellulolytic enhancing activity for 1-7 days at a suitable temperature, *e.g.,* 50°C, 55°C, or 60°C, and a suitable pH, such as 4-9, *e.g.,* 5.0 or 5.5, compared to a control hydrolysis with equal total protein loading without cellulolytic enhancing activity (1-50 mg of cellulolytic protein/g of cellulose in PCS). In a preferred aspect, a mixture of CELLUCLAST® 1.5L (Novozymes A/S, Bagsvaerd, Denmark) in the presence of 2-3% of total protein weight *Aspergillus oryzae* beta-glucosidase (recombinantly produced in *Aspergillus oryzae* according to WO 02/095014) or 2-3% of total protein weight *Aspergillus fumigatus* beta-glucosidase (recombinantly produced in *Aspergillus oryzae* as described in WO 2002/095014) of cellulase protein loading is used as the source of the cellulolytic activity.

The GH61 polypeptides having cellulolytic enhancing activity enhance the hydrolysis of a cellulosic material catalyzed by enzyme having cellulolytic activity by reducing the amount of cellulolytic enzyme required to reach the same degree of hydrolysis preferably at least 1.01-fold, e.g., at least 1.05-fold, at least 1.10-fold, at least 1.25-fold, at least 1.5-fold, at least 2-fold, at least 3-fold, at least 4-fold, at least 5-fold, at least 10-fold, or at least 20-fold.

**Pretreated corn stover:** The term "PCS" or "Pretreated Corn Stover" means a cellulosic material derived from corn stover by treatment with heat and dilute sulfuric acid, alkaline pretreatment, or neutral pretreatment.

**Sequence identity:** The relatedness between two amino acid sequences or between two nucleotide sequences is described by the parameter "sequence identity".

For purposes of the present invention, the sequence identity between two amino acid sequences is determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends Genet. 16: 276-277), preferably version 3.0.0, 5.0.0, or later. The parameters used are gap open penalty of 10, gap extension penalty of 0.5, and the EBLOSUM62 (EMBOSS version of BLOSUM62) substitution matrix. The output of Needle labeled "longest identity" (obtained using the -nobrief option) is used as the percent identity and is calculated as follows:

(Identical Residues x 100)/(Length of Alignment – Total Number of Gaps in Alignment)

For purposes of the present invention, the sequence identity between two deoxyribonucleotide sequences is determined using the Needleman-Wunsch algorithm (Needleman and Wunsch, 1970, *supra)* as implemented in the Needle program of the EMBOSS package (EMBOSS: The European Molecular Biology Open Software Suite, Rice et al., 2000, *supra*), preferably version 3.0.0, 5.0.0, or later. The parameters used are gap open penalty of 10, gap extension penalty of 0.5, and the EDNAFULL (EMBOSS version of NCBI NUC4.4) substitution matrix. The output of Needle labeled "longest identity" (obtained using the -nobrief option) is used as the percent identity and is calculated as follows:

(Identical Deoxyribonucleotides x 100)/(Length of Alignment – Total Number of Gaps in Alignment)

**Subsequence:** The term "subsequence" means a polynucleotide having one or more (*e.g.,* several) nucleotides absent from the 5'- and/or 3'-end of a mature polypeptide coding sequence; wherein the subsequence encodes a fragment having beta-glucosidase activity. In one aspect, a subsequence contains at least 85%, *e.g.,* at least 90% or at least 95% of the nucleotides of the mature polypeptide coding sequence of SEQ ID NO: 1, SEQ ID NO: 3, SEQ ID NO: 5, or SEQ ID NO: 7; or the cDNA sequence thereof.

**Variant:** The term "variant" means a beta-glucosidase or a chimeric beta-glucosidase comprising an alteration, *i.e.*, a substitution, insertion, and/or deletion, at one or more (*e.g.,* several) positions. A substitution means replacement of the amino acid occupying a position with a different amino acid; a deletion means removal of the amino acid occupying a position; and an insertion means adding an amino acid adjacent to and immediately following the amino acid occupying a position.

**Very high stringency conditions:** The term "very high stringency conditions" means for probes of at least 100 nucleotides in length, prehybridization and hybridization at 42°C in 5X SSPE, 0.3% SDS, 200 micrograms/ml sheared and denatured salmon sperm DNA, and 50% formamide, following standard Southern blotting procedures for 12 to 24 hours. The carrier material is finally washed three times each for 15 minutes using 2X SSC, 0.2% SDS at 70°C.

**Very low stringency conditions:** The term "very low stringency conditions" means for probes of at least 100 nucleotides in length, prehybridization and hybridization at 42°C in 5X SSPE, 0.3% SDS, 200 micrograms/ml sheared and denatured salmon sperm DNA, and 25% formamide, following standard Southern blotting procedures for 12 to 24 hours. The carrier material is finally washed three times each for 15 minutes using 2X SSC, 0.2% SDS at 45°C.

**Xylan-containing material:** The term "xylan-containing material" means any material comprising a plant cell wall polysaccharide containing a backbone of beta-(1-4)-linked xylose residues. Xylans of terrestrial plants are heteropolymers possessing a beta-(1-4)-D-xylopyranose backbone, which is branched by short carbohydrate chains. They comprise D-glucuronic acid or its 4-*O*-methyl ether, L-arabinose, and/or various oligosaccharides, composed of D-xylose, L-arabinose, D- or L-galactose, and D-glucose. Xylan-type polysaccharides can be divided into homoxylans and heteroxylans, which include glucuronoxylans, (arabino)glucuronoxylans, (glucurono)arabinoxylans, arabinoxylans, and complex heteroxylans. See, for example, Ebringerova et al., 2005, Adv. Polym. Sci. 186: 1-67.

In the methods of the present invention, any material containing xylan may be used. In a preferred aspect, the xylan-containing material is lignocellulose.

**Xylan degrading activity or xylanolytic activity:** The term "xylan degrading activity" or "xylanolytic activity" means a biological activity that hydrolyzes xylan-containing material. The two basic approaches for measuring xylanolytic activity include: (1) measuring the total xylanolytic activity, and (2) measuring the individual xylanolytic activities (*e.g.,* endoxylanases, beta-xylosidases, arabinofuranosidases, alpha-glucuronidases, acetylxylan esterases, feruloyl esterases, and alpha-glucuronyl esterases). Recent progress in assays of xylanolytic enzymes was summarized in several publications including Biely and Puchard, 2006, Recent progress in the assays of xylanolytic enzymes, Journal of the Science of Food and Agriculture 86(11): 1636-1647; Spanikova and Biely, 2006, Glucuronoyl esterase - Novel carbohydrate esterase produced by Schizophyllum commune, FEBS Letters 580(19): 4597-4601; Herrmann, Vrsanska, Jurickova, Hirsch, Biely, and Kubicek, 1997, The beta-D-xylosidase of Trichoderma reesei is a multifunctional beta-D-xylan xylohydrolase, Biochemical Journal 321: 375-381.

Total xylan degrading activity can be measured by determining the reducing sugars formed from various types of xylan, including, for example, oat spelt, beechwood, and larchwood xylans, or by photometric determination of dyed xylan fragments released from various covalently dyed xylans. The most common total xylanolytic activity assay is based on production of reducing sugars from polymeric 4-O-methyl glucuronoxylan as described in Bailey, Biely, Poutanen, 1992, Interlaboratory testing of methods for assay of xylanase activity, Journal of Biotechnology 23(3): 257-270. Xylanase activity can also be determined with 0.2% AZCL-arabinoxylan as substrate in 0.01% TRITON® X-100 (4-(1,1,3,3-tetramethylbutyl)phenyl-polyethylene glycol) and 200 mM sodium phosphate buffer pH 6 at 37°C. One unit of xylanase activity is defined as 1.0 μmole of azurine produced per minute at 37°C, pH 6 from 0.2% AZCL-arabinoxylan as substrate in 200 mM sodium phosphate pH 6 buffer.

For purposes of the present invention, xylan degrading activity is determined by measuring the increase in hydrolysis of birchwood xylan (Sigma Chemical Co., Inc., St. Louis, MO, USA) by xylan-degrading enzyme(s) under the following typical conditions: 1 ml reactions, 5 mg/ml substrate (total solids), 5 mg of xylanolytic protein/g of substrate, 50 mM sodium acetate pH 5, 50°C, 24 hours, sugar analysis using p-hydroxybenzoic acid hydrazide (PHBAH) assay as described by Lever, 1972, A new reaction for colorimetric determination of carbohydrates, Anal. Biochem 47: 273-279.

**Xylanase:** The term "xylanase" means a 1,4-beta-D-xylan-xylohydrolase (E.C. 3.2.1.8) that catalyzes the endohydrolysis of 1,4-beta-D-xylosidic linkages in xylans. For purposes of the present invention, xylanase activity is determined with 0.2% AZCL-arabinoxylan as substrate in 0.01 % TRITON® X-100 and 200 mM sodium phosphate buffer pH 6 at 37°C. One unit of xylanase activity is defined as 1.0 μmole of azurine produced per minute at 37°C, pH 6 from 0.2% AZCL-arabinoxylan as substrate in 200 mM sodium phosphate pH 6 buffer.

*Wild-type polypeptide:* The term "wild-type polypeptide" means an enzyme expressed by a naturally occurring microorganism, such as a bacterium, yeast, or filamentous fungus found in nature.

## *Detailed Description of the* **Invention**

The present invention relates to isolated chimeric polypeptides having beta-glucosidase activity, comprising:

(a) a first polypeptide fragment at the N-terminal end of the chimeric polypeptide selected from the group consisting of (i) a polypeptide fragment having at least 90% identity to amino acids 20 to 404 of SEQ ID NO: 2; and (ii) a polypeptide fragment comprising or consisting of amino acids 20 to 404 of SEQ ID NO: 2;
(b) a second polypeptide fragment at the C-terminal end of the first polypeptide fragment selected from the group consisting of (i) a polypeptide fragment having at least 90% identity to amino acids 406 to 589 of SEQ ID NO: 4; and (ii) a polypeptide fragment comprising or consisting of amino acids 406 to 589 of SEQ ID NO: 4; and
(c) a third polypeptide fragment at the C-terminal end of the second polypeptide fragment selected from the group consisting of (i) a polypeptide fragment having at least 90% identity to amino acids 589 to 860 of SEQ ID NO: 2; and (ii) a polypeptide fragment comprising or consisting of amino acids 589 to 860 of SEQ ID NO: 2, wherein said chimeric polypeptide has increased specific activity compared to both the mature polypeptide of SEQ ID NO: 2 and the mature polypeptide of SEQ ID NO: 4.

A chimeric polypeptide according to the present invention has increased specific activity compared to the mature polypeptide of SEQ ID NO: 2, the mature polypeptide of SEQ ID NO: 4, or both the mature polypeptide of SEQ ID NO: 2 and the mature polypeptide of SEQ ID NO: 4.

In a first aspect, the first polypeptide fragment at the N-terminal end of the chimeric polypeptide has a sequence identity to amino acids 20 to 404 of SEQ ID NO: 2 of at least 60%, *e.g.,* at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100%. In one aspect, the first polypeptide fragment differs by up to 10 amino acids, e.g., by 1, 2, 3, 4, 5, 6, 7, 8, 9, or 10 amino acids from amino acids 20 to 404 of SEQ ID NO: 2.

In another aspect, the first polypeptide fragment is at least 240 amino acids, *e.g.,* at least 260, at least 280, at least 300, at least 320, at least 340, at least 360 amino acids, at least 370 amino acids, or at least 380 amino acids. In another aspect, the first polypeptide fragment is 385 amino acids.

In another aspect, the first polypeptide fragment comprises or consists of amino acids 20 to 404 of SEQ ID NO: 2 or an allelic variant thereof; or is a fragment thereof. In another aspect, the first polypeptide fragment comprises or consists of amino acids 20 to 404 of SEQ ID NO: 2.

In another first aspect, the second polypeptide fragment at the C-terminal end of the first polypeptide fragment has a sequence identity to amino acids 406 to 589 of SEQ ID NO: 4 of at least 60%, *e.g.,* at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100%. In one aspect, the second polypeptide fragment differs by up to 10 amino acids, *e.g.,* by 1, 2, 3, 4, 5, 6, 7, 8, 9, or 10 amino acids from amino acids 406 to 589 of SEQ ID NO: 4.

In another aspect, the second polypeptide fragment is at least 100 amino acids, *e.g.,* at least 110, at least 120, at least 130, at least 140, at least 150, at least 160, at least 170 amino acids, or at least 180 amino acids. In another aspect, the second polypeptide fragment is 184 amino acids.

In another aspect, the second polypeptide fragment comprises or consists of amino acids 406 to 589 of SEQ ID NO: 4 or an allelic variant thereof; or is a fragment thereof. In another aspect, the second polypeptide fragment comprises or consists of amino acids 406 to 589 of SEQ ID NO: 4.

In another first aspect, the third polypeptide fragment at the C-terminal end of the second polypeptide fragment has a sequence identity to amino acids 589 to 860 of SEQ ID NO: 2 of at least 60%, *e.g.,* at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100%. In one aspect, the third polypeptide fragment differs by up to 10 amino acids, e.g., by 1, 2, 3, 4, 5, 6, 7, 8, 9, or 10 amino acids from amino acids 589 to 860 of SEQ ID NO: 2.

In another aspect, the third polypeptide fragment is at least 160 amino acids, *e.g.,* at least 170, at least 180, at least 190, at least 200, at least 210, at least 220, at least 230, at least 240, at least 250, at least 260 amino acids, or at least 270 amino acids. In another aspect, the third polypeptide fragment is 272 amino acids.

In another aspect, the third polypeptide fragment comprises or consists of amino acids 589 to 860 of SEQ ID NO: 2 or an allelic variant thereof; or is a fragment thereof. In another aspect, the third polypeptide fragment comprises or consists of amino acids 589 to 860 of SEQ ID NO: 2.

In a second aspect, the first polypeptide fragment is encoded by a polynucleotide that hybridizes under very low stringency conditions, low stringency conditions, medium stringency conditions, medium-high stringency conditions, high stringency conditions, or very high stringency conditions with (i) nucleotides 131 to 1455 of SEQ ID NO: 1, (ii) the cDNA sequence thereof, or (iii) the full-length complement of (i) or (ii) (Sambrook *et al.*, 1989, *Molecular Cloning, A Laboratory Manual,* 2d edition, Cold Spring Harbor, New York).

In another second aspect, the second polypeptide fragment is encoded by a polynucleotide that hybridizes under very low stringency conditions, low stringency conditions, medium stringency conditions, medium-high stringency conditions, high stringency conditions, or very high stringency conditions with (i) nucleotides 1570 to 2184 of SEQ ID NO: 3, (ii) the cDNA sequence thereof, or (iii) the full-length complement of (i) or (ii) (Sambrook *et al.*, 1989, *supra*).

In another second aspect, the third polypeptide fragment is encoded by a polynucleotide that hybridizes under very low stringency conditions, low stringency conditions, medium stringency conditions, medium-high stringency conditions, high stringency conditions, or very high stringency conditions with (i) nucleotides 2072 to 2937 of SEQ ID NO: 1, (ii) the cDNA sequence thereof, or (iii) the full-length complement of (i) or (ii) (Sambrook *et al.*, 1989, *supra)*.

Nucleotides 131 to 1455 of SEQ ID NO: 1, nucleotides 1570 to 2184 of SEQ ID NO: 3, or nucleotides 2072 to 2937 of SEQ ID NO: 1, or subsequences thereof, as well as amino acids 20 to 404 of SEQ ID NO: 2, amino acids 406 to 589 of SEQ ID NO: 4, or amino acids 589 to 860 of SEQ ID NO: 2, or fragments thereof, may be used to design nucleic acid probes to identify and clone DNA encoding polypeptides having beta-glucosidase activity from strains of different genera or species according to methods well known in the art. In particular, such probes can be used for hybridization with the genomic DNA or cDNA of a cell of interest, following standard Southern blotting procedures, in order to identify and isolate the corresponding gene therein. Such probes can be considerably shorter than the entire sequence, but should be at least 15, *e.g.,* at least 25, at least 35, or at least 70 nucleotides in length. Preferably, the nucleic acid probe is at least 100 nucleotides in length, *e.g.,* at least 200 nucleotides, at least 300 nucleotides, at least 400 nucleotides, at least 500 nucleotides, at least 600 nucleotides, at least 700 nucleotides, at least 800 nucleotides, or at least 900 nucleotides in length. Both DNA and RNA probes can be used. The probes are typically labeled for detecting the corresponding gene (for example, with $^{32}P$, $^{3}H$, $^{35}S$, biotin, or avidin). Such probes are encompassed by the present disclosure.

A genomic DNA or cDNA library prepared from such other strains may be screened for DNA that hybridizes with the probes described above and encodes a polypeptide having beta-glucosidase activity. Genomic or other DNA from such other strains may be separated by agarose or polyacrylamide gel electrophoresis, or other separation techniques. DNA from the libraries or the separated DNA may be transferred to and immobilized on nitrocellulose or other suitable carrier material. In order to identify a clone or DNA, the carrier material is preferably used in a Southern blot.

For purposes of the present disclosure, hybridization indicates that the polynucleotide hybridizes to a labeled nucleic acid probe corresponding to nucleotides 131 to 1455 of SEQ ID NO: 1 or the cDNA sequence thereof; nucleotides 1570 to 2184 of SEQ ID NO: 3 or the cDNA sequence thereof; or nucleotides 2072 to 2937 of SEQ ID NO: 1 or the cDNA sequence thereof; or full-length complements thereof; or subsequences thereof; under very low to very high stringency conditions. Molecules to which the nucleic acid probe hybridizes under these conditions can be detected using, for example, X-ray film or any other detection means known in the art.

In one aspect, the nucleic acid probe is nucleotides 131 to 1455 of SEQ ID NO: 1 or the cDNA sequence thereof. In another aspect, the nucleic acid probe is nucleotides 1570 to 2184 of SEQ ID NO: 3 or the cDNA sequence thereof. In another aspect, the nucleic acid probe is nucleotides 2072 to 2937 of SEQ ID NO: 1 or the cDNA sequence thereof. In another aspect, the nucleic acid probe is a polynucleotide that encodes amino acids 20 to 404 of SEQ ID NO: 2 or a fragment thereof. In another aspect, the nucleic acid probe is a polynucleotide that encodes amino acids 406 to 589 of SEQ ID NO: 4 or a fragment thereof. In another aspect, the nucleic acid probe is a polynucleotide that encodes amino acids 589 to 860 of SEQ ID NO: 2 or a fragment thereof.

In a third aspect, the first polypeptide fragment is encoded by a polynucleotide having a sequence identity to nucleotides 131 to 1455 of SEQ ID NO: 1 or the cDNA sequence thereof of at least 60%, *e.g.,* at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100%.

In another third aspect, the second polypeptide fragment is encoded by a polynucleotide having a sequence identity to nucleotides 1570 to 2184 of SEQ ID NO: 3 or the cDNA sequence thereof of at least 60%, *e.g.,* at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100%.

In another third aspect, the third polypeptide fragment is encoded by a polynucleotide having a sequence identity to nucleotides 2072 to 2937 of SEQ ID NO: 1 or the cDNA sequence thereof of at least 60%, *e.g.,* at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100%.

In one aspect, the chimeric beta-glucosidase comprises or consists of SEQ ID NO: 6 or the mature polypeptide thereof. In another aspect, the chimeric beta-glucosidase comprises or consists of amino acids 20 to 860 of SEQ ID NO: 6. In another aspect, the chimeric beta-glucosidase is encoded by the polynucleotide of SEQ ID NO: 5 or the mature polypeptide thereof, or the cDNA sequence thereof. In another aspect, the chimeric polypeptide beta-glucosidase is encoded by nucleotides 131 to 2936 of SEQ ID NO: 5 or the cDNA sequence thereof. In each aspect above, the mature chimeric polypeptide may further comprise a signal peptide. In one aspect, the signal peptide is the signal peptide of SEQ ID NO: 2. In another aspect, the signal peptide is amino acids 1 to 19 of SEQ ID NO: 2. In another aspect, the signal peptide is the signal peptide of SEQ ID NO: 4. In another aspect, the signal peptide is amino acids 1 to 19 of SEQ ID NO: 4.

In another aspect, the chimeric polypeptides of the present invention comprise a substitution at one or both positions corresponding to positions 455 and 511 of the mature polypeptide of SEQ ID NO: 6, wherein the chimeric beta-glucosidase variants have beta-glucosidase activity.

In describing the chimeric beta-glucosidase variants of the present invention, the nomenclature described below is adapted for ease of reference. The accepted IUPAC single letter or three letter amino acid abbreviation is employed.

Substitutions. For an amino acid substitution, the following nomenclature is used: Original amino acid, position, substituted amino acid. Accordingly, the substitution of threonine at position 226 with alanine is designated as "Thr226Ala" or "T226A". Multiple mutations are separated by addition marks ("+"), *e.g.,* "Gly205Arg + Ser411 Phe" or "G205R + S411 F", representing substitutions at positions 205 and 411 of glycine (G) with arginine (R) and serine (S) with phenylalanine (F), respectively.

Deletions. For an amino acid deletion, the following nomenclature is used: Original amino acid, position, *. Accordingly, the deletion of glycine at position 195 is designated as "Gly195*" or "G195*". Multiple deletions are separated by addition marks ("+"), *e.g.,* "Gly195* + Ser411*" or "G195* + S411*".

Insertions. For an amino acid insertion, the following nomenclature is used: Original amino acid, position, original amino acid, inserted amino acid. Accordingly the insertion of lysine after glycine at position 195 is designated "Gly195GlyLys" or "G195GK". An insertion of multiple amino acids is designated [Original amino acid, position, original amino acid, inserted amino acid #1, inserted amino acid #2; etc.]. For example, the insertion of lysine and alanine after glycine at position 195 is indicated as "Gly195GlyLysAla" or "G195GKA".

In such cases the inserted amino acid residue(s) are numbered by the addition of lower case letters to the position number of the amino acid residue preceding the inserted amino acid residue(s). In the above example, the sequence would thus be:

| Parent: | Variant: |
|---|---|
| 195 | 195 195a 195b |
| G | G - K - A |

Multiple alterations. Variants comprising multiple alterations are separated by addition marks ("+"), *e.g.,* "Arg170Tyr+Gly195Glu" or "R170Y+G195E" representing a substitution of arginine and glycine at positions 170 and 195 with tyrosine and glutamic acid, respectively.

Different alterations. Where different alterations can be introduced at a position, the different alterations are separated by a comma, *e.g.,* "Arg170Tyr,Glu" represents a substitution of arginine at position 170 with tyrosine or glutamic acid. Thus, "Tyr167Gly,Ala + Arg170Gly,Ala" designates the following variants:

"Tyr167Gly+Arg170Gly", "Tyr167Gly+Arg170Ala", "Tyr167Ala+Arg170Gly", and "Tyr167Ala+Arg170Ala".

In an aspect, the chimeric beta-glucosidase variants have a sequence identity of at least 60%, *e.g.,* at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99%, but less than 100%, to the amino acid sequence of the parent polypeptide having beta-glucosidase activity.

In another aspect, the chimeric beta-glucosidase variants have at least 60%, e.g., at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, such as at least 96%, at least 97%, at least 98%, or at least 99%, but less than 100%, sequence identity to the mature polypeptide of SEQ ID NO: 6.

In another aspect, the number of substitutions in the chimeric beta-glucosidase variants of the present invention is 1 substitution. In another aspect, the number of substitutions in the chimeric beta-glucosidase variants of the present invention is 2 substitutions

In another aspect, a chimeric beta-glucosidase variant comprises a substitution at one of the positions corresponding to positions 455 and 511. In another aspect, a variant comprises a substitution at each position corresponding to positions 455 and 511.

In another aspect, a chimeric beta-glucosidase variant comprises or consists of a substitution at a position corresponding to position 455. In another aspect, the amino acid at a position corresponding to position 455 is substituted with Ala, Arg, Asn, Asp, Cys, Gln, Glu, Gly, His, Ile, Leu, Lys, Met, Phe, Pro, Ser, Thr, Trp, Tyr, or Val, preferably with Glu. In another aspect, the variant comprises or consists of the substitution N455E of the mature polypeptide of SEQ ID NO: 6.

In another aspect, a chimeric beta-glucosidase variant comprises or consists of a substitution at a position corresponding to position 511. In another aspect, the amino acid at a position corresponding to position 511 is substituted with Ala, Arg, Asn, Asp, Cys, Gln, Glu, Gly, His, Ile, Leu, Lys, Met, Phe, Pro, Ser, Thr, Trp, Tyr, or Val, preferably with Tyr. In another aspect, the variant comprises or consists of the substitution F511Y of the mature polypeptide of SEQ ID NO: 6.

In another aspect, a chimeric beta-glucosidase variant comprises or consists of a substitution at positions corresponding to positions 455 and 511, such as those described above.

In another aspect, a chimeric beta-glucosidase variant comprises or consists of one or both of the substitutions N455E and F511Y.

In another aspect, the chimeric beta-glucosidase variant comprises or consists of the substitutions N455E + F511Y of the mature polypeptide of SEQ ID NO: 6.

In another aspect, the DNA sequence and deduced amino acid sequence of a chimeric beta-glucosidase variant are shown in SEQ ID NO: 7 and SEQ ID NO: 8, respectively.

In another aspect, the chimeric beta-glucosidase variant comprises or consists of SEQ ID NO: 8 or the mature polypeptide thereof. In another aspect, the chimeric beta-glucosidase variant comprises or consists of amino acids 20 to 860 of SEQ ID NO: 8. In another aspect, the chimeric beta-glucosidase variant is encoded by the polynucleotide of SEQ ID NO: 7 or the mature polypeptide coding sequence thereof, or the cDNA sequence thereof. In another aspect, the chimeric beta-glucosidase variant is encoded by nucleotides 131 to 2936 of SEQ ID NO: 7 or the cDNA sequence thereof. In each aspect above, the mature chimeric beta-glucosidase variant may further comprise a signal peptide. In one aspect, the signal peptide is the signal peptide of SEQ ID NO: 2. In another aspect, the signal peptide is amino acids 1 to 19 of SEQ ID NO: 2. In another aspect, the signal peptide is the signal peptide of SEQ ID NO: 4. In another aspect, the signal peptide is amino acids 1 to 19 of SEQ ID NO: 4.

The chimeric beta-glucosidase variants of the present disclosure may further comprise an alteration, *e.g.,* deletion, insertion, and/or substitution, at one or more (*e.g.,* several) other positions.

The amino acid changes may be of a minor nature, that is conservative amino acid substitutions or insertions that do not significantly affect the folding and/or activity of the protein; small deletions, typically of 1-30 amino acids; small amino- or carboxyl-terminal extensions, such as an amino-terminal methionine residue; a small linker peptide of up to 20-25 residues; or a small extension that facilitates purification by changing net charge or another function, such as a poly-histidine tract, an antigenic epitope or a binding domain.

Examples of conservative substitutions are within the groups of basic amino acids (arginine, lysine and histidine), acidic amino acids (glutamic acid and aspartic acid), polar amino acids (glutamine and asparagine), hydrophobic amino acids (leucine, isoleucine and valine), aromatic amino acids (phenylalanine, tryptophan and tyrosine), and small amino acids (glycine, alanine, serine, threonine and methionine). Amino acid substitutions that do not generally alter specific activity are known in the art and are described, for example, by H. Neurath and R.L. Hill, 1979, In, The Proteins, Academic Press, New York. Common substitutions are Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu, and Asp/Gly.

Alternatively, the amino acid changes are of such a nature that the physico-chemical properties of the polypeptides are altered. For example, amino acid changes may improve the thermal stability of the polypeptide, alter the substrate specificity, change the pH optimum, and the like.

Essential amino acids in a polypeptide can be identified according to procedures known in the art, such as site-directed mutagenesis or alanine-scanning mutagenesis (Cunningham and Wells, 1989, Science 244: 1081-1085). In the latter technique, single alanine mutations are introduced at every residue in the molecule, and the resultant mutant molecules are tested for beta-glucosidase activity to identify amino acid residues that are critical to the activity of the molecule. See also, Hilton et al., 1996, J. Biol. Chem. 271: 4699-4708. The active site of the polypeptide having beta-glucosidase activity or other biological interaction can also be determined by physical analysis of structure, as determined by such techniques as nuclear magnetic resonance, crystallography, electron diffraction, or photoaffinity labeling, in conjunction with mutation of putative contact site amino acids. See, for example, de Vos et al., 1992, Science 255: 306-312; Smith et al., 1992, J. Mol. Biol. 224: 899-904; Wlodaver et al., 1992, FEBS Lett. 309: 59-64. The identity of essential amino acids can also be inferred from an alignment with a related polypeptide.

The chimeric beta-glucosidase variants may consist of 720 to 857 amino acids, *e.g.,* 720 to 739, 740 to 759, 760 to 779, 780 to 799, 800 to 819, and 820 to 841 amino acids.

In an embodiment, the chimeric beta-glucosidase variant has increased specific activity compared to the mature polypeptide of SEQ ID NO: 2 and SEQ ID NO: 4.

The parent beta-glucosidase may be (a) a polypeptide having at least 60% sequence identity to the mature polypeptide of SEQ ID NO: 6; (b) a polypeptide encoded by a polynucleotide that hybridizes under low stringency conditions with (i) the mature polypeptide coding sequence of SEQ ID NO: 5, (ii) the cDNA sequence thereof, or (iii) the full-length complement of (i) or (ii); or (c) a polypeptide encoded by a polynucleotide having at least 60% sequence identity to the mature polypeptide coding sequence of SEQ ID NO: 5 or the cDNA sequence thereof.

In a first aspect, the parent has a sequence identity to the mature polypeptide of SEQ ID NO: 6 of at least 60%, *e.g.,* at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100%, which has beta-glucosidase activity. In one aspect, the amino acid sequence of the parent differs by up to 10 amino acids, *e.g.,* by 1, 2, 3, 4, 5, 6, 7, 8, 9, or 10 amino acids from the mature polypeptide of SEQ ID NO: 6.

The parent preferably comprises or consists of the amino acid sequence of SEQ ID NO: 6. In another aspect, the parent comprises or consists of the mature polypeptide of SEQ ID NO: 6. In another aspect, the parent comprises or consists of amino acids 20 to 860 of SEQ ID NO: 6.

In an aspect, the parent is a fragment of the mature polypeptide of SEQ ID NO: 6 containing at least 720 amino acid residues, *e.g.,* at least 760 amino acid residues or at least 800 amino acid residues.

In another aspect, the parent is an allelic variant of the mature polypeptide of SEQ ID NO: 6.

In a second aspect, the parent is encoded by a polynucleotide that hybridizes under very low stringency conditions, low stringency conditions, medium stringency conditions, medium-high stringency conditions, high stringency conditions, or very high stringency conditions with (i) the mature polypeptide coding sequence of SEQ ID NO: 5, (ii) the cDNA sequence thereof, or (iii) the full-length complement of (i) or (ii) (Sambrook *et al.,* 1989, *Molecular Cloning, A Laboratory Manual*, 2d edition, Cold Spring Harbor, New York).

The polynucleotide of SEQ ID NO: 5 or a subsequence thereof, as well as the polypeptide of SEQ ID NO: 6 or a fragment thereof, may be used to design nucleic acid probes to identify and clone DNA encoding a parent from a genomic DNA or cDNA library as described herein.

In one aspect, the nucleic acid probe is the mature polypeptide coding sequence of SEQ ID NO: 5 or the cDNA sequence thereof. In another aspect, the nucleic acid probe is nucleotides 131 to 2936 of SEQ ID NO: 5. In another aspect, the nucleic acid probe is a polynucleotide that encodes the polypeptide of SEQ ID NO: 6 or a fragment thereof. In another aspect, the nucleic acid probe is SEQ ID NO: 5 or the cDNA sequence thereof.

In a third aspect, the parent is encoded by a polynucleotide having a sequence identity to the mature polypeptide coding sequence of SEQ ID NO: 5 or the cDNA sequence thereof of at least 60%, e.g., at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100%, which encodes a polypeptide having beta-glucosidase activity. In one aspect, the mature polypeptide coding sequence is nucleotides 131 to 2936 of SEQ ID NO: 5 or the cDNA sequence thereof. In an aspect, the parent is encoded by a polynucleotide comprising or consisting of SEQ ID NO: 5, the mature polypeptide coding sequence thereof, or the cDNA sequence thereof.

The parent may be obtained from microorganisms of any genus. For purposes of the present invention, the term "obtained from" as used herein in connection with a given source shall mean that the parent encoded by a polynucleotide is produced by the source or by a cell in which the polynucleotide from the source has been inserted. In one aspect, the parent is secreted extracellularly.

**Polynucleotides**

The present invention also relates to isolated polynucleotides that encode any of the chimeric polypeptides of the present invention.

**Nucleic Acid Constructs**

The present disclosure also relates to nucleic acid constructs comprising a polynucleotide encoding a chimeric beta-glucosidase or a variant thereof of the present invention operably linked to one or more control sequences that direct the expression of the coding sequence in a suitable host cell under conditions compatible with the control sequences.

The polynucleotide may be manipulated in a variety of ways to provide for expression of the chimeric beta-glucosidase or the variant thereof. Manipulation of the polynucleotide prior to its insertion into a vector may be desirable or necessary depending on the expression vector. The techniques for modifying polynucleotides utilizing recombinant DNA methods are well known in the art.

The control sequence may be a promoter, a polynucleotide that is recognized by a host cell for expression of the polynucleotide. The promoter contains transcriptional control sequences that mediate the expression of the chimeric beta-glucosidase or the variant thereof. The promoter may be any polynucleotide that shows transcriptional activity in the host cell including mutant, truncated, and hybrid promoters, and may be obtained from genes encoding extracellular or intracellular polypeptides either homologous or heterologous to the host cell.

Examples of suitable promoters for directing transcription of the nucleic acid constructs of the present disclosure in a bacterial host cell are the promoters obtained from the *Bacillus amyloliquefaciens* alpha-amylase gene (*amyQ*), *Bacillus licheniformis* alpha-amylase gene (*amyL*), *Bacillus licheniformis* penicillinase gene (*penP*), *Bacillus stearothermophilus* maltogenic amylase gene (*amyM*), *Bacillus subtilis* levansucrase gene (*sacB*), *Bacillus subtilis xylA* and *xylB* genes, *Bacillus thuringiensis cryIIIA* gene (Agaisse and Lereclus, 1994, Molecular Microbiology 13: 97-107), *E. coli lac* operon, *E. coli trc* promoter (Egon et al., 1988, Gene 69: 301-315), *Streptomyces coelicolor* agarase gene (*dagA*), and prokaryotic beta-lactamase gene (Villa-Kamaroff et al., 1978, Proc. Natl. Acad. Sci. USA 75: 3727-3731), as well as the *tac* promoter (DeBoer et al., 1983, Proc. Natl. Acad. Sci. USA 80: 21-25). Further promoters are described in "Useful proteins from recombinant bacteria" in Gilbert et al., 1980, Scientific American 242: 74-94; and in Sambrook *et al.,* 1989, *supra.* Examples of tandem promoters are disclosed in WO 99/43835.

Examples of suitable promoters for directing transcription of the nucleic acid constructs of the present disclosure in a filamentous fungal host cell are promoters obtained from the genes for *Aspergillus nidulans* acetamidase, *Aspergillus niger* neutral alpha-amylase, *Aspergillus niger* acid stable alpha-amylase, *Aspergillus niger* or *Aspergillus awamori* glucoamylase (*glaA*), *Aspergillus oryzae* TAKA amylase, *Aspergillus oryzae* alkaline protease, *Aspergillus oryzae* triose phosphate isomerase, *Fusarium oxysporum* trypsin-like protease (WO 96/00787), *Fusarium venenatum* amyloglucosidase (WO 00/56900), *Fusarium venenatum* Daria (WO 00/56900), *Fusarium venenatum* Quinn (WO 00/56900), *Rhizomucor miehei* lipase, *Rhizomucor miehei* aspartic proteinase, *Trichoderma reesei* beta-glucosidase, *Trichoderma reesei* cellobiohydrolase I, *Trichoderma reesei* cellobiohydrolase II, *Trichoderma reesei* endoglucanase I, *Trichoderma reesei* endoglucanase II, *Trichoderma reesei* endoglucanase III, *Trichoderma reesei* endoglucanase V, *Trichoderma reesei* xylanase I, *Trichoderma reesei* xylanase II, *Trichoderma reesei* xylanase III, *Trichoderma reesei* beta-xylosidase, and *Trichoderma reesei* translation elongation factor, as well as the NA2-tpi promoter (a modified promoter from an *Aspergillus* neutral alpha-amylase gene in which the untranslated leader has been replaced by an untranslated leader from an *Aspergillus* triose phosphate isomerase gene; non-limiting examples include modified promoters from an *Aspergillus niger* neutral alpha-amylase gene in which the untranslated leader has been replaced by an untranslated leader from an *Aspergillus nidulans* or *Aspergillus oryzae* triose phosphate isomerase gene); and mutant, truncated, and hybrid promoters thereof. Other promoters are described in U.S. Patent No. 6,011,147.

In a yeast host, useful promoters are obtained from the genes for *Saccharomyces cerevisiae* enolase (ENO-1), *Saccharomyces cerevisiae* galactokinase (GAL1), *Saccharomyces cerevisiae* alcohol dehydrogenase/glyceraldehyde-3-phosphate dehydrogenase (ADH1, ADH2/GAP), *Saccharomyces cerevisiae* triose phosphate isomerase (TPI), *Saccharomyces cerevisiae* metallothionein (CUP1), and *Saccharomyces cerevisiae* 3-phosphoglycerate kinase. Other useful promoters for yeast host cells are described by Romanos et al., 1992, Yeast 8: 423-488.

The control sequence may also be a transcription terminator, which is recognized by a host cell to terminate transcription. The terminator is operably linked to the 3'-terminus of the polynucleotide encoding the chimeric beta-glucosidase or the variant thereof. Any terminator that is functional in the host cell may be used in the present disclosure.

Preferred terminators for bacterial host cells are obtained from the genes for *Bacillus clausii* alkaline protease (*aprH*), *Bacillus licheniformis* alpha-amylase (*amyL*), and *Escherichia coli* ribosomal RNA (*rrnB*).

Preferred terminators for filamentous fungal host cells are obtained from the genes for *Aspergillus nidulans* acetamidase, *Aspergillus nidulans* anthranilate synthase, *Aspergillus niger* glucoamylase, *Aspergillus niger* alpha-glucosidase, *Aspergillus oryzae* TAKA amylase, *Fusarium oxysporum* trypsin-like protease, *Trichoderma reesei* beta-glucosidase, *Trichoderma reesei* cellobiohydrolase I, *Trichoderma reesei* cellobiohydrolase II, *Trichoderma reesei* endoglucanase I, *Trichoderma reesei* endoglucanase II, *Trichoderma reesei* endoglucanase III, *Trichoderma reesei* endoglucanase V, *Trichoderma reesei* xylanase I, *Trichoderma reesei* xylanase II, *Trichoderma reesei* xylanase III, *Trichoderma reesei* beta-xylosidase, and *Trichoderma reesei* translation elongation factor.

Preferred terminators for yeast host cells are obtained from the genes for *Saccharomyces cerevisiae* enolase, *Saccharomyces cerevisiae* cytochrome C (CYC1), and *Saccharomyces cerevisiae* glyceraldehyde-3-phosphate dehydrogenase. Other useful terminators for yeast host cells are described by Romanos *et al.*, 1992, *supra.*

The control sequence may also be an mRNA stabilizer region downstream of a promoter and upstream of the coding sequence of a chimeric beta-glucosidase or a variant thereof of the present invention which increases expression of the chimeric beta-glucosidase or the variant thereof.

Examples of suitable mRNA stabilizer regions are obtained from a *Bacillus thuringiensis cryIIIA* gene (WO 94/25612) and a *Bacillus subtilis* SP82 gene (Hue et al., 1995, Journal of Bacteriology 177: 3465-3471).

The control sequence may also be a leader, a nontranslated region of an mRNA that is important for translation by the host cell. The leader is operably linked to the 5'-terminus of the polynucleotide encoding the chimeric beta-glucosidase or the variant thereof. Any leader that is functional in the host cell may be used.

Preferred leaders for filamentous fungal host cells are obtained from the genes for *Aspergillus oryzae* TAKA amylase and *Aspergillus nidulans* triose phosphate isomerase.

Suitable leaders for yeast host cells are obtained from the genes for *Saccharomyces cerevisiae* enolase (ENO-1), *Saccharomyces cerevisiae* 3-phosphoglycerate kinase, *Saccharomyces cerevisiae* alpha-factor, and *Saccharomyces cerevisiae* alcohol dehydrogenase/glyceraldehyde-3-phosphate dehydrogenase (ADH2/GAP).

The control sequence may also be a polyadenylation sequence, a sequence operably linked to the 3'-terminus of the chimeric beta-glucosidase or the variant thereof coding sequence and, when transcribed, is recognized by the host cell as a signal to add polyadenosine residues to transcribed mRNA. Any polyadenylation sequence that is functional in the host cell may be used.

Preferred polyadenylation sequences for filamentous fungal host cells are obtained from the genes for *Aspergillus nidulans* anthranilate synthase, *Aspergillus niger* glucoamylase, *Aspergillus niger* alpha-glucosidase, *Aspergillus oryzae* TAKA amylase, and *Fusarium oxysporum* trypsin-like protease.

Useful polyadenylation sequences for yeast host cells are described by Guo and Sherman, 1995, Mol. Cellular Biol. 15: 5983-5990.

The control sequence may also be a signal peptide coding region that encodes a signal peptide linked to the N-terminus of a chimeric beta-glucosidase or a variant thereof and directs the chimeric beta-glucosidase or the variant thereof into the cell's secretory pathway. The 5'-end of the coding sequence of the polynucleotide may inherently contain a signal peptide coding sequence naturally linked in translation reading frame with the segment of the coding sequence that encodes the chimeric beta-glucosidase or the variant thereof. Alternatively, the 5'-end of the coding sequence may contain a signal peptide coding sequence that is foreign to the coding sequence. A foreign signal peptide coding sequence may be required where the coding sequence does not naturally contain a signal peptide coding sequence. Alternatively, a foreign signal peptide coding sequence may simply replace the natural signal peptide coding sequence in order to enhance secretion of the chimeric beta-glucosidase or the variant thereof. However, any signal peptide coding sequence that directs the expressed chimeric beta-glucosidase or a variant thereof into the secretory pathway of a host cell may be used.

Effective signal peptide coding sequences for bacterial host cells are the signal peptide coding sequences obtained from the genes for *Bacillus* NCIB 11837 maltogenic amylase, *Bacillus licheniformis* subtilisin, *Bacillus licheniformis* beta-lactamase, *Bacillus stearothermophilus* alpha-amylase, *Bacillus stearothermophilus* neutral proteases (*nprT*, *nprS*, *nprM*), and *Bacillus subtilis prsA.* Further signal peptides are described by Simonen and Palva, 1993, Microbiological Reviews 57: 109-137.

Effective signal peptide coding sequences for filamentous fungal host cells are the signal peptide coding sequences obtained from the genes for *Aspergillus niger* neutral amylase, *Aspergillus niger* glucoamylase, *Aspergillus oryzae* TAKA amylase, *Humicola insolens* cellulase, *Humicola insolens* endoglucanase V, *Humicola lanuginosa* lipase, and *Rhizomucor miehei* aspartic proteinase.

Useful signal peptides for yeast host cells are obtained from the genes for *Saccharomyces cerevisiae* alpha-factor and *Saccharomyces cerevisiae* invertase. Other useful signal peptide coding sequences are described by Romanos *et al*., 1992, *supra.*

The control sequence may also be a propeptide coding sequence that encodes a propeptide positioned at the N-terminus of a chimeric beta-glucosidase or a variant thereof of the present invention. The resultant polypeptide is known as a proenzyme or propolypeptide (or a zymogen in some cases). A propolypeptide is generally inactive and can be converted to an active polypeptide by catalytic or autocatalytic cleavage of the propeptide from the propolypeptide. The propeptide coding sequence may be obtained from the genes for *Bacillus subtilis* alkaline protease (*aprE*), *Bacillus subtilis* neutral protease (*nprT*), *Myceliophthora thermophila* laccase (WO 95/33836), *Rhizomucor miehei* aspartic proteinase, and *Saccharomyces cerevisiae* alpha-factor.

Where both signal peptide and propeptide sequences are present, the propeptide sequence is positioned next to the N-terminus of the chimeric beta-glucosidase or the variant thereof and the signal peptide sequence is positioned next to the N-terminus of the propeptide sequence.

It may also be desirable to add regulatory sequences that regulate expression of the chimeric beta-glucosidase or the variant thereof relative to the growth of the host cell. Examples of regulatory sequences are those that cause expression of the gene to be turned on or off in response to a chemical or physical stimulus, including the presence of a regulatory compound. Regulatory sequences in prokaryotic systems include the *lac*, *tac*, and *trp* operator systems. In yeast, the ADH2 system or GAL1 system may be used. In filamentous fungi, the *Aspergillus niger* glucoamylase promoter, *Aspergillus oryzae* TAKA alpha-amylase promoter, and *Aspergillus oryzae* glucoamylase promoter, *Trichoderma reesei* cellobiohydrolase I promoter, and *Trichoderma reesei* cellobiohydrolase II promoter may be used. Other examples of regulatory sequences are those that allow for gene amplification. In eukaryotic systems, these regulatory sequences include the dihydrofolate reductase gene that is amplified in the presence of methotrexate, and the metallothionein genes that are amplified with heavy metals. In these cases, the polynucleotide encoding the chimeric beta-glucosidase or the variant thereof would be operably linked to the regulatory sequence.

**Expression Vectors**

The present disclosure also relates to recombinant expression vectors comprising a polynucleotide encoding a chimeric beta-glucosidase or a variant thereof of the present invention, a promoter, and transcriptional and translational stop signals. The various nucleotide and control sequences may be joined together to produce a recombinant expression vector that may include one or more (*e.g.*, several) convenient restriction sites to allow for insertion or substitution of the polynucleotide encoding the chimeric beta-glucosidase or the variant thereof at such sites. Alternatively, the polynucleotide may be expressed by inserting the polynucleotide or a nucleic acid construct comprising the polynucleotide into an appropriate vector for expression. In creating the expression vector, the coding sequence is located in the vector so that the coding sequence is operably linked with the appropriate control sequences for expression.

The recombinant expression vector may be any vector (*e.g.*, a plasmid or virus) that can be conveniently subjected to recombinant DNA procedures and can bring about expression of the polynucleotide. The choice of the vector will typically depend on the compatibility of the vector with the host cell into which the vector is to be introduced. The vector may be a linear or closed circular plasmid.

The vector may be an autonomously replicating vector, *i.e.*, a vector that exists as an extrachromosomal entity, the replication of which is independent of chromosomal replication, *e.g.*, a plasmid, an extrachromosomal element, a minichromosome, or an artificial chromosome. The vector may contain any means for assuring self-replication. Alternatively, the vector may be one that, when introduced into the host cell, is integrated into the genome and replicated together with the chromosome(s) into which it has been integrated. Furthermore, a single vector or plasmid or two or more vectors or plasmids that together contain the total DNA to be introduced into the genome of the host cell, or a transposon, may be used.

The vector preferably contains one or more (*e.g.*, several) selectable markers that permit easy selection of transformed, transfected, transduced, or the like cells. A selectable marker is a gene the product of which provides for biocide or viral resistance, resistance to heavy metals, prototrophy to auxotrophs, and the like.

Examples of bacterial selectable markers are *Bacillus licheniformis* or *Bacillus subtilis dal* genes, or markers that confer antibiotic resistance such as ampicillin, chloramphenicol, kanamycin, neomycin, spectinomycin, or tetracycline resistance. Suitable markers for yeast host cells include, but are not limited to, ADE2, HIS3, LEU2, LYS2, MET3, TRP1, and URA3. Selectable markers for use in a filamentous fungal host cell include, but are not limited to, *adeA* (phosphoribosylaminoimidazole-succinocarboxamide synthase), *adeB* (phosphoribosyl-aminoimidazole synthase), *amdS* (acetamidase), *argB* (ornithine carbamoyltransferase), *bar* (phosphinothricin acetyltransferase), *hph* (hygromycin phosphotransferase), *niaD* (nitrate reductase), *pyrG* (orotidine-5'-phosphate decarboxylase), sC (sulfate adenyltransferase), and *trpC* (anthranilate synthase), as well as equivalents thereof. Preferred for use in an *Aspergillus* cell are *Aspergillus nidulans* or *Aspergillus oryzae amdS* and *pyrG* genes and a *Streptomyces hygroscopicus bar* gene. Preferred for use in a *Trichoderma* cell are *adeA*, *adeB*, *amdS*, *hph*, and *pyrG* genes.

The selectable marker may be a dual selectable marker system as described in WO 2010/039889. In one aspect, the dual selectable marker is a *hph-tk* dual selectable marker system.

The vector preferably contains an element(s) that permits integration of the vector into the host cell's genome or autonomous replication of the vector in the cell independent of the genome.

For integration into the host cell genome, the vector may rely on the polynucleotide's sequence encoding the chimeric beta-glucosidase or the variant thereof or any other element of the vector for integration into the genome by homologous or non-homologous recombination. Alternatively, the vector may contain additional polynucleotides for directing integration by homologous recombination into the genome of the host cell at a precise location(s) in the chromosome(s). To increase the likelihood of integration at a precise location, the integrational elements should contain a sufficient number of nucleic acids, such as 100 to 10,000 base pairs, 400 to 10,000 base pairs, and 800 to 10,000 base pairs, which have a high degree of sequence identity to the corresponding target sequence to enhance the probability of homologous recombination. The integrational elements may be any sequence that is homologous with the target sequence in the genome of the host cell. Furthermore, the integrational elements may be non-encoding or encoding polynucleotides. On the other hand, the vector may be integrated into the genome of the host cell by non-homologous recombination.

For autonomous replication, the vector may further comprise an origin of replication enabling the vector to replicate autonomously in the host cell in question. The origin of replication may be any plasmid replicator mediating autonomous replication that functions in a cell. The term "origin of replication" or "plasmid replicator" means a polynucleotide that enables a plasmid or vector to replicate *in vivo.*

Examples of bacterial origins of replication are the origins of replication of plasmids pBR322, pUC19, pACYC177, and pACYC184 permitting replication in *E. coli*, and pUB110, pE194, pTA1060, and pAMß1 permitting replication in *Bacillus.*

Examples of origins of replication for use in a yeast host cell are the 2 micron origin of replication, ARS1, ARS4, the combination of ARS1 and CEN3, and the combination of ARS4 and CEN6.

Examples of origins of replication useful in a filamentous fungal cell are AMA1 and ANS1 (Gems et al., 1991, Gene 98: 61-67; Cullen et al., 1987, Nucleic Acids Res. 15: 9163-9175; WO 00/24883). Isolation of the AMA1 gene and construction of plasmids or vectors comprising the gene can be accomplished according to the methods disclosed in WO 00/24883.

More than one copy of a polynucleotide of the present invention may be inserted into a host cell to increase production of a chimeric beta-glucosidase or a variant thereof. An increase in the copy number of the polynucleotide can be obtained by integrating at least one additional copy of the sequence into the host cell genome or by including an amplifiable selectable marker gene with the polynucleotide where cells containing amplified copies of the selectable marker gene, and thereby additional copies of the polynucleotide, can be selected for by cultivating the cells in the presence of the appropriate selectable agent.

The procedures used to ligate the elements described above to construct the recombinant expression vectors of the present disclosure are well known to one skilled in the art (see, *e.g.*, Sambrook *et al.,* 1989, *supra*).

**Host Cells**

The present disclosure also relates to recombinant host cells, comprising a polynucleotide encoding a chimeric beta-glucosidase or a variant thereof of the present invention operably linked to one or more (e.g., several) control sequences that direct the production of the chimeric beta-glucosidase or the variant thereof. A construct or vector comprising a polynucleotide is introduced into a host cell so that the construct or vector is maintained as a chromosomal integrant or as a self-replicating extra-chromosomal vector as described earlier. The term "host cell" encompasses any progeny of a parent cell that is not identical to the parent cell due to mutations that occur during replication. The choice of a host cell will to a large extent depend upon the gene encoding the chimeric beta-glucosidase or the variant thereof and its source.

The host cell may be any cell useful in the recombinant production of a chimeric beta-glucosidase or a variant thereof, e.g., a prokaryote or a eukaryote.

The prokaryotic host cell may be any Gram-positive or Gram-negative bacterium. Gram-positive bacteria include, but are not limited to, *Bacillus*, *Clostridium*, *Enterococcus*, *Geobacillus, Lactobacillus, Lactococcus, Oceanobacillus, Staphylococcus, Streptococcus,* and *Streptomyces.* Gram-negative bacteria include, but are not limited to, *Campylobacter*, *E. coli, Flavobacterium, Fusobacterium, Helicobacter, Ilyobacter*, *Neisseria, Pseudomonas, Salmonella*, and *Ureaplasma.*

The bacterial host cell may be any *Bacillus* cell, including, but not limited to, *Bacillus alkalophilus*, *Bacillus amyloliquefaciens*, *Bacillus brevis, Bacillus circulans, Bacillus clausii, Bacillus coagulans, Bacillus firmus, Bacillus lautus, Bacillus lentus*, *Bacillus licheniformis*, *Bacillus megaterium*, *Bacillus pumilus*, *Bacillus stearothermophilus*, *Bacillus subtilis*, and *Bacillus thuringiensis* cells.

The bacterial host cell may also be any *Streptococcus* cell, including, but not limited to, *Streptococcus equisimilis*, *Streptococcus pyogenes*, *Streptococcus uberis*, and *Streptococcus equi* subsp. *Zooepidemicus* cells.

The bacterial host cell may also be any *Streptomyces* cell, including, but not limited to, *Streptomyces achromogenes*, *Streptomyces avermitilis*, *Streptomyces coelicolor*, *Streptomyces griseus*, and *Streptomyces lividans* cells.

The introduction of DNA into a *Bacillus* cell may be effected by protoplast transformation (see, *e.g.*, Chang and Cohen, 1979, Mol. Gen. Genet. 168: 111-115), competent cell transformation (see, *e.g.*, Young and Spizizen, 1961, J. Bacteriol. 81: 823-829, or Dubnau and Davidoff-Abelson, 1971, J. Mol. Biol. 56: 209-221), electroporation (see, *e.g.*, Shigekawa and Dower, 1988, Biotechniques 6: 742-751), or conjugation (see, *e.g.*, Koehler and Thorne, 1987, J. Bacteriol. 169: 5271-5278). The introduction of DNA into an *E. coli* cell may be effected by protoplast transformation (see, *e.g.*, Hanahan, 1983, J. Mol. Biol. 166: 557-580) or electroporation (see, *e.g.*, Dower et al., 1988, Nucleic Acids Res. 16: 6127-6145). The introduction of DNA into a *Streptomyces* cell may be effected by protoplast transformation, electroporation (see, *e.g.*, Gong et al., 2004, Folia Microbiol. (Praha) 49: 399-405), conjugation (see, *e.g.*, Mazodier et al., 1989, J. Bacteriol. 171: 3583-3585), or transduction (see, *e.g.*, Burke et al., 2001, Proc. Natl. Acad. Sci. USA 98: 6289-6294). The introduction of DNA into a *Pseudomonas* cell may be effected by electroporation (see, *e.g.*, Choi et al., 2006, J. Microbiol. Methods 64: 391-397), or conjugation (see, *e.g.*, Pinedo and Smets, 2005, Appl. Environ. Microbiol. 71: 51-57). The introduction of DNA into a *Streptococcus* cell may be effected by natural competence (see, *e.g.*, Perry and Kuramitsu, 1981, Infect. Immun. 32: 1295-1297), protoplast transformation (see, *e.g.*, Catt and Jollick, 1991, Microbios 68: 189-207), electroporation (see, *e.g.*, Buckley et al., 1999, Appl. Environ. Microbiol. 65: 3800-3804) or conjugation (see, *e.g.*, Clewell, 1981, Microbiol. Rev. 45: 409-436). However, any method known in the art for introducing DNA into a host cell can be used.

The host cell may also be a eukaryote, such as a mammalian, insect, plant, or fungal cell.

The host cell may be a fungal cell. "Fungi" as used herein includes the phyla Ascomycota, Basidiomycota, Chytridiomycota, and Zygomycota as well as the Oomycota and all mitosporic fungi (as defined by Hawksworth et al., In, Ainsworth and Bisby's Dictionary of The Fungi, 8th edition, 1995, CAB International, University Press, Cambridge, UK).

The fungal host cell may be a yeast cell. "Yeast" as used herein includes ascosporogenous yeast (Endomycetales), basidiosporogenous yeast, and yeast belonging to the Fungi Imperfecti (Blastomycetes). Since the classification of yeast may change in the future, for the purposes of this invention, yeast shall be defined as described in Biology and Activities of Yeast (Skinner, Passmore, and Davenport, editors, Soc. App. Bacteriol. Symposium Series No. 9, 1980).

The yeast host cell may be a *Candida*, *Hansenula*, *Kluyveromyces*, *Pichia*, *Saccharomyces*, *Schizosaccharomyces*, or *Yarrowia* cell such as a *Kluyveromyces lactis*, *Saccharomyces carlsbergensis, Saccharomyces cerevisiae*, *Saccharomyces diastaticus*, *Saccharomyces douglasii, Saccharomyces kluyveri*, *Saccharomyces norbensis*, *Saccharomyces oviformis*, or *Yarrowia lipolytica* cell.

The fungal host cell may be a filamentous fungal cell. "Filamentous fungi" include all filamentous forms of the subdivision Eumycota and Oomycota (as defined by Hawksworth *et al*., 1995, *supra*). The filamentous fungi are generally characterized by a mycelial wall composed of chitin, cellulose, glucan, chitosan, mannan, and other complex polysaccharides. Vegetative growth is by hyphal elongation and carbon catabolism is obligately aerobic. In contrast, vegetative growth by yeasts such as *Saccharomyces cerevisiae* is by budding of a unicellular thallus and carbon catabolism may be fermentative.

The filamentous fungal host cell may be an *Acremonium*, *Aspergillus*, *Aureobasidium, Bjerkandera*, *Ceriporiopsis, Chrysosporium, Coprinus, Coriolus, Cryptococcus, Filibasidium, Fusarium, Humicola*, *Magnaporthe*, *Mucor, Myceliophthora, Neocallimastix, Neurospora, Paecilomyces*, *Penicillium, Phanerochaete, Phlebia, Piromyces*, *Pleurotus, Schizophyllum, Talaromyces, Thermoascus, Thielavia*, *Tolypocladium, Trametes,* or *Trichoderma* cell.

For example, the filamentous fungal host cell may be an *Aspergillus awamori*, *Aspergillus foetidus*, *Aspergillus fumigatus, Aspergillus japonicus, Aspergillus nidulans*, *Aspergillus niger*, *Aspergillus oryzae*, *Bjerkandera adusta*, *Ceriporiopsis aneirina*, *Ceriporiopsis caregiea*, *Ceriporiopsis gilvescens*, *Ceriporiopsis pannocinta*, *Ceriporiopsis rivulosa*, *Ceriporiopsis subrufa*, *Ceriporiopsis subvermispora*, *Chrysosporium inops*, *Chrysosporium keratinophilum*, *Chrysosporium lucknowense*, *Chrysosporium merdarium*, *Chrysosporium pannicola*, *Chrysosporium queenslandicum*, *Chrysosporium tropicum*, *Chrysosporium zonatum, Coprinus cinereus, Coriolus hirsutus, Fusarium bactridioides, Fusarium cerealis*, *Fusarium crookwellense*, *Fusarium culmorum*, *Fusarium graminearum*, *Fusarium graminum*, *Fusarium heterosporum*, *Fusarium negundi*, *Fusarium oxysporum*, *Fusarium reticulatum*, *Fusarium roseum*, *Fusarium sambucinum*, *Fusarium sarcochroum*, *Fusarium sporotrichioides, Fusarium sulphureum, Fusarium torulosum, Fusarium trichothecioides*, *Fusarium venenatum*, *Humicola insolens*, *Humicola lanuginosa*, *Mucor miehei*, *Myceliophthora thermophila*, *Neurospora crassa*, *Penicillium purpurogenum*, *Phanerochaete chrysosporium*, *Phlebia radiata*, *Pleurotus eryngii*, *Thielavia terrestris, Trametes villosa*, *Trametes versicolor*, *Trichoderma harzianum*, *Trichoderma koningii*, *Trichoderma longibrachiatum*, *Trichoderma reesei*, or *Trichoderma viride* cell.

Fungal cells may be transformed by a process involving protoplast formation, transformation of the protoplasts, and regeneration of the cell wall in a manner known *per* se. Suitable procedures for transformation of *Aspergillus* and *Trichoderma* host cells are described in EP 238023, Yelton et al., 1984, Proc. Natl. Acad. Sci. USA 81: 1470-1474, and Christensen et al., 1988, Bio/Technology 6: 1419-1422. Suitable methods for transforming *Fusarium* species are described by Malardier et al., 1989, Gene 78: 147-156, and WO 96/00787. Yeast may be transformed using the procedures described by Becker and Guarente, In Abelson, J.N. and Simon, M.I., editors, Guide to Yeast Genetics and Molecular Biology, Methods in Enzymology, Volume 194, pp 182-187, Academic Press, Inc., New York; Ito et al., 1983, J. Bacteriol. 153: 163; and Hinnen et al., 1978, Proc. Natl. Acad. Sci. USA 75: 1920.

## Methods of Production

The present invention also relates to methods of producing a chimeric beta-glucosidase or a variant thereof, comprising: (a) cultivating a host cell of the present invention under conditions suitable for expression of the chimeric beta-glucosidase or the variant thereof; and optionally (b) recovering the chimeric beta-glucosidase or the variant thereof.

The host cells are cultivated in a nutrient medium suitable for production of the chimeric beta-glucosidase or the variant thereof using methods known in the art. For example, the cells may be cultivated by shake flask cultivation, or small-scale or large-scale fermentation (including continuous, batch, fed-batch, or solid state fermentations) in laboratory or industrial fermentors performed in a suitable medium and under conditions allowing the chimeric beta-glucosidase or the variant thereof to be expressed and/or isolated. The cultivation takes place in a suitable nutrient medium comprising carbon and nitrogen sources and inorganic salts, using procedures known in the art. Suitable media are available from commercial suppliers or may be prepared according to published compositions (e.g., in catalogues of the American Type Culture Collection). If the chimeric beta-glucosidase or the variant thereof is secreted into the nutrient medium, the chimeric beta-glucosidase or the variant thereof can be recovered directly from the medium. If the chimeric beta-glucosidase or the variant thereof is not secreted, it can be recovered from cell lysates.

The chimeric beta-glucosidase or the variant thereof may be detected using methods known in the art that are specific for the chimeric beta-glucosidases or the variants thereof. These detection methods include, but are not limited to, use of specific antibodies, formation of an enzyme product, or disappearance of an enzyme substrate. For example, an enzyme assay may be used to determine the activity of the chimeric beta-glucosidase or the variant thereof.

The chimeric beta-glucosidase or the variant thereof may be recovered using methods known in the art. For example, the chimeric beta-glucosidase or the variant thereof may be recovered from the nutrient medium by conventional procedures including, but not limited to, collection, centrifugation, filtration, extraction, spray-drying, evaporation, or precipitation. In one aspect, a whole fermentation broth comprising the polypeptide is recovered.

The chimeric beta-glucosidase or the variant thereof may be purified by a variety of procedures known in the art including, but not limited to, chromatography (*e.g.*, ion exchange, affinity, hydrophobic, chromatofocusing, and size exclusion), electrophoretic procedures (*e.g.*, preparative isoelectric focusing), differential solubility (*e.g.*, ammonium sulfate precipitation), SDS-PAGE, or extraction (see, *e.g.*, Protein Purification, Janson and Ryden, editors, VCH Publishers, New York, 1989) to obtain substantially pure chimeric beta-glucosidases or variants thereof.

**Fermentation Broth Formulations or Cell Compositions**

The present invention also relates to a fermentation broth formulation or a cell composition comprising a chimeric beta-glucosidase or a variant thereof of the present invention. The fermentation broth product further comprises additional ingredients used in the fermentation process, such as, for example, cells (including, the host cells containing the gene encoding the chimeric beta-glucosidase or the variant thereof of the present invention which are used to produce the polypeptide of interest), cell debris, biomass, fermentation media and/or fermentation products. In some aspects, the composition is a cell-killed whole broth containing organic acid(s), killed cells and/or cell debris, and culture medium.

The term "fermentation broth" as used herein refers to a preparation produced by cellular fermentation that undergoes no or minimal recovery and/or purification. For example, fermentation broths are produced when microbial cultures are grown to saturation, incubated under carbon-limiting conditions to allow protein synthesis (*e.g.*, expression of enzymes by host cells) and secretion into cell culture medium. The fermentation broth can contain unfractionated or fractionated contents of the fermentation materials derived at the end of the fermentation. Typically, the fermentation broth is unfractionated and comprises the spent culture medium and cell debris present after the microbial cells (*e.g.*, filamentous fungal cells) are removed, *e.g.*, by centrifugation. In some aspects, the fermentation broth contains spent cell culture medium, extracellular enzymes, and viable and/or nonviable microbial cells.

In an aspect, the fermentation broth formulation and cell compositions comprise a first organic acid component comprising at least one 1-5 carbon organic acid and/or a salt thereof and a second organic acid component comprising at least one 6 or more carbon organic acid and/or a salt thereof. In a specific aspect, the first organic acid component is acetic acid, formic acid, propionic acid, a salt thereof, or a mixture of two or more of the foregoing and the second organic acid component is benzoic acid, cyclohexanecarboxylic acid, 4-methylvaleric acid, phenylacetic acid, a salt thereof, or a mixture of two or more of the foregoing.

In one aspect, the composition contains an organic acid(s), and optionally further contains killed cells and/or cell debris. In one aspect, the killed cells and/or cell debris are removed from a cell-killed whole broth to provide a composition that is free of these components.

The fermentation broth formulations or cell compostions may further comprise a preservative and/or antimicrobial (*e.g.*, bacteriostatic) agent, including, but not limited to, sorbitol, sodium chloride, potassium sorbate, and others known in the art.

The fermentation broth formulations or cell compositions may further comprise multiple enzymatic activities, such as one or more (*e.g.*, several) enzymes selected from the group consisting of a cellulase, a hemicellulase, an esterase, an expansin, a laccase, a ligninolytic enzyme, a pectinase, a peroxidase, a protease, and a swollenin. The fermentation broth formulations or cell compositions may also comprise one or more (*e.g.*, several) enzymes selected from the group consisting of a hydrolase, an isomerase, a ligase, a lyase, an oxidoreductase, or a transferase, *e.g.*, an alpha-galactosidase, alpha-glucosidase, aminopeptidase, amylase, beta-galactosidase, beta-glucosidase, beta-xylosidase, carbohydrase, carboxypeptidase, catalase, cellobiohydrolase, cellulase, chitinase, cutinase, cyclodextrin glycosyltransferase, deoxyribonuclease, endoglucanase, esterase, glucoamylase, invertase, laccase, lipase, mannosidase, mutanase, oxidase, pectinolytic enzyme, peroxidase, phytase, polyphenoloxidase, proteolytic enzyme, ribonuclease, transglutaminase, or xylanase.

The cell-killed whole broth or composition may contain the unfractionated contents of the fermentation materials derived at the end of the fermentation. Typically, the cell-killed whole broth or composition contains the spent culture medium and cell debris present after the microbial cells (*e.g.*, filamentous fungal cells) are grown to saturation, incubated under carbon-limiting conditions to allow protein synthesis (*e.g.*, expression of cellulase and/or glucosidase enzyme(s)). In some aspects, the cell-killed whole broth or composition contains the spent cell culture medium, extracellular enzymes, and killed filamentous fungal cells. In some aspects, the microbial cells present in the cell-killed whole broth or composition can be permeabilized and/or lysed using methods known in the art.

A whole broth or cell composition as described herein is typically a liquid, but may contain insoluble components, such as killed cells, cell debris, culture media components, and/or insoluble enzyme(s). In some aspects, insoluble components may be removed to provide a clarified liquid composition.

The whole broth formulations and cell compositions of the present invention may be produced by a method described in WO 90/15861 or WO 2010/096673.

Examples are given below of preferred uses of the compositions of the present invention. The dosage of the composition and other conditions under which the composition is used may be determined on the basis of methods known in the art.

**Enzyme Compositions**

The present invention also relates to compositions comprising a chimeric beta-glucosidase or a variant thereof of the present invention. Preferably, the compositions are enriched in such a polypeptide. The term "enriched" indicates that the beta-glucosidase activity of the composition has been increased, *e.g.*, with an enrichment factor of at least 1.1.

The compositions may comprise a chimeric beta-glucosidase or a variant thereof of the present invention as the major enzymatic component, *e.g.*, a mono-component composition. Alternatively, the compositions may comprise multiple enzymatic activities, such as one or more (*e.g.*, several) enzymes selected from the group consisting of a cellulase, a hemicellulase, an esterase, an expansin, a laccase, a ligninolytic enzyme, a pectinase, a peroxidase, a protease, and a swollenin. The compositions may also comprise one or more (*e.g.*, several) enzymes selected from the group consisting of a hydrolase, an isomerase, a ligase, a lyase, an oxidoreductase, or a transferase, *e.g.*, an alpha-galactosidase, alpha-glucosidase, aminopeptidase, amylase, beta-galactosidase, beta-glucosidase, beta-xylosidase, carbohydrase, carboxypeptidase, catalase, cellobiohydrolase, cellulase, chitinase, cutinase, cyclodextrin glycosyltransferase, deoxyribonuclease, endoglucanase, esterase, glucoamylase, invertase, laccase, lipase, mannosidase, mutanase, oxidase, pectinolytic enzyme, peroxidase, phytase, polyphenoloxidase, proteolytic enzyme, ribonuclease, transglutaminase, or xylanase. The compositions may be prepared in accordance with methods known in the art and may be in the form of a liquid or a dry composition. The compositions may be stabilized in accordance with methods known in the art.

Examples are given below of preferred uses of the compositions of the present invention. The dosage of the composition and other conditions under which the composition is used may be determined on the basis of methods known in the art.

**Uses**

The present invention is also directed to the following methods for using the chimeric beta-glucosidases or variants thereof, or compositions thereof.

The present invention also relates to methods for degrading or converting a cellulosic material, comprising: treating the cellulosic material with an enzyme composition in the presence of a chimeric beta-glucosidase or a variant thereof of the present invention. In one aspect, the methods further comprise recovering the degraded or converted cellulosic material. Soluble products of degradation or conversion of the cellulosic material can be separated from insoluble cellulosic material using a method known in the art such as, for example, centrifugation, filtration, or gravity settling.

The present invention also relates to methods for producing a fermentation product, comprising: (a) saccharifying a cellulosic material with an enzyme composition in the presence of a chimeric beta-glucosidase or a variant thereof of the present invention; (b) fermenting the saccharified cellulosic material with one or more (*e.g.*, several) fermenting microorganisms to produce the fermentation product; and (c) recovering the fermentation product from the fermentation.

The present invention also relates to methods of fermenting a cellulosic material, comprising: fermenting the cellulosic material with one or more (*e.g.*, several) fermenting microorganisms, wherein the cellulosic material is saccharified with an enzyme composition in the presence of a chimeric beta-glucosidase or a variant thereof of the present invention. In one aspect, the fermenting of the cellulosic material produces a fermentation product. In another aspect, the methods further comprise recovering the fermentation product from the fermentation.

The methods of the present invention can be used to saccharify the cellulosic material to fermentable sugars and to convert the fermentable sugars to many useful fermentation products, *e.g.*, fuel, potable ethanol, and/or platform chemicals (*e.g.*, acids, alcohols, ketones, gases, and the like). The production of a desired fermentation product from the cellulosic material typically involves pretreatment, enzymatic hydrolysis (saccharification), and fermentation.

The processing of the cellulosic material according to the present invention can be accomplished using processes conventional in the art. Moreover, the methods of the present invention can be implemented using any conventional biomass processing apparatus configured to operate in accordance with the invention.

Hydrolysis (saccharification) and fermentation, separate or simultaneous, include, but are not limited to, separate hydrolysis and fermentation (SHF); simultaneous saccharification and fermentation (SSF); simultaneous saccharification and co-fermentation (SSCF); hybrid hydrolysis and fermentation (HHF); separate hydrolysis and co-fermentation (SHCF); hybrid hydrolysis and co-fermentation (HHCF); and direct microbial conversion (DMC), also sometimes called consolidated bioprocessing (CBP). SHF uses separate process steps to first enzymatically hydrolyze the cellulosic material to fermentable sugars, *e.g.*, glucose, cellobiose, and pentose monomers, and then ferment the fermentable sugars to ethanol. In SSF, the enzymatic hydrolysis of the cellulosic material and the fermentation of sugars to ethanol are combined in one step (Philippidis, G. P., 1996, Cellulose bioconversion technology, in Handbook on Bioethanol: Production and Utilization, Wyman, C. E., ed., Taylor & Francis, Washington, DC, 179-212). SSCF involves the co-fermentation of multiple sugars (Sheehan, J., and Himmel, M., 1999, Enzymes, energy and the environment: A strategic perspective on the U.S. Department of Energy's research and development activities for bioethanol, Biotechnol. Prog. 15: 817-827). HHF involves a separate hydrolysis step, and in addition a simultaneous saccharification and hydrolysis step, which can be carried out in the same reactor. The steps in an HHF process can be carried out at different temperatures, *i.e.*, high temperature enzymatic saccharification followed by SSF at a lower temperature that the fermentation strain can tolerate. DMC combines all three processes (enzyme production, hydrolysis, and fermentation) in one or more (*e.g.*, several) steps where the same organism is used to produce the enzymes for conversion of the cellulosic material to fermentable sugars and to convert the fermentable sugars into a final product (Lynd et al., 2002, Microbial cellulose utilization: Fundamentals and biotechnology, Microbiol. Mol. Biol. Reviews 66: 506-577). It is understood herein that any method known in the art comprising pretreatment, enzymatic hydrolysis (saccharification), fermentation, or a combination thereof, can be used in the practicing the methods of the present invention.

A conventional apparatus can include a fed-batch stirred reactor, a batch stirred reactor, a continuous flow stirred reactor with ultrafiltration, and/or a continuous plug-flow column reactor (Fernanda de Castilhos Corazza, Flávio Faria de Moraes, Gisella Maria Zanin and Ivo Neitzel, 2003, Optimal control in fed-batch reactor for the cellobiose hydrolysis, Acta Scientiarum. Technology 25: 33-38; Gusakov, A. V., and Sinitsyn, A. P., 1985, Kinetics of the enzymatic hydrolysis of cellulose: 1. A mathematical model for a batch reactor process, Enz. Microb. Technol. 7: 346-352), an attrition reactor (Ryu and Lee, 1983, Bioconversion of waste cellulose by using an attrition bioreactor, Biotechnol. Bioeng. 25: 53-65), or a reactor with intensive stirring induced by an electromagnetic field (Gusakov et al., 1996, Enhancement of enzymatic cellulose hydrolysis using a novel type of bioreactor with intensive stirring induced by electromagnetic field, Appl. Biochem. Biotechnol. 56: 141-153). Additional reactor types include fluidized bed, upflow blanket, immobilized, and extruder type reactors for hydrolysis and/or fermentation.

Pretreatment. In practicing the methods of the present invention, any pretreatment process known in the art can be used to disrupt plant cell wall components of the cellulosic material (Chandra et al., 2007, Substrate pretreatment: The key to effective enzymatic hydrolysis of lignocellulosics? Adv. Biochem. Engin./Biotechnol. 108: 67-93; Galbe and Zacchi, 2007, Pretreatment of lignocellulosic materials for efficient bioethanol production, Adv. Biochem. Engin./Biotechnol. 108: 41-65; Hendriks and Zeeman, 2009, Pretreatments to enhance the digestibility of lignocellulosic biomass, Bioresource Technol. 100: 10-18; Mosier et al., 2005, Features of promising technologies for pretreatment of lignocellulosic biomass, Bioresource Technol. 96: 673-686; Taherzadeh and Karimi, 2008, Pretreatment of lignocellulosic wastes to improve ethanol and biogas production: A review, Int. J. of Mol. Sci. 9: 1621-1651; Yang and Wyman, 2008, Pretreatment: the key to unlocking low-cost cellulosic ethanol, Biofuels Bioproducts and Biorefining-Biofpr. 2: 26-40).

The cellulosic material can also be subjected to particle size reduction, sieving, presoaking, wetting, washing, and/or conditioning prior to pretreatment using methods known in the art.

Conventional pretreatments include, but are not limited to, steam pretreatment (with or without explosion), dilute acid pretreatment, hot water pretreatment, alkaline pretreatment, lime pretreatment, wet oxidation, wet explosion, ammonia fiber explosion, organosolv pretreatment, and biological pretreatment. Additional pretreatments include ammonia percolation, ultrasound, electroporation, microwave, supercritical $CO_2$, supercritical $H_2O$, ozone, ionic liquid, and gamma irradiation pretreatments.

The cellulosic material can be pretreated before hydrolysis and/or fermentation. Pretreatment is preferably performed prior to the hydrolysis. Alternatively, the pretreatment can be carried out simultaneously with enzyme hydrolysis to release fermentable sugars, such as glucose, xylose, and/or cellobiose. In most cases the pretreatment step itself results in some conversion of biomass to fermentable sugars (even in absence of enzymes).

Steam Pretreatment. In steam pretreatment, the cellulosic material is heated to disrupt the plant cell wall components, including lignin, hemicellulose, and cellulose to make the cellulose and other fractions, *e.g.*, hemicellulose, accessible to enzymes. The cellulosic material is passed to or through a reaction vessel where steam is injected to increase the temperature to the required temperature and pressure and is retained therein for the desired reaction time. Steam pretreatment is preferably performed at 140-250°C, *e.g.*, 160-200°C or 170-190°C, where the optimal temperature range depends on addition of a chemical catalyst. Residence time for the steam pretreatment is preferably 1-60 minutes, *e.g.*, 1-30 minutes, 1-20 minutes, 3-12 minutes, or 4-10 minutes, where the optimal residence time depends on temperature range and addition of a chemical catalyst. Steam pretreatment allows for relatively high solids loadings, so that the cellulosic material is generally only moist during the pretreatment. The steam pretreatment is often combined with an explosive discharge of the material after the pretreatment, which is known as steam explosion, that is, rapid flashing to atmospheric pressure and turbulent flow of the material to increase the accessible surface area by fragmentation (Duff and Murray, 1996, Bioresource Technology 855: 1-33; Galbe and Zacchi, 2002, Appl. Microbiol. Biotechnol. 59:

618-628; U.S. Patent Application No. 20020164730). During steam pretreatment, hemicellulose acetyl groups are cleaved and the resulting acid autocatalyzes partial hydrolysis of the hemicellulose to monosaccharides and oligosaccharides. Lignin is removed to only a limited extent.

Chemical Pretreatment: The term "chemical treatment" refers to any chemical pretreatment that promotes the separation and/or release of cellulose, hemicellulose, and/or lignin. Such a pretreatment can convert crystalline cellulose to amorphous cellulose. Examples of suitable chemical pretreatment processes include, for example, dilute acid pretreatment, lime pretreatment, wet oxidation, ammonia fiber/freeze explosion (AFEX), ammonia percolation (APR), ionic liquid, and organosolv pretreatments.

A catalyst such as $H_2SO_4$ or $SO_2$ (typically 0.3 to 5% w/w) is often added prior to steam pretreatment, which decreases the time and temperature, increases the recovery, and improves enzymatic hydrolysis (Ballesteros et al., 2006, Appl. Biochem. Biotechnol. 129-132: 496-508; Varga et al., 2004, Appl. Biochem. Biotechnol. 113-116: 509-523; Sassner et al., 2006, Enzyme Microb. Technol. 39: 756-762). In dilute acid pretreatment, the cellulosic material is mixed with dilute acid, typically $H_2SO_4$, and water to form a slurry, heated by steam to the desired temperature, and after a residence time flashed to atmospheric pressure. The dilute acid pretreatment can be performed with a number of reactor designs, *e.g.*, plug-flow reactors, counter-current reactors, or continuous counter-current shrinking bed reactors (Duff and Murray, 1996, *supra*; Schell et al., 2004, Bioresource Technol. 91: 179-188; Lee et al., 1999, Adv. Biochem. Eng. Biotechnol. 65: 93-115).

Several methods of pretreatment under alkaline conditions can also be used. These alkaline pretreatments include, but are not limited to, sodium hydroxide, lime, wet oxidation, ammonia percolation (APR), and ammonia fiber/freeze explosion (AFEX).

Lime pretreatment is performed with calcium oxide or calcium hydroxide at temperatures of 85-150°C and residence times from 1 hour to several days (Wyman et al., 2005, Bioresource Technol. 96: 1959-1966; Mosier et al., 2005, Bioresource Technol. 96: 673-686). WO 2006/110891, WO 2006/110899, WO 2006/110900, and WO 2006/110901 disclose pretreatment methods using ammonia.

Wet oxidation is a thermal pretreatment performed typically at 180-200°C for 5-15 minutes with addition of an oxidative agent such as hydrogen peroxide or over-pressure of oxygen (Schmidt and Thomsen, 1998, Bioresource Technol. 64: 139-151; Palonen et al., 2004, Appl. Biochem. Biotechnol. 117: 1-17; Varga et al., 2004, Biotechnol. Bioeng. 88: 567-574; Martin et al., 2006, J. Chem. Technol. Biotechnol. 81: 1669-1677). The pretreatment is performed preferably at 1-40% dry matter, *e.g.*, 2-30% dry matter or 5-20% dry matter, and often the initial pH is increased by the addition of alkali such as sodium carbonate.

A modification of the wet oxidation pretreatment method, known as wet explosion (combination of wet oxidation and steam explosion) can handle dry matter up to 30%. In wet explosion, the oxidizing agent is introduced during pretreatment after a certain residence time. The pretreatment is then ended by flashing to atmospheric pressure (WO 2006/032282).

Ammonia fiber explosion (AFEX) involves treating the cellulosic material with liquid or gaseous ammonia at moderate temperatures such as 90-150°C and high pressure such as 17-20 bar for 5-10 minutes, where the dry matter content can be as high as 60% (Gollapalli et al., 2002, Appl. Biochem. Biotechnol. 98: 23-35; Chundawat et al., 2007, Biotechnol. Bioeng. 96: 219-231; Alizadeh et al., 2005, Appl. Biochem. Biotechnol. 121: 1133-1141; Teymouri et al., 2005, Bioresource Technol. 96: 2014-2018). During AFEX pretreatment cellulose and hemicelluloses remain relatively intact. Lignin-carbohydrate complexes are cleaved.

Organosolv pretreatment delignifies the cellulosic material by extraction using aqueous ethanol (40-60% ethanol) at 160-200°C for 30-60 minutes (Pan et al., 2005, Biotechnol. Bioeng. 90: 473-481; Pan et al., 2006, Biotechnol. Bioeng. 94: 851-861; Kurabi et al., 2005, Appl. Biochem. Biotechnol. 121: 219-230). Sulphuric acid is usually added as a catalyst. In organosolv pretreatment, the majority of hemicellulose and lignin is removed.

Other examples of suitable pretreatment methods are described by Schell et al., 2003, Appl. Biochem. and Biotechnol. Vol. 105-108, p. 69-85, and Mosier et al., 2005, Bioresource Technology 96: 673-686, and U.S. Published Application 2002/0164730.

In one aspect, the chemical pretreatment is preferably carried out as a dilute acid treatment, and more preferably as a continuous dilute acid treatment. The acid is typically sulfuric acid, but other acids can also be used, such as acetic acid, citric acid, nitric acid, phosphoric acid, tartaric acid, succinic acid, hydrogen chloride, or mixtures thereof. Mild acid treatment is conducted in the pH range of preferably 1-5, *e.g.*, 1-4 or 1-2.5. In one aspect, the acid concentration is in the range from preferably 0.01 to 10 wt % acid, *e.g.*, 0.05 to 5 wt % acid or 0.1 to 2 wt % acid. The acid is contacted with the cellulosic material and held at a temperature in the range of preferably 140-200°C, *e.g.*, 165-190°C, for periods ranging from 1 to 60 minutes.

In another aspect, pretreatment takes place in an aqueous slurry. In preferred aspects, the cellulosic material is present during pretreatment in amounts preferably between 10-80 wt %, *e.g.*, 20-70 wt % or 30-60 wt %, such as around 40 wt %. The pretreated cellulosic material can be unwashed or washed using any method known in the art, *e.g.*, washed with water.

Mechanical Pretreatment or Physical Pretreatment: The term "mechanical pretreatment" or "physical pretreatment" refers to any pretreatment that promotes size reduction of particles. For example, such pretreatment can involve various types of grinding or milling (*e.g.*, dry milling, wet milling, or vibratory ball milling).

The cellulosic material can be pretreated both physically (mechanically) and chemically. Mechanical or physical pretreatment can be coupled with steaming/steam explosion, hydrothermolysis, dilute or mild acid treatment, high temperature, high pressure treatment, irradiation (*e.g.*, microwave irradiation), or combinations thereof. In one aspect, high pressure means pressure in the range of preferably about 100 to about 400 psi, *e.g.*, about 150 to about 250 psi. In another aspect, high temperature means temperatures in the range of about 100 to about 300°C, *e.g.*, about 140 to about 200°C. In a preferred aspect, mechanical or physical pretreatment is performed in a batch-process using a steam gun hydrolyzer system that uses high pressure and high temperature as defined above, *e.g.*, a Sunds Hydrolyzer available from Sunds Defibrator AB, Sweden. The physical and chemical pretreatments can be carried out sequentially or simultaneously, as desired.

Accordingly, in a preferred aspect, the cellulosic material is subjected to physical (mechanical) or chemical pretreatment, or any combination thereof, to promote the separation and/or release of cellulose, hemicellulose, and/or lignin.

Biological Pretreatment: The term "biological pretreatment" refers to any biological pretreatment that promotes the separation and/or release of cellulose, hemicellulose, and/or lignin from the cellulosic material. Biological pretreatment techniques can involve applying lignin-solubilizing microorganisms and/or enzymes (see, for example, Hsu, T.-A., 1996, Pretreatment of biomass, in Handbook on Bioethanol: Production and Utilization, Wyman, C. E., ed., Taylor & Francis, Washington, DC, 179-212; Ghosh and Singh, 1993, Physicochemical and biological treatments for enzymatic/microbial conversion of cellulosic biomass, Adv. Appl. Microbiol. 39: 295-333; McMillan, J. D., 1994, Pretreating lignocellulosic biomass: a review, in Enzymatic Conversion of Biomass for Fuels Production, Himmel, M. E., Baker, J. O., and Overend, R. P., eds., ACS Symposium Series 566, American Chemical Society, Washington, DC, chapter 15; Gong, C. S., Cao, N. J., Du, J., and Tsao, G. T., 1999, Ethanol production from renewable resources, in Advances in Biochemical Engineering/Biotechnology, Scheper, T., ed., Springer-Verlag Berlin Heidelberg, Germany, 65: 207-241; Olsson and Hahn-Hagerdal, 1996, Fermentation of lignocellulosic hydrolysates for ethanol production, Enz. Microb. Tech. 18: 312-331; and Vallander and Eriksson, 1990, Production of ethanol from lignocellulosic materials: State of the art, Adv. Biochem. Eng./Biotechnol. 42: 63-95).

Saccharification. In the hydrolysis step, also known as saccharification, the cellulosic material, e.g., pretreated, is hydrolyzed to break down cellulose and/or hemicellulose to fermentable sugars, such as glucose, cellobiose, xylose, xylulose, arabinose, mannose, galactose, and/or soluble oligosaccharides. The hydrolysis is performed enzymatically by an enzyme composition in the presence of a chimeric beta-glucosidase or a variant thereof of the present invention. The components of the compositions can be added simultaneously or sequentially.

Enzymatic hydrolysis is preferably carried out in a suitable aqueous environment under conditions that can be readily determined by one skilled in the art. In one aspect, hydrolysis is performed under conditions suitable for the activity of the enzyme components, *i.e.*, optimal for the enzyme components. The hydrolysis can be carried out as a fed batch or continuous process where the cellulosic material is fed gradually to, for example, an enzyme containing hydrolysis solution.

The saccharification is generally performed in stirred-tank reactors or fermentors under controlled pH, temperature, and mixing conditions. Suitable process time, temperature and pH conditions can readily be determined by one skilled in the art. For example, the saccharification can last up to 200 hours, but is typically performed for preferably about 12 to about 120 hours, e.g., about 16 to about 72 hours or about 24 to about 48 hours. The temperature is in the range of preferably about 25°C to about 70°C, *e.g.*, about 30°C to about 65°C, about 40°C to about 60°C, or about 50°C to about 55°C. The pH is in the range of preferably about 3 to about 8, *e.g.*, about 3.5 to about 7, about 4 to about 6, or about 5.0 to about 5.5. The dry solids content is in the range of preferably about 5 to about 50 wt %, *e.g.*, about 10 to about 40 wt % or about 20 to about 30 wt %.

The enzyme compositions can comprise any protein useful in degrading the cellulosic material.

In one aspect, the enzyme composition comprises or further comprises one or more (*e.g.*, several) proteins selected from the group consisting of a cellulase, a polypeptide having cellulolytic enhancing activity, a hemicellulase, an esterase, an expansin, a laccase, a ligninolytic enzyme, a pectinase, a peroxidase, a protease, and a swollenin. In another aspect, the cellulase is preferably one or more (*e.g.*, several) enzymes selected from the group consisting of an endoglucanase, a cellobiohydrolase, and a beta-glucosidase. In another aspect, the hemicellulase is preferably one or more (*e.g.*, several) enzymes selected from the group consisting of an acetylmannan esterase, an acetylxylan esterase, an arabinanase, an arabinofuranosidase, a coumaric acid esterase, a feruloyl esterase, a galactosidase, a glucuronidase, a glucuronoyl esterase, a mannanase, a mannosidase, a xylanase, and a xylosidase.

In another aspect, the enzyme composition comprises one or more (*e.g.*, several) cellulolytic enzymes. In another aspect, the enzyme composition comprises or further comprises one or more (*e.g.*, several) hemicellulolytic enzymes. In another aspect, the enzyme composition comprises one or more (*e.g.*, several) cellulolytic enzymes and one or more (*e.g.*, several) hemicellulolytic enzymes. In another aspect, the enzyme composition comprises one or more (*e.g.*, several) enzymes selected from the group of cellulolytic enzymes and hemicellulolytic enzymes. In another aspect, the enzyme composition comprises an endoglucanase. In another aspect, the enzyme composition comprises a cellobiohydrolase. In another aspect, the enzyme composition comprises a beta-glucosidase. In another aspect, the enzyme composition comprises a polypeptide having cellulolytic enhancing activity. In another aspect, the enzyme composition comprises an endoglucanase and a polypeptide having cellulolytic enhancing activity. In another aspect, the enzyme composition comprises a cellobiohydrolase and a polypeptide having cellulolytic enhancing activity,. In another aspect, the enzyme composition comprises a beta-glucosidase and a polypeptide having cellulolytic enhancing activity. In another aspect, the enzyme composition comprises an endoglucanase and a cellobiohydrolase. In another aspect, the enzyme composition comprises an endoglucanase and a beta-glucosidase. In another aspect, the enzyme composition comprises a cellobiohydrolase and a beta-glucosidase. In another aspect, the enzyme composition comprises an endoglucanase, a cellobiohydrolase, and a polypeptide having cellulolytic enhancing activity. In another aspect, the enzyme composition comprises an endoglucanase, a beta-glucosidase, and a polypeptide having cellulolytic enhancing activity. In another aspect, the enzyme composition comprises a cellobiohydrolase, a beta-glucosidase, and a polypeptide having cellulolytic enhancing activity. In another aspect, the enzyme composition comprises an endoglucanase, a cellobiohydrolase, and a beta-glucosidase. In another aspect, the enzyme composition comprises an endoglucanase, a cellobiohydrolase, a beta-glucosidase, and a polypeptide having cellulolytic enhancing activity.

In another aspect, the enzyme composition comprises an acetylmannan esterase. In another aspect, the enzyme composition comprises an acetylxylan esterase. In another aspect, the enzyme composition comprises an arabinanase (*e.g.*, alpha-L-arabinanase). In another aspect, the enzyme composition comprises an arabinofuranosidase (*e.g.*, alpha-L-arabinofuranosidase). In another aspect, the enzyme composition comprises a coumaric acid esterase. In another aspect, the enzyme composition comprises a feruloyl esterase. In another aspect, the enzyme composition comprises a galactosidase (*e.g.*, alpha-galactosidase and/or beta-galactosidase). In another aspect, the enzyme composition comprises a glucuronidase (*e.g.*, alpha-D-glucuronidase). In another aspect, the enzyme composition comprises a glucuronoyl esterase. In another aspect, the enzyme composition comprises a mannanase. In another aspect, the enzyme composition comprises a mannosidase (*e.g.*, beta-mannosidase). In another aspect, the enzyme composition comprises a xylanase. In a preferred aspect, the xylanase is a Family 10 xylanase. In another aspect, the enzyme composition comprises a xylosidase (*e.g.*, beta-xylosidase).

In another aspect, the enzyme composition comprises an esterase. In another aspect, the enzyme composition comprises an expansin. In another aspect, the enzyme composition comprises a laccase. In another aspect, the enzyme composition comprises a ligninolytic enzyme. In a preferred aspect, the ligninolytic enzyme is a manganese peroxidase. In another preferred aspect, the ligninolytic enzyme is a lignin peroxidase. In another preferred aspect, the ligninolytic enzyme is a $H_2O_2$-producing enzyme. In another aspect, the enzyme composition comprises a pectinase. In another aspect, the enzyme composition comprises a peroxidase. In another aspect, the enzyme composition comprises a protease. In another aspect, the enzyme composition comprises a swollenin.

In the methods of the present invention, the enzyme(s) can be added prior to or during saccharification, saccharification and fermentation, or fermentation.

One or more (*e.g.*, several) components of the enzyme composition may be wild-type proteins, recombinant proteins, or a combination of wild-type proteins and recombinant proteins. For example, one or more (*e.g.*, several) components may be native proteins of a cell, which is used as a host cell to express recombinantly one or more (*e.g.*, several) other components of the enzyme composition. One or more (*e.g.*, several) components of the enzyme composition may be produced as monocomponents, which are then combined to form the enzyme composition. The enzyme composition may be a combination of multicomponent and monocomponent protein preparations.

The enzymes used in the methods of the present invention may be in any form suitable for use, such as, for example, a fermentation broth formulation or a cell composition, a cell lysate with or without cellular debris, a semipurified or purified enzyme preparation, or a host cell as a source of the enzymes. The enzyme composition may be a dry powder or granulate, a non-dusting granulate, a liquid, a stabilized liquid, or a stabilized protected enzyme. Liquid enzyme preparations may, for instance, be stabilized by adding stabilizers such as a sugar, a sugar alcohol or another polyol, and/or lactic acid or another organic acid according to established processes.

The optimum amounts of the enzymes and a chimeric beta-glucosidase or a variant thereof of the present invention depend on several factors including, but not limited to, the mixture of cellulolytic and/or hemicellulolytic enzyme components, the cellulosic substrate, the concentration of cellulosic substrate, the pretreatment(s) of the cellulosic substrate, temperature, time, pH, and inclusion of fermenting organism (*e.g.*, yeast for Simultaneous Saccharification and Fermentation).

In one aspect, an effective amount of cellulolytic or hemicellulolytic enzyme to the cellulosic material is about 0.5 to about 50 mg, *e.g.*, about 0.5 to about 40 mg, about 0.5 to about 25 mg, about 0.75 to about 20 mg, about 0.75 to about 15 mg, about 0.5 to about 10 mg, or about 2.5 to about 10 mg per g of the cellulosic material.

In another aspect, an effective amount of a chimeric beta-glucosidase or a variant thereof of the present invention to the cellulosic material is about 0.01 to about 50.0 mg, *e.g.*, about 0.01 to about 40 mg, about 0.01 to about 30 mg, about 0.01 to about 20 mg, about 0.01 to about 10 mg, about 0.01 to about 5 mg, about 0.025 to about 1.5 mg, about 0.05 to about 1.25 mg, about 0.075 to about 1.25 mg, about 0.1 to about 1.25 mg, about 0.15 to about 1.25 mg, or about 0.25 to about 1.0 mg per g of the cellulosic material.

In another aspect, an effective amount of a chimeric beta-glucosidase or a variant thereof of the present invention to cellulolytic or hemicellulolytic enzyme is about 0.005 to about 1.0 g, *e.g.*, about 0.01 to about 1.0 g, about 0.15 to about 0.75 g, about 0.15 to about 0.5 g, about 0.1 to about 0.5 g, about 0.1 to about 0.25 g, or about 0.05 to about 0.2 g per g of cellulolytic or hemicellulolytic enzyme.

The polypeptides having cellulolytic enzyme activity or hemicellulolytic enzyme activity as well as other proteins/polypeptides useful in the degradation of the cellulosic material, *e.g.*, polypeptides having cellulolytic enhancing activity (collectively hereinafter "polypeptides having enzyme activity") can be derived or obtained from any suitable origin, including, bacterial, fungal, yeast, plant, or mammalian origin. The term "obtained" also means herein that the enzyme may have been produced recombinantly in a host organism employing methods described herein, wherein the recombinantly produced enzyme is either native or foreign to the host organism or has a modified amino acid sequence, *e.g.*, having one or more (*e.g.*, several) amino acids that are deleted, inserted and/or substituted, *i.e.*, a recombinantly produced enzyme that is a mutant and/or a fragment of a native amino acid sequence or an enzyme produced by nucleic acid shuffling processes known in the art. Encompassed within the meaning of a native enzyme are natural variants and within the meaning of a foreign enzyme are variants obtained recombinantly, such as by site-directed mutagenesis or shuffling.

A polypeptide having enzyme activity may be a bacterial polypeptide. For example, the polypeptide may be a Gram positive bacterial polypeptide such as a *Bacillus, Streptococcus, Streptomyces, Staphylococcus, Enterococcus, Lactobacillus, Lactococcus, Clostridium*, *Geobacillus*, *Caldicellulosiruptor*, *Acidothermus*, *Thermobifidia*, or *Oceanobacillus* polypeptide having enzyme activity, or a Gram negative bacterial polypeptide such as an *E. coli*, *Pseudomonas*, *Salmonella*, *Campylobacter*, *Helicobacter*, *Flavobacterium*, *Fusobacterium*, *Ilyobacter*, *Neisseria*, or *Ureaplasma* polypeptide having enzyme activity.

In one aspect, the polypeptide is a *Bacillus alkalophilus*, *Bacillus amyloliquefaciens*, *Bacillus brevis, Bacillus circulans, Bacillus clausii, Bacillus coagulans, Bacillus firmus, Bacillus lautus, Bacillus lentus, Bacillus licheniformis*, *Bacillus megaterium, Bacillus pumilus, Bacillus stearothermophilus, Bacillus subtilis*, or *Bacillus thuringiensis* polypeptide having enzyme activity.

In another aspect, the polypeptide is a *Streptococcus equisimilis*, *Streptococcus pyogenes, Streptococcus uberis,* or *Streptococcus equi* subsp. *Zooepidemicus* polypeptide having enzyme activity.

In another aspect, the polypeptide is a *Streptomyces achromogenes*, *Streptomyces avermitilis*, *Streptomyces coelicolor*, *Streptomyces griseus*, or *Streptomyces lividans* polypeptide having enzyme activity.

The polypeptide having enzyme activity may also be a fungal polypeptide, and more preferably a yeast polypeptide such as a *Candida*, *Kluyveromyces*, *Pichia*, *Saccharomyces*, *Schizosaccharomyces*, or *Yarrowia* polypeptide having enzyme activity; or more preferably a filamentous fungal polypeptide such as an *Acremonium*, *Agaricus*, *Alternaria*, *Aspergillus, Aureobasidium, Botryospaeria, Ceriporiopsis, Chaetomidium, Chrysosporium, Claviceps*, *Cochliobolus, Coprinopsis*, *Coptotermes*, *Corynascus, Cryphonectria, Cryptococcus, Diplodia*, *Exidia*, *Filibasidium*, *Fusarium*, *Gibberella*, *Holomastigotoides*, *Humicola*, *Irpex*, *Lentinula*, *Leptospaeria*, *Magnaporthe*, *Melanocarpus*, *Meripilus*, *Mucor*, *Myceliophthora*, *Neocallimastix*, *Neurospora*, *Paecilomyces*, *Penicillium*, *Phanerochaete*, *Piromyces*, *Poitrasia*, *Pseudoplectania*, *Pseudotrichonympha*, *Rhizomucor*, *Schizophyllum*, *Scytalidium*, *Talaromyces, Thermoascus, Thielavia, Tolypocladium, Trichoderma, Trichophaea, Verticillium*, *Volvariella*, or *Xylaria* polypeptide having enzyme activity.

In one aspect, the polypeptide is a *Saccharomyces carlsbergensis*, *Saccharomyces cerevisiae*, *Saccharomyces diastaticus*, *Saccharomyces douglasii*, *Saccharomyces kluyveri*, *Saccharomyces norbensis*, or *Saccharomyces oviformis* polypeptide having enzyme activity.

In another aspect, the polypeptide is an *Acremonium cellulolyticus*, *Aspergillus aculeatus*, *Aspergillus awamori*, *Aspergillus fumigatus*, *Aspergillus foetidus*, *Aspergillus japonicus*, *Aspergillus nidulans*, *Aspergillus niger*, *Aspergillus oryzae*, *Chrysosporium keratinophilum*, *Chrysosporium lucknowense*, *Chrysosporium tropicum*, *Chrysosporium merdarium*, *Chrysosporium inops*, *Chrysosporium pannicola*, *Chrysosporium queenslandicum*, *Chrysosporium zonatum*, *Fusarium bactridioides*, *Fusarium cerealis*, *Fusarium crookwellense*, *Fusarium culmorum*, *Fusarium graminearum*, *Fusarium graminum*, *Fusarium heterosporum*, *Fusarium negundi*, *Fusarium oxysporum*, *Fusarium reticulatum*, *Fusarium roseum, Fusarium sambucinum, Fusarium sarcochroum, Fusarium sporotrichioides*, *Fusarium sulphureum*, *Fusarium torulosum*, *Fusarium trichothecioides, Fusarium venenatum*, *Humicola grisea*, *Humicola insolens*, *Humicola lanuginosa*, *Irpex lacteus*, *Mucor miehei*, *Myceliophthora thermophila*, *Neurospora crassa*, *Penicillium funiculosum, Penicillium purpurogenum, Phanerochaete chrysosporium, Thielavia achromatica*, *Thielavia albomyces*, *Thielavia albopilosa*, *Thielavia australeinsis*, *Thielavia fimeti*, *Thielavia microspora*, *Thielavia ovispora*, *Thielavia peruviana*, *Thielavia spededonium, Thielavia setosa, Thielavia subthermophila, Thielavia terrestris, Trichoderma harzianum*, *Trichoderma koningii*, *Trichoderma longibrachiatum*, *Trichoderma reesei*, *Trichoderma viride*, or *Trichophaea saccata* polypeptide having enzyme activity.

Chemically modified or protein engineered mutants of polypeptides having enzyme activity may also be used.

One or more (*e.g.*, several) components of the enzyme composition may be a recombinant component, *i.e.*, produced by cloning of a DNA sequence encoding the single component and subsequent cell transformed with the DNA sequence and expressed in a host (see, for example, WO 91/17243 and WO 91/17244). The host is preferably a heterologous host (enzyme is foreign to host), but the host may under certain conditions also be a homologous host (enzyme is native to host). Monocomponent cellulolytic proteins may also be prepared by purifying such a protein from a fermentation broth.

In one aspect, the one or more (*e.g.*, several) cellulolytic enzymes comprise a commercial cellulolytic enzyme preparation. Examples of commercial cellulolytic enzyme preparations suitable for use in the present invention include, for example, CELLIC® CTec (Novozymes A/S), CELLIC® CTec2 (Novozymes A/S), CELLIC® CTec3 (Novozymes A/S), CELLUCLAST™ (Novozymes A/S), NOVOZYM™ 188 (Novozymes A/S), CELLUZYME™ (Novozymes A/S), CEREFLO™ (Novozymes A/S), and ULTRAFLO™ (Novozymes A/S), ACCELERASE™ (Genencor Int.), LAMINEX™ (Genencor Int.), SPEZYME™ CP (Genencor Int.), FILTRASE® NL (DSM); METHAPLUS® S/L 100 (DSM), ROHAMENT™ 7069 W (Röhm GmbH), FIBREZYME® LDI (Dyadic International, Inc.), FIBREZYME® LBR (Dyadic International, Inc.), or VISCOSTAR® 150L (Dyadic International, Inc.). The cellulase enzymes are added in amounts effective from about 0.001 to about 5.0 wt % of solids, e.g., about 0.025 to about 4.0 wt % of solids or about 0.005 to about 2.0 wt % of solids.

Examples of bacterial endoglucanases that can be used in the processes of the present invention, include, but are not limited to, an *Acidothermus cellulolyticus* endoglucanase (WO 91/05039; WO 93/15186; U.S. Patent No. 5,275,944; WO 96/02551; U.S. Patent No. 5,536,655, WO 00/70031, WO 05/093050); *Thermobifida fusca* endoglucanase III (WO 05/093050); and *Thermobifida fusca* endoglucanase V (WO 05/093050).

Examples of fungal endoglucanases that can be used in the present invention, include, but are not limited to, a *Trichoderma reesei* endoglucanase I (Penttila et al., 1986, Gene 45: 253-263, *Trichoderma reesei* Cel7B endoglucanase I (GENBANK™ accession no. M15665), *Trichoderma reesei* endoglucanase II (Saloheimo, et al., 1988, Gene 63:11-22), *Trichoderma reesei* Cel5A endoglucanase II (GENBANK™ accession no. M19373), *Trichoderma reesei* endoglucanase III (Okada et al., 1988, Appl. Environ. Microbiol. 64: 555-563, GENBANK™ accession no. AB003694), *Trichoderma reesei* endoglucanase V (Saloheimo et al., 1994, Molecular Microbiology 13: 219-228, GENBANK™ accession no. Z33381), *Aspergillus aculeatus* endoglucanase (Ooi et al., 1990, Nucleic Acids Research 18: 5884), *Aspergillus kawachii* endoglucanase (Sakamoto et al., 1995, Current Genetics 27: 435-439), *Erwinia carotovara* endoglucanase (Saarilahti et al., 1990, Gene 90: 9-14), *Fusarium oxysporum* endoglucanase (GENBANK™ accession no. L29381), *Humicola grisea* var. *thermoidea* endoglucanase (GENBANK™ accession no. AB003107), *Melanocarpus albomyces* endoglucanase (GENBANK™ accession no. MAL515703), *Neurospora crassa* endoglucanase (GENBANK™ accession no. XM_324477), *Humicola insolens* endoglucanase V, *Myceliophthora thermophila* CBS 117.65 endoglucanase, basidiomycete CBS 495.95 endoglucanase, basidiomycete CBS 494.95 endoglucanase, *Thielavia terrestris* NRRL 8126 CEL6B endoglucanase, *Thielavia terrestris* NRRL 8126 CEL6C endoglucanase, *Thielavia terrestris* NRRL 8126 CEL7C endoglucanase, *Thielavia terrestris* NRRL 8126 CEL7E endoglucanase, *Thielavia terrestris* NRRL 8126 CEL7F endoglucanase, *Cladorrhinum foecundissimum* ATCC 62373 CEL7A endoglucanase, and *Trichoderma reesei* strain No. VTT-D-80133 endoglucanase (GENBANK™ accession no. M15665).

Examples of cellobiohydrolases useful in the present invention include, but are not limited to, *Aspergillus aculeatus* cellobiohydrolase II (WO 2011/059740), *Chaetomium thermophilum* cellobiohydrolase I, *Chaetomium thermophilum* cellobiohydrolase II, *Humicola insolens* cellobiohydrolase I, *Myceliophthora thermophila* cellobiohydrolase II (WO 2009/042871), *Thielavia hyrcanie* cellobiohydrolase II (WO 2010/141325), *Thielavia terrestris* cellobiohydrolase II (CEL6A, WO 2006/074435), *Trichoderma reesei* cellobiohydrolase I, *Trichoderma reesei* cellobiohydrolase II, and *Trichophaea saccata* cellobiohydrolase II (WO 2010/057086).

Examples of beta-glucosidases useful in the present invention include, but are not limited to, beta-glucosidases from *Aspergillus aculeatus* (Kawaguchi et al., 1996, Gene 173: 287-288), *Aspergillus fumigatus* (WO 2005/047499), *Aspergillus niger* (Dan et al., 2000, J. Biol. Chem. 275: 4973-4980), *Aspergillus oryzae* (WO 2002/095014), *Penicillium brasilianum* IBT 20888 (WO 2007/019442 and WO 2010/088387), *Thielavia terrestris* (WO 2011/035029), and *Trichophaea saccata* (WO 2007/019442).

The beta-glucosidase may be a fusion protein. In one aspect, the beta-glucosidase is an *Aspergillus oryzae* beta-glucosidase variant BG fusion protein (WO 2008/057637) or an *Aspergillus oryzae* beta-glucosidase fusion protein (WO 2008/057637).

Other useful endoglucanases, cellobiohydrolases, and beta-glucosidases are disclosed in numerous Glycosyl Hydrolase families using the classification according to Henrissat B., 1991, A classification of glycosyl hydrolases based on amino-acid sequence similarities, Biochem. J. 280: 309-316, and Henrissat B., and Bairoch A., 1996, Updating the sequence-based classification of glycosyl hydrolases, Biochem. J. 316: 695-696.

Other cellulolytic enzymes that may be used in the present invention are described in WO 98/13465, WO 98/015619, WO 98/015633, WO 99/06574, WO 99/10481, WO 99/025847, WO 99/031255, WO 2002/101078, WO 2003/027306, WO 2003/052054, WO 2003/052055, WO 2003/052056, WO 2003/052057, WO 2003/052118, WO 2004/016760, WO 2004/043980, WO 2004/048592, WO 2005/001065, WO 2005/028636, WO 2005/093050, WO 2005/093073, WO 2006/074005, WO 2006/117432, WO 2007/071818, WO 2007/071820, WO 2008/008070, WO 2008/008793, U.S. Patent No. 5,457,046, U.S. Patent No. 5,648,263, and U.S. Patent No. 5,686,593.

In the methods of the present invention, any GH61 polypeptide having cellulolytic enhancing activity can be used as a component of the enzyme composition.

Examples of GH61 polypeptides having cellulolytic enhancing activity useful in the processes of the present invention include, but are not limited to, GH61 polypeptides from *Thielavia terrestris* (WO 2005/074647, WO 2008/148131, and WO 2011/035027), *Thermoascus aurantiacus* (WO 2005/074656 and WO 2010/065830), *Trichoderma reesei* (WO 2007/089290), *Myceliophthora thermophila* (WO 2009/085935, WO 2009/085859, WO 2009/085864, and WO 2009/085868), *Aspergillus fumigatus* (WO 2010/138754), *Penicillium pinophilum* (WO 2011/005867), *Thermoascus* sp. (WO 2011/039319), *Penicillium* sp. (WO 2011/041397), and *Thermoascus crustaceous* (WO 2011/041504).

In one aspect, the GH61 polypeptide having cellulolytic enhancing activity is used in the presence of a soluble activating divalent metal cation according to WO 2008/151043, e.g., manganese or copper.

In another aspect, the GH61 polypeptide having cellulolytic enhancing activity is used in the presence of a dioxy compound, a bicylic compound, a heterocyclic compound, a nitrogen-containing compound, a quinone compound, a sulfur-containing compound, or a liquor obtained from a pretreated cellulosic material such as pretreated corn stover (PCS).

The dioxy compound may include any suitable compound containing two or more oxygen atoms. In some aspects, the dioxy compounds contain a substituted aryl moiety as described herein. The dioxy compounds may comprise one or more (*e.g.*, several) hydroxyl and/or hydroxyl derivatives, but also include substituted aryl moieties lacking hydroxyl and hydroxyl derivatives. Non-limiting examples of the dioxy compounds include pyrocatechol or catechol; caffeic acid; 3,4-dihydroxybenzoic acid; 4-tert-butyl-5-methoxy-1,2-benzenediol; pyrogallol; gallic acid; methyl-3,4,5-trihydroxybenzoate; 2,3,4-trihydroxybenzophenone; 2,6-dimethoxyphenol; sinapinic acid; 3,5-dihydroxybenzoic acid; 4-chloro-1,2-benzenediol; 4-nitro-1,2-benzenediol; tannic acid; ethyl gallate; methyl glycolate; dihydroxyfumaric acid; 2-butyne-1,4-diol; (croconic acid; 1,3-propanediol; tartaric acid; 2,4-pentanediol; 3-ethyoxy-1,2-propanediol; 2,4,4'-trihydroxybenzophenone; cis-2-butene-1,4-diol; 3,4-dihydroxy-3-cyclobutene-1,2-dione; dihydroxyacetone; acrolein acetal; methyl-4-hydroxybenzoate; 4-hydroxybenzoic acid; and methyl-3,5-dimethoxy-4-hydroxybenzoate; or a salt or solvate thereof.

The bicyclic compound may include any suitable substituted fused ring system as described herein. The compounds may comprise one or more (*e.g.*, several) additional rings, and are not limited to a specific number of rings unless otherwise stated. In one aspect, the bicyclic compound is a flavonoid. In another aspect, the bicyclic compound is an optionally substituted isoflavonoid. In another aspect, the bicyclic compound is an optionally substituted flavylium ion, such as an optionally substituted anthocyanidin or optionally substituted anthocyanin, or derivative thereof. Non-limiting examples of the bicyclic compounds include epicatechin; quercetin; myricetin; taxifolin; kaempferol; morin; acacetin; naringenin; isorhamnetin; apigenin; cyanidin; cyanin; kuromanin; keracyanin; or a salt or solvate thereof.

The heterocyclic compound may be any suitable compound, such as an optionally substituted aromatic or non-aromatic ring comprising a heteroatom, as described herein. In one aspect, the heterocyclic is a compound comprising an optionally substituted heterocycloalkyl moiety or an optionally substituted heteroaryl moiety. In another aspect, the optionally substituted heterocycloalkyl moiety or optionally substituted heteroaryl moiety is an optionally substituted 5-membered heterocycloalkyl or an optionally substituted 5-membered heteroaryl moiety. In another aspect, the optionally substituted heterocycloalkyl or optionally substituted heteroaryl moiety is an optionally substituted moiety selected from pyrazolyl, furanyl, imidazolyl, isoxazolyl, oxadiazolyl, oxazolyl, pyrrolyl, pyridyl, pyrimidyl, pyridazinyl, thiazolyl, triazolyl, thienyl, dihydrothieno-pyrazolyl, thianaphthenyl, carbazolyl, benzimidazolyl, benzothienyl, benzofuranyl, indolyl, quinolinyl, benzotriazolyl, benzothiazolyl, benzooxazolyl, benzimidazolyl, isoquinolinyl, isoindolyl, acridinyl, benzoisazolyl, dimethylhydantoin, pyrazinyl, tetrahydrofuranyl, pyrrolinyl, pyrrolidinyl, morpholinyl, indolyl, diazepinyl, azepinyl, thiepinyl, piperidinyl, and oxepinyl. In another aspect, the optionally substituted heterocycloalkyl moiety or optionally substituted heteroaryl moiety is an optionally substituted furanyl. Non-limiting examples of the heterocyclic compounds include (1,2-dihydroxyethyl)-3,4-dihydroxyfuran-2(5H)-one; 4-hydroxy-5-methyl-3-furanone; 5-hydroxy-2(5H)-furanone; [1,2-dihydroxyethyl]furan-2,3,4(5H)-trione; $\alpha$-hydroxy-$\gamma$-butyrolactone; ribonic $\gamma$-lactone; aldohexuronicaldohexuronic acid $\gamma$-lactone; gluconic acid $\delta$-lactone; 4-hydroxycoumarin; dihydrobenzofuran; 5-(hydroxymethyl)furfural; furoin; 2(5H)-furanone; 5,6-dihydro-2H-pyran-2-one; and 5,6-dihydro-4-hydroxy-6-methyl-2H-pyran-2-one; or a salt or solvate thereof.

The nitrogen-containing compound may be any suitable compound with one or more nitrogen atoms. In one aspect, the nitrogen-containing compound comprises an amine, imine, hydroxylamine, or nitroxide moiety. Non-limiting examples of the nitrogen-containing compounds include acetone oxime; violuric acid; pyridine-2-aldoxime; 2-aminophenol; 1,2-benzenediamine; 2,2,6,6-tetramethyl-1-piperidinyloxy; 5,6,7,8-tetrahydrobiopterin; 6,7-dimethyl-5,6,7,8-tetrahydropterine; and maleamic acid; or a salt or solvate thereof.

The quinone compound may be any suitable compound comprising a quinone moiety as described herein. Non-limiting examples of the quinone compounds include 1,4-benzoquinone; 1,4-naphthoquinone; 2-hydroxy-1,4-naphthoquinone; 2,3-dimethoxy-5-methyl-1,4-benzoquinone or coenzyme $Q_0$; 2,3,5,6-tetramethyl-1,4-benzoquinone or duroquinone; 1,4-dihydroxyanthraquinone; 3-hydroxy-1-methyl-5,6-indolinedione or adrenochrome; 4-tert-butyl-5-methoxy-1,2-benzoquinone; pyrroloquinoline quinone; or a salt or solvate thereof.

The sulfur-containing compound may be any suitable compound comprising one or more sulfur atoms. In one aspect, the sulfur-containing comprises a moiety selected from thionyl, thioether, sulfinyl, sulfonyl, sulfamide, sulfonamide, sulfonic acid, and sulfonic ester. Non-limiting examples of the sulfur-containing compounds include ethanethiol; 2-propanethiol; 2-propene-1-thiol; 2-mercaptoethanesulfonic acid; benzenethiol; benzene-1,2-dithiol; cysteine; methionine; glutathione; cystine; or a salt or solvate thereof.

In one aspect, an effective amount of such a compound described above to cellulosic material as a molar ratio to glucosyl units of cellulose is about $10^{-6}$ to about 10, *e.g.*, about $10^{-6}$ to about 7.5, about $10^{-6}$ to about 5, about $10^{-6}$ to about 2.5, about $10^{-6}$ to about 1, about $10^{-5}$ to about 1, about $10^{-5}$ to about $10^{-1}$, about $10^{-4}$ to about $10^{-1}$, about $10^{-3}$ to about $10^{-1}$, or about $10^{-3}$ to about $10^{-2}$. In another aspect, an effective amount of such a compound described above is about 0.1 $\mu$M to about 1 M, *e.g.*, about 0.5 $\mu$M to about 0.75 M, about 0.75 $\mu$M to about 0.5 M, about 1 $\mu$M to about 0.25 M, about 1 $\mu$M to about 0.1 M, about 5 $\mu$M to about 50 mM, about 10 $\mu$M to about 25 mM, about 50 $\mu$M to about 25 mM, about 10 $\mu$M to about 10 mM, about 5 $\mu$M to about 5 mM, or about 0.1 mM to about 1 mM.

The term "liquor" means the solution phase, either aqueous, organic, or a combination thereof, arising from treatment of a lignocellulose and/or hemicellulose material in a slurry, or monosaccharides thereof, *e.g.,* xylose, arabinose, mannose, *etc.*, under conditions as described herein, and the soluble contents thereof. A liquor for cellulolytic enhancement of a GH61 polypeptide can be produced by treating a lignocellulose or hemicellulose material (or feedstock) by applying heat and/or pressure, optionally in the presence of a catalyst, *e.g.*, acid, optionally in the presence of an organic solvent, and optionally in combination with physical disruption of the material, and then separating the solution from the residual solids. Such conditions determine the degree of cellulolytic enhancement obtainable through the combination of liquor and a GH61 polypeptide during hydrolysis of a cellulosic substrate by a cellulase preparation. The liquor can be separated from the treated material using a method standard in the art, such as filtration, sedimentation, or centrifugation.

In one aspect, an effective amount of the liquor to cellulose is about $10^{-6}$ to about 10 g per g of cellulose, *e.g.*, about $10^{-6}$ to about 7.5 g, about $10^{-6}$ to about 5 g, about $10^{-6}$ to about 2.5 g, about $10^{-6}$ to about 1 g, about $10^{-5}$ to about 1 g, about $10^{-5}$ to about $10^{-1}$ g, about $10^{-4}$ to about $10^{-1}$ g, about $10^{-3}$ to about $10^{-1}$ g, or about $10^{-3}$ to about $10^{-2}$ g per g of cellulose.

In one aspect, the one or more (*e.g.*, several) hemicellulolytic enzymes comprise a commercial hemicellulolytic enzyme preparation. Examples of commercial hemicellulolytic enzyme preparations suitable for use in the present invention include, for example, SHEARZYME™ (Novozymes A/S), CELLIC® HTec (Novozymes A/S), CELLIC® HTec2 (Novozymes A/S), CELLIC® HTec3 (Novozymes A/S), VISCOZYME® (Novozymes A/S), ULTRAFLO® (Novozymes A/S), PULPZYME® HC (Novozymes A/S), MULTIFECT® Xylanase (Genencor), ACCELLERASE® XY (Genencor), ACCELLERASE® XC (Genencor), ECOPULP® TX-200A (AB Enzymes), HSP 6000 Xylanase (DSM), DEPOL™ 333P (Biocatalysts Limit, Wales, UK), DEPOL™ 740L. (Biocatalysts Limit, Wales, UK), and DEPOL™ 762P (Biocatalysts Limit, Wales, UK).

Examples of xylanases useful in the processes of the present invention include, but are not limited to, xylanases from *Aspergillus aculeatus* (GeneSeqP:AAR63790; WO 94/21785), *Aspergillus fumigatus* (WO 2006/078256), *Penicillium pinophilum* (WO 2011/041405), *Penicillium* sp. (WO 2010/126772), *Thielavia terrestris* NRRL 8126 (WO 2009/079210), and *Trichophaea saccata* GH10 (WO 2011/057083).

Examples of beta-xylosidases useful in the processes of the present invention include, but are not limited to, beta-xylosidases from *Neurospora crassa* (SwissProt accession number Q7SOW4), *Trichoderma reesei* (UniProtKB/TrEMBL accession number Q92458), and *Talaromyces emersonii* (SwissProt accession number Q8X212).

Examples of acetylxylan esterases useful in the processes of the present invention include, but are not limited to, acetylxylan esterases from *Aspergillus aculeatus* (WO 2010/108918), *Chaetomium globosum* (Uniprot accession number Q2GWX4), *Chaetomium gracile* (GeneSeqP accession number AAB82124), *Humicola insolens* DSM 1800 (WO 2009/073709), *Hypocrea jecorina* (WO 2005/001036), *Myceliophtera thermophila* (WO 2010/014880), *Neurospora crassa* (UniProt accession number q7s259), *Phaeosphaeria nodorum* (Uniprot accession number Q0UHJ1) and *Thielavia terrestris* NRRL 8126 (WO 2009/042846).

Examples of feruloyl esterases (ferulic acid esterases) useful in the processes of the present invention include, but are not limited to, feruloyl esterases form *Humicola insolens* DSM 1800 (WO 2009/076122), *Neosartorya fischeri* (UniProt Accession number A1D9T4), *Neurospora crassa* (UniProt accession number Q9HGR3), *Penicillium aurantiogriseum* (WO 2009/127729), and *Thielavia terrestris* (WO 2010/053838 and WO 2010/065448).

Examples of arabinofuranosidases useful in the processes of the present invention include, but are not limited to, arabinofuranosidases from *Aspergillus niger* (GeneSeqP accession number AAR94170), *Humicola insolens* DSM 1800 (WO 2006/114094 and WO 2009/073383), and *M. giganteus* (WO 2006/114094).

Examples of alpha-glucuronidases useful in the processes of the present invention include, but are not limited to, alpha-glucuronidases from *Aspergillus clavatus* (UniProt accession number alcc12), *Aspergillus fumigatus* (SwissProt accession number Q4WW45), *Aspergillus niger* (Uniprot accession number Q96WX9), *Aspergillus terreus* (SwissProt accession number Q0CJP9) *Humicola insolens* (WO 2010/014706), *Penicillium aurantiogriseum* (WO 2009/068565), *Talaromyces emersonii* (UniProt accession number Q8X211), and *Trichoderma reesei* (Uniprot accession number Q99024).

The polypeptides having enzyme activity used in the processes of the present invention may be produced by fermentation of the above-noted microbial strains on a nutrient medium containing suitable carbon and nitrogen sources and inorganic salts, using procedures known in the art (see, *e.g.*, Bennett, J.W. and LaSure, L. (eds.), More Gene Manipulations in Fungi, Academic Press, CA, 1991). Suitable media are available from commercial suppliers or may be prepared according to published compositions (*e.g.*, in catalogues of the American Type Culture Collection). Temperature ranges and other conditions suitable for growth and enzyme production are known in the art (see, *e.g.*, Bailey, J.E., and Ollis, D.F., Biochemical Engineering Fundamentals, McGraw-Hill Book Company, NY, 1986).

The fermentation can be any method of cultivation of a cell resulting in the expression or isolation of an enzyme or protein. Fermentation may, therefore, be understood as comprising shake flask cultivation, or small- or large-scale fermentation (including continuous, batch, fed-batch, or solid state fermentations) in laboratory or industrial fermentors performed in a suitable medium and under conditions allowing the enzyme to be expressed or isolated. The resulting enzymes produced by the methods described above may be recovered from the fermentation medium and purified by conventional procedures.

Fermentation. The fermentable sugars obtained from the hydrolyzed cellulosic material can be fermented by one or more (*e.g.*, several) fermenting microorganisms capable of fermenting the sugars directly or indirectly into a desired fermentation product. "Fermentation" or "fermentation process" refers to any fermentation process or any process comprising a fermentation step. Fermentation processes also include fermentation processes used in the consumable alcohol industry (*e.g.*, beer and wine), dairy industry (*e.g.*, fermented dairy products), leather industry, and tobacco industry. The fermentation conditions depend on the desired fermentation product and fermenting organism and can easily be determined by one skilled in the art.

In the fermentation step, sugars, released from the cellulosic material as a result of the pretreatment and enzymatic hydrolysis steps, are fermented to a product, *e.g.*, ethanol, by a fermenting organism, such as yeast. Hydrolysis (saccharification) and fermentation can be separate or simultaneous, as described herein.

Any suitable hydrolyzed cellulosic material can be used in the fermentation step in practicing the present invention. The material is generally selected based on the desired fermentation product, *i.e.*, the substance to be obtained from the fermentation, and the process employed, as is well known in the art.

The term "fermentation medium" is understood herein to refer to a medium before the fermenting microorganism(s) is(are) added, such as, a medium resulting from a saccharification process, as well as a medium used in a simultaneous saccharification and fermentation process (SSF).

"Fermenting microorganism" refers to any microorganism, including bacterial and fungal organisms, suitable for use in a desired fermentation process to produce a fermentation product. The fermenting organism can be hexose and/or pentose fermenting organisms, or a combination thereof. Both hexose and pentose fermenting organisms are well known in the art. Suitable fermenting microorganisms are able to ferment, *i.e.*, convert, sugars, such as glucose, xylose, xylulose, arabinose, maltose, mannose, galactose, and/or oligosaccharides, directly or indirectly into the desired fermentation product. Examples of bacterial and fungal fermenting organisms producing ethanol are described by Lin et al., 2006, Appl. Microbiol. Biotechnol. 69: 627-642.

Examples of fermenting microorganisms that can ferment hexose sugars include bacterial and fungal organisms, such as yeast. Preferred yeast includes strains of *Candida*, *Kluyveromyces,* and *Saccharomyces*, *e.g.*, *Candida sonorensis*, *Kluyveromyces marxianus*, and *Saccharomyces cerevisiae*.

Examples of fermenting organisms that can ferment pentose sugars in their native state include bacterial and fungal organisms, such as some yeast. Preferred xylose fermenting yeast include strains of *Candida,* preferably *C. sheatae* or *C. sonorensis*; and strains of *Pichia*, preferably *P. stipitis*, such as *P. stipitis* CBS 5773. Preferred pentose fermenting yeast include strains of *Pachysolen,* preferably *P. tannophilus.* Organisms not capable of fermenting pentose sugars, such as xylose and arabinose, may be genetically modified to do so by methods known in the art.

Examples of bacteria that can efficiently ferment hexose and pentose to ethanol include, for example, *Bacillus coagulans*, *Clostridium acetobutylicum*, *Clostridium thermocellum*, *Clostridium phytofermentans*, *Geobacillus* sp., *Thermoanaerobacter saccharolyticum*, and *Zymomonas mobilis* (Philippidis, 1996, *supra*).

Other fermenting organisms include strains of *Bacillus*, such as *Bacillus coagulans*; *Candida*, such as *C. sonorensis*, *C. methanosorbosa, C. diddensiae*, *C. parapsilosis, C. naedodendra*, *C. blankii*, *C. entomophilia, C. brassicae, C. pseudotropicalis*, *C. boidinii, C. utilis,* and *C. scehatae; Clostridium*, such as *C. acetobutylicum*, *C. thermocellum*, and *C. phytofermentans*; *E. coli*, especially *E. coli* strains that have been genetically modified to improve the yield of ethanol; *Geobacillus* sp.; *Hansenula*, such as *Hansenula anomala*; *Klebsiella,* such as *K. oxytoca*; *Kluyveromyces*, such as *K. marxianus*, *K. lactis*, *K. thermotolerans,* and *K. fragilis*; *Schizosaccharomyces*, such as *S. pombe*; *Thermoanaerobacter*, such as *Thermoanaerobacter saccharolyticum*; and *Zymomonas*, such as *Zymomonas mobilis.*

In a preferred aspect, the yeast is a *Bretannomyces.* In a more preferred aspect, the yeast is *Bretannomyces clausenii.* In another preferred aspect, the yeast is a *Candida.* In another more preferred aspect, the yeast is *Candida sonorensis.* In another more preferred aspect, the yeast is *Candida boidinii.* In another more preferred aspect, the yeast is *Candida blankii.* In another more preferred aspect, the yeast is *Candida brassicae.* In another more preferred aspect, the yeast is *Candida diddensii.* In another more preferred aspect, the yeast is *Candida entomophiliia.* In another more preferred aspect, the yeast is *Candida pseudotropicalis.* In another more preferred aspect, the yeast is *Candida scehatae.* In another more preferred aspect, the yeast is *Candida utilis.* In another preferred aspect, the yeast is a *Clavispora.* In another more preferred aspect, the yeast is *Clavispora lusitaniae.* In another more preferred aspect, the yeast is *Clavispora opuntiae.* In another preferred aspect, the yeast is a *Kluyveromyces.* In another more preferred aspect, the yeast is *Kluyveromyces fragilis.* In another more preferred aspect, the yeast is *Kluyveromyces marxianus.* In another more preferred aspect, the yeast is *Kluyveromyces thermotolerans.* In another preferred aspect, the yeast is a *Pachysolen.* In another more preferred aspect, the yeast is *Pachysolen tannophilus.* In another preferred aspect, the yeast is a *Pichia.* In another more preferred aspect, the yeast is a *Pichia stipitis.* In another preferred aspect, the yeast is a *Saccharomyces* spp. In another more preferred aspect, the yeast is *Saccharomyces cerevisiae.* In another more preferred aspect, the yeast is *Saccharomyces distaticus.* In another more preferred aspect, the yeast is *Saccharomyces uvarum.*

In a preferred aspect, the bacterium is a *Bacillus.* In a more preferred aspect, the bacterium is *Bacillus coagulans.* In another preferred aspect, the bacterium is a *Clostridium.* In another more preferred aspect, the bacterium is *Clostridium acetobutylicum.* In another more preferred aspect, the bacterium is *Clostridium phytofermentans.* In another more preferred aspect, the bacterium is *Clostridium thermocellum.* In another more preferred aspect, the bacterium is *Geobacilus* sp. In another more preferred aspect, the bacterium is a *Thermoanaerobacter.* In another more preferred aspect, the bacterium is *Thermoanaerobacter saccharolyticum.* In another preferred aspect, the bacterium is a *Zymomonas.* In another more preferred aspect, the bacterium is *Zymomonas mobilis.*

Commercially available yeast suitable for ethanol production include, *e.g.*, BIOFERM™ AFT and XR (NABC - North American Bioproducts Corporation, GA, USA), ETHANOL RED™ yeast (Fermentis/Lesaffre, USA), FALI™ (Fleischmann's Yeast, USA), FERMIOL™ (DSM Specialties), GERT STRAND™ (Gert Strand AB, Sweden), and SUPERSTART™ and THERMOSACC™ fresh yeast (Ethanol Technology, WI, USA).

In a preferred aspect, the fermenting microorganism has been genetically modified to provide the ability to ferment pentose sugars, such as xylose utilizing, arabinose utilizing, and xylose and arabinose co-utilizing microorganisms.

The cloning of heterologous genes into various fermenting microorganisms has led to the construction of organisms capable of converting hexoses and pentoses to ethanol (co-fermentation) (Chen and Ho, 1993, Cloning and improving the expression of Pichia stipitis xylose reductase gene in Saccharomyces cerevisiae, Appl. Biochem. Biotechnol. 39-40: 135-147; Ho et al., 1998, Genetically engineered Saccharomyces yeast capable of effectively cofermenting glucose and xylose, Appl. Environ. Microbiol. 64: 1852-1859; Kotter and Ciriacy, 1993, Xylose fermentation by Saccharomyces cerevisiae, Appl. Microbiol. Biotechnol. 38: 776-783; Walfridsson et al., 1995, Xylose-metabolizing Saccharomyces cerevisiae strains overexpressing the TKL1 and TAL1 genes encoding the pentose phosphate pathway enzymes transketolase and transaldolase, Appl. Environ. Microbiol. 61: 4184-4190; Kuyper et al., 2004, Minimal metabolic engineering of Saccharomyces cerevisiae for efficient anaerobic xylose fermentation: a proof principle, FEMS Yeast Research 4: 655-664; Beall et al., 1991, Parametric studies of ethanol production from xylose and other sugars by recombinant Escherichia coli, Biotech. Bioeng. 38: 296-303; Ingram et al., 1998, Metabolic engineering of bacteria for ethanol production, Biotechnol. Bioeng. 58: 204-214; Zhang et al., 1995, Metabolic engineering of a pentose metabolism pathway in ethanologenic Zymomonas mobilis, Science 267: 240-243; Deanda et al., 1996, Development of an arabinose-fermenting Zymomonas mobilis strain by metabolic pathway engineering, Appl. Environ. Microbiol. 62: 4465-4470; WO 2003/062430, xylose isomerase).

In a preferred aspect, the genetically modified fermenting microorganism is *Candida sonorensis*. In another preferred aspect, the genetically modified fermenting microorganism is *Escherichia coli*. In another preferred aspect, the genetically modified fermenting microorganism is *Klebsiella oxytoca.* In another preferred aspect, the genetically modified fermenting microorganism is *Kluyveromyces marxianus.* In another preferred aspect, the genetically modified fermenting microorganism is *Saccharomyces cerevisiae*. In another preferred aspect, the genetically modified fermenting microorganism is *Zymomonas mobilis.*

It is well known in the art that the organisms described above can also be used to produce other substances, as described herein.

The fermenting microorganism is typically added to the degraded cellulosic material or hydrolysate and the fermentation is performed for about 8 to about 96 hours, *e.g.*, about 24 to about 60 hours. The temperature is typically between about 26°C to about 60°C, *e.g.*, about 32°C or 50°C, and about pH 3 to about pH 8, *e.g.*, pH 4-5, 6, or 7.

In one aspect, the yeast and/or another microorganism are applied to the degraded cellulosic material and the fermentation is performed for about 12 to about 96 hours, such as typically 24-60 hours. In another aspect, the temperature is preferably between about 20°C to about 60°C, *e.g.*, about 25°C to about 50°C, about 32°C to about 50°C, or about 32°C to about 50°C, and the pH is generally from about pH 3 to about pH 7, *e.g.*, about pH 4 to about pH 7. However, some fermenting organisms, *e.g.*, bacteria, have higher fermentation temperature optima. Yeast or another microorganism is preferably applied in amounts of approximately $10^5$ to $10^{12}$, preferably from approximately $10^7$ to $10^{10}$, especially approximately 2 x $10^8$ viable cell count per ml of fermentation broth. Further guidance in respect of using yeast for fermentation can be found in, *e.g.*, "The Alcohol Textbook" (Editors K. Jacques, T.P. Lyons and D.R. Kelsall, Nottingham University Press, United Kingdom 1999).

A fermentation stimulator can be used in combination with any of the methods described herein to further improve the fermentation process, and in particular, the performance of the fermenting microorganism, such as, rate enhancement and ethanol yield. A "fermentation stimulator" refers to stimulators for growth of the fermenting microorganisms, in particular, yeast. Preferred fermentation stimulators for growth include vitamins and minerals. Examples of vitamins include multivitamins, biotin, pantothenate, nicotinic acid, meso-inositol, thiamine, pyridoxine, para-aminobenzoic acid, folic acid, riboflavin, and Vitamins A, B, C, D, and E. See, for example, Alfenore et al., Improving ethanol production and viability of Saccharomyces cerevisiae by a vitamin feeding strategy during fed-batch process, Springer-Verlag (2002). Examples of minerals include minerals and mineral salts that can supply nutrients comprising P, K, Mg, S, Ca, Fe, Zn, Mn, and Cu.

Fermentation products: A fermentation product can be any substance derived from the fermentation. The fermentation product can be, without limitation, an alcohol (*e.g.*, arabinitol, n-butanol, isobutanol, ethanol, glycerol, methanol, ethylene glycol, 1,3-propanediol [propylene glycol], butanediol, glycerin, sorbitol, and xylitol); an alkane (*e.g.*, pentane, hexane, heptane, octane, nonane, decane, undecane, and dodecane), a cycloalkane (*e.g.*, cyclopentane, cyclohexane, cycloheptane, and cyclooctane), an alkene (*e.g.* pentene, hexene, heptene, and octene); an amino acid (*e.g.*, aspartic acid, glutamic acid, glycine, lysine, serine, and threonine); a gas (*e.g.*, methane, hydrogen ($H_2$), carbon dioxide ($CO_2$), and carbon monoxide (CO)); isoprene; a ketone (*e.g.*, acetone); an organic acid (*e.g.*, acetic acid, acetonic acid, adipic acid, ascorbic acid, citric acid, 2,5-diketo-D-gluconic acid, formic acid, fumaric acid, glucaric acid, gluconic acid, glucuronic acid, glutaric acid, 3-hydroxypropionic acid, itaconic acid, lactic acid, malic acid, malonic acid, oxalic acid, oxaloacetic acid, propionic acid, succinic acid, and xylonic acid); and polyketide. The fermentation product can also be protein as a high value product.

In a preferred aspect, the fermentation product is an alcohol. It will be understood that the term "alcohol" encompasses a substance that contains one or more hydroxyl moieties. In a more preferred aspect, the alcohol is n-butanol. In another more preferred aspect, the alcohol is isobutanol. In another more preferred aspect, the alcohol is ethanol. In another more preferred aspect, the alcohol is methanol. In another more preferred aspect, the alcohol is arabinitol. In another more preferred aspect, the alcohol is butanediol. In another more preferred aspect, the alcohol is ethylene glycol. In another more preferred aspect, the alcohol is glycerin. In another more preferred aspect, the alcohol is glycerol. In another more preferred aspect, the alcohol is 1,3-propanediol. In another more preferred aspect, the alcohol is sorbitol. In another more preferred aspect, the alcohol is xylitol. See, for example, Gong et al., 1999, Ethanol production from renewable resources, in Advances in Biochemical Engineering/Biotechnology, Scheper, T., ed., Springer-Verlag Berlin Heidelberg, Germany, 65: 207-241; Silveira and Jonas, 2002, The biotechnological production of sorbitol, Appl. Microbiol. Biotechnol. 59: 400-408; Nigam and Singh, 1995, Processes for fermentative production of xylitol - a sugar substitute, Process Biochemistry 30 (2): 117-124; Ezeji et al., 2003, Production of acetone, butanol and ethanol by Clostridium beijerinckii BA101 and in situ recovery by gas stripping, World Journal of Microbiology and Biotechnology 19 (6): 595-603.

In another preferred aspect, the fermentation product is an alkane. The alkane can be an unbranched or a branched alkane. In another more preferred aspect, the alkane is pentane. In another more preferred aspect, the alkane is hexane. In another more preferred aspect, the alkane is heptane. In another more preferred aspect, the alkane is octane. In another more preferred aspect, the alkane is nonane. In another more preferred aspect, the alkane is decane. In another more preferred aspect, the alkane is undecane. In another more preferred aspect, the alkane is dodecane.

In another preferred aspect, the fermentation product is a cycloalkane. In another more preferred aspect, the cycloalkane is cyclopentane. In another more preferred aspect, the cycloalkane is cyclohexane. In another more preferred aspect, the cycloalkane is cycloheptane. In another more preferred aspect, the cycloalkane is cyclooctane.

In another preferred aspect, the fermentation product is an alkene. The alkene can be an unbranched or a branched alkene. In another more preferred aspect, the alkene is pentene. In another more preferred aspect, the alkene is hexene. In another more preferred aspect, the alkene is heptene. In another more preferred aspect, the alkene is octene.

In another preferred aspect, the fermentation product is an amino acid. In another more preferred aspect, the organic acid is aspartic acid. In another more preferred aspect, the amino acid is glutamic acid. In another more preferred aspect, the amino acid is glycine. In another more preferred aspect, the amino acid is lysine. In another more preferred aspect, the amino acid is serine. In another more preferred aspect, the amino acid is threonine. See, for example, Richard and Margaritis, 2004, Empirical modeling of batch fermentation kinetics for poly(glutamic acid) production and other microbial biopolymers, Biotechnology and Bioengineering 87 (4): 501-515.

In another preferred aspect, the fermentation product is a gas. In another more preferred aspect, the gas is methane. In another more preferred aspect, the gas is $H_2$. In another more preferred aspect, the gas is $CO_2$. In another more preferred aspect, the gas is CO. See, for example, Kataoka et al., 1997, Studies on hydrogen production by continuous culture system of hydrogen-producing anaerobic bacteria, Water Science and Technology 36 (6-7): 41-47; and Gunaseelan, 1997, Biomass and Bioenerg 13(1-2): 83-114, Anaerobic digestion of biomass for methane production: A review.

In another preferred aspect, the fermentation product is isoprene.

In another preferred aspect, the fermentation product is a ketone. It will be understood that the term "ketone" encompasses a substance that contains one or more ketone moieties. In another more preferred aspect, the ketone is acetone. See, for example, Qureshi and Blaschek, 2003, *supra.*

In another preferred aspect, the fermentation product is an organic acid. In another more preferred aspect, the organic acid is acetic acid. In another more preferred aspect, the organic acid is acetonic acid. In another more preferred aspect, the organic acid is adipic acid. In another more preferred aspect, the organic acid is ascorbic acid. In another more preferred aspect, the organic acid is citric acid. In another more preferred aspect, the organic acid is 2,5-diketo-D-gluconic acid. In another more preferred aspect, the organic acid is formic acid. In another more preferred aspect, the organic acid is fumaric acid. In another more preferred aspect, the organic acid is glucaric acid. In another more preferred aspect, the organic acid is gluconic acid. In another more preferred aspect, the organic acid is glucuronic acid. In another more preferred aspect, the organic acid is glutaric acid. In another preferred aspect, the organic acid is 3-hydroxypropionic acid. In another more preferred aspect, the organic acid is itaconic acid. In another more preferred aspect, the organic acid is lactic acid. In another more preferred aspect, the organic acid is malic acid. In another more preferred aspect, the organic acid is malonic acid. In another more preferred aspect, the organic acid is oxalic acid. In another more preferred aspect, the organic acid is propionic acid. In another more preferred aspect, the organic acid is succinic acid. In another more preferred aspect, the organic acid is xylonic acid. See, for example, Chen and Lee, 1997, Membrane-mediated extractive fermentation for lactic acid production from cellulosic biomass, Appl. Biochem. Biotechnol. 63-65: 435-448.

In another preferred aspect, the fermentation product is polyketide.

Recovery. The fermentation product(s) can be optionally recovered from the fermentation medium using any method known in the art including, but not limited to, chromatography, electrophoretic procedures, differential solubility, distillation, or extraction. For example, alcohol is separated from the fermented cellulosic material and purified by conventional methods of distillation. Ethanol with a purity of up to about 96 vol.% can be obtained, which can be used as, for example, fuel ethanol, drinking ethanol, *i.e.*, potable neutral spirits, or industrial ethanol.

**Plants**

The present disclosure also relates to isolated plants, *e.g.*, a transgenic plant, plant part, or plant cell, comprising a polynucleotide of the present invention so as to express and produce a chimeric beta-glucosidase or a variant thereof in recoverable quantities. The chimeric beta-glucosidase or the variant thereof may be recovered from the plant or plant part. Alternatively, the plant or plant part containing the chimeric beta-glucosidase or the variant thereof may be used as such for improving the quality of a food or feed, *e.g.*, improving nutritional value, palatability, and rheological properties, or to destroy an antinutritive factor.

The transgenic plant can be dicotyledonous (a dicot) or monocotyledonous (a monocot). Examples of monocot plants are grasses, such as meadow grass (blue grass, Poa), forage grass such as Festuca, Lolium, temperate grass, such as Agrostis, and cereals, *e.g.*, wheat, oats, rye, barley, rice, sorghum, and maize (corn).

Examples of dicot plants are tobacco, legumes, such as lupins, potato, sugar beet, pea, bean and soybean, and cruciferous plants (family Brassicaceae), such as cauliflower, rape seed, and the closely related model organism *Arabidopsis thaliana.*

Examples of plant parts are stem, callus, leaves, root, fruits, seeds, and tubers as well as the individual tissues comprising these parts, *e.g.*, epidermis, mesophyll, parenchyme, vascular tissues, meristems. Specific plant cell compartments, such as chloroplasts, apoplasts, mitochondria, vacuoles, peroxisomes and cytoplasm are also considered to be a plant part. Furthermore, any plant cell, whatever the tissue origin, is considered to be a plant part. Likewise, plant parts such as specific tissues and cells isolated to facilitate the utilization of the invention are also considered plant parts, *e.g.*, embryos, endosperms, aleurone and seed coats.

Also included within the scope of the present disclosure are the progeny of such plants, plant parts, and plant cells.

The transgenic plant or plant cell expressing a chimeric beta-glucosidase or a variant thereof may be constructed in accordance with methods known in the art. In short, the plant or plant cell is constructed by incorporating one or more (*e.g.*, several) expression constructs encoding a chimeric beta-glucosidase or a variant thereof into the plant host genome or chloroplast genome and propagating the resulting modified plant or plant cell into a transgenic plant or plant cell.

The expression construct is conveniently a nucleic acid construct that comprises a polynucleotide encoding a chimeric beta-glucosidase or a variant thereof operably linked with appropriate regulatory sequences required for expression of the polynucleotide in the plant or plant part of choice. Furthermore, the expression construct may comprise a selectable marker useful for identifying plant cells into which the expression construct has been integrated and DNA sequences necessary for introduction of the construct into the plant in question (the latter depends on the DNA introduction method to be used).

The choice of regulatory sequences, such as promoter and terminator sequences and optionally signal or transit sequences, is determined, for example, on the basis of when, where, and how the chimeric beta-glucosidase or the variant thereof is desired to be expressed. For instance, the expression of the gene encoding a chimeric beta-glucosidase or a variant thereof may be constitutive or inducible, or may be developmental, stage or tissue specific, and the gene product may be targeted to a specific tissue or plant part such as seeds or leaves. Regulatory sequences are, for example, described by Tague et al., 1988, Plant Physiol. 86: 506.

For constitutive expression, the 35S-CaMV, the maize ubiquitin 1, and the rice actin 1 promoter may be used (Franck et al., 1980, Cell 21: 285-294; Christensen et al., 1992, Plant Mol. Biol. 18: 675-689; Zhang et al., 1991, Plant Cell 3: 1155-1165). Organ-specific promoters may be, for example, a promoter from storage sink tissues such as seeds, potato tubers, and fruits (Edwards and Coruzzi, 1990, Ann. Rev. Genet. 24: 275-303), or from metabolic sink tissues such as meristems (Ito et al., 1994, Plant Mol. Biol. 24: 863-878), a seed specific promoter such as the glutelin, prolamin, globulin, or albumin promoter from rice (Wu et al., 1998, Plant Cell Physiol. 39: 885-889), a *Vicia faba* promoter from the legumin B4 and the unknown seed protein gene from *Vicia faba* (Conrad et al., 1998, J. Plant Physiol. 152: 708-711), a promoter from a seed oil body protein (Chen et al., 1998, Plant Cell Physiol. 39: 935-941), the storage protein *napA* promoter from *Brassica napus,* or any other seed specific promoter known in the art, *e.g.*, as described in WO 91/14772. Furthermore, the promoter may be a leaf specific promoter such as the *rbcs* promoter from rice or tomato (Kyozuka et al., 1993, Plant Physiol. 102: 991-1000), the chlorella virus adenine methyltransferase gene promoter (Mitra and Higgins, 1994, Plant Mol. Biol. 26: 85-93), the *aldP* gene promoter from rice (Kagaya et al., 1995, Mol. Gen. Genet. 248: 668-674), or a wound inducible promoter such as the potato *pin2* promoter (Xu et al., 1993, Plant Mol. Biol. 22: 573-588). Likewise, the promoter may be induced by abiotic treatments such as temperature, drought, or alterations in salinity or induced by exogenously applied substances that activate the promoter, e.g., ethanol, oestrogens, plant hormones such as ethylene, abscisic acid, and gibberellic acid, and heavy metals.

A promoter enhancer element may also be used to achieve higher expression of a chimeric beta-glucosidase or a variant thereof in the plant. For instance, the promoter enhancer element may be an intron that is placed between the promoter and the polynucleotide encoding a chimeric beta-glucosidase or a variant thereof. For instance, Xu *et al.*, 1993, *supra*, disclose the use of the first intron of the rice actin 1 gene to enhance expression.

The selectable marker gene and any other parts of the expression construct may be chosen from those available in the art.

The nucleic acid construct is incorporated into the plant genome according to conventional techniques known in the art, including *Agrobacterium*-mediated transformation, virus-mediated transformation, microinjection, particle bombardment, biolistic transformation, and electroporation (Gasser et al., 1990, Science 244: 1293; Potrykus, 1990, Bio/Technology 8: 535; Shimamoto et al., 1989, Nature 338: 274).

*Agrobacterium tumefaciens*-mediated gene transfer is a method for generating transgenic dicots (for a review, see Hooykas and Schilperoort, 1992, Plant Mol. Biol. 19: 15-38) and for transforming monocots, although other transformation methods may be used for these plants. A method for generating transgenic monocots is particle bombardment (microscopic gold or tungsten particles coated with the transforming DNA) of embryonic calli or developing embryos (Christou, 1992, Plant J. 2: 275-281; Shimamoto, 1994, Curr. Opin. Biotechnol. 5: 158-162; Vasil et al., 1992, Bio/Technology 10: 667-674). An alternative method for transformation of monocots is based on protoplast transformation as described by Omirulleh et al., 1993, Plant Mol. Biol. 21: 415-428. Additional transformation methods include those described in U.S. Patent Nos. 6,395,966 and 7,151,204.

Following transformation, the transformants having incorporated the expression construct are selected and regenerated into whole plants according to methods well known in the art. Often the transformation procedure is designed for the selective elimination of selection genes either during regeneration or in the following generations by using, for example, co-transformation with two separate T-DNA constructs or site specific excision of the selection gene by a specific recombinase.

In addition to direct transformation of a particular plant genotype with a construct prepared of the present disclosure, transgenic plants may be made by crossing a plant having the construct to a second plant lacking the construct. For example, a construct encoding a chimeric beta-glucosidase or a variant thereof can be introduced into a particular plant variety by crossing, without the need for ever directly transforming a plant of that given variety. Therefore, the present disclosure encompasses not only a plant directly regenerated from cells which have been transformed in accordance with the present disclosure, but also the progeny of such plants. As used herein, progeny may refer to the offspring of any generation of a parent plant prepared in accordance with the present disclosure. Such progeny may include a DNA construct prepared in accordance with the present disclosure. Crossing results in the introduction of a transgene into a plant line by cross pollinating a starting line with a donor plant line. Non-limiting examples of such steps are described in U.S. Patent No. 7,151,204.

Plants may be generated through a process of backcross conversion. For example, plants include plants referred to as a backcross converted genotype, line, inbred, or hybrid.

Genetic markers may be used to assist in the introgression of one or more transgenes of the disclosure from one genetic background into another. Marker assisted selection offers advantages relative to conventional breeding in that it can be used to avoid errors caused by phenotypic variations. Further, genetic markers may provide data regarding the relative degree of elite germplasm in the individual progeny of a particular cross. For example, when a plant with a desired trait which otherwise has a non-agronomically desirable genetic background is crossed to an elite parent, genetic markers may be used to select progeny which not only possess the trait of interest, but also have a relatively large proportion of the desired germplasm. In this way, the number of generations required to introgress one or more traits into a particular genetic background is minimized.

The present disclosure also relates to methods of producing a chimeric beta-glucosidase or a variant thereof of the present disclosure comprising: (a) cultivating a transgenic plant or a plant cell comprising a polynucleotide encoding the chimeric beta-glucosidase or the variant thereof under conditions conducive for production of the chimeric beta-glucosidase or the variant thereof; and (b) recovering the chimeric beta-glucosidase or the variant thereof.

The present invention is further described by the following examples.

## Examples

### Plasmids and Strains

Plasmid pDFng116 (SEQ ID NO: 9) was used as the source of DNA encoding the *Aspergillus aculeatus* Family 3A beta-glucosidase. Plasmid pAG114 (SEQ ID NO: 10) was used as the source of DNA encoding the *Aspergillus fumigatus* Family 3A beta-glucosidase. *Aspergillus oryzae* JaL250 strain (WO 99/61651) was used for expression of a chimeric beta-glucosidase activity and a variant thereof.

### Media

PDA plates were composed of 39 grams of potato dextrose agar and deionized water to 1 liter.

MDU2BP medium was composed of 45 g of maltose, 1 g of $MgSO_4 \cdot 7H_2O$, 1 g of NaCl, 2 g of $K_2SO_4$, 12 g of $KH_2PO_4$, 7 g of yeast extract, 2 g of urea, 0.5 ml of AMG trace metals solution, and deionized water to 1 liter, pH to 5.0.

AMG trace metals solution was composed of 14.3 g of $ZnSO_4 \cdot 7H_2O$, 2.5 g of $CuSO_4 \cdot 5H_2O$, 0.5 g of $NiCl_2 \cdot 6H_2O$, 13.8 g of $FeSO_4 \cdot 7H_2O$, 8.5 g of $MnSO_4 \cdot H_2O$, 3 g of citric acid, and deionized water to 1 liter.

### Example 1: Preparation and purification of *Aspergillus aculeatus* GH3A beta-glucosidase and *Aspergillus fumigatus* GH3A beta-glucosidase

The *A. aculeatus* GH3A beta glucosidase (SEQ ID NO: 1 [DNA sequence] and SEQ ID NO: 2 [deduced amino acid sequence]) shown in Figures 1A and 1B was prepared according to WO 2011/057140. The *A. fumigatus* GH3A beta-glucosidase (SEQ ID NO: 3 [DNA sequence] and SEQ ID NO: 4 [deduced amino acid sequence]) shown in Figures 2A and 2B was prepared according to U.S. Patent No. 7,244,605. Protein concentration was determined using a Microplate BCA™ Protein Assay Kit (Thermo Fischer Scientific, Waltham, MA, USA) in which bovine serum albumin was used as a protein standard.

Broth supernatant containing either the *Aspergillus fumigatus* GH3A beta-glucosidase or the *Aspergillus aculeatus* GH3A beta-glucosidase was concentrated and desalted using ECONOPAC® 10 DG columns (Bio-Rad Laboratories, Inc., Hercules, CA, USA) equilibrated with 50 mM sodium acetate pH 5.0, containing 100 mM NaCl.

### Example 2: Construction of an *Aspergillus fumigatus* GH3A beta-glucosidase and *Aspergillus aculeatus* GH3A beta-glucosidase chimeric polypeptide

A chimeric polypeptide having beta-glucosidase activity composed of fragments of the *Aspergillus fumigatus* GH3A beta-glucosidase and the *Aspergillus aculeatus* GH3A beta-glucosidase was constructed and expressed in *Aspergillus oryzae* JaL250. The beta-glucosidase chimeric polypeptide is composed of three different fragments of the *A. fumigatus* GH3A beta-glucosidase and the *A. aculeatus* GH3A beta-glucosidase. The first GH3A fragment contains amino acids 1 to 404 of SEQ ID NO: 2 of the *A. aculeatus* GH3A beta-glucosidase (amino acids 1 to 19 are the signal peptide), the second GH3A fragment contains amino acids 406 to 589 of SEQ ID NO: 4 of the *A. fumigatus* GH3A beta-glucosidase, and the third GH3A fragment contains amino acids 589 to 860 of SEQ ID NO: 2 of the *A. aculeatus* GH3A beta-glucosidase.

The polynucleotides encoding the first and third fragments were PCR amplified from plasmid pAG114 (Figure 3), and the polynucleotide encoding the second fragment was PCR amplified from plasmid pDFng116 (Figure 4). The PCR amplified sections for the first, second, and third beta-glucosidase polypeptide fragments were then subcloned simultaneously into plasmid pAILo2 (WO 2004/099228) gapped with *Pac* I and *Nco* I according to the method of Zhu et al., 2007, BioTechniques 43: 354-359 using an IN-FUSION™ Advantage PCR Cloning Kit (Clontech Laboratories, Inc., Mountain View, CA, USA). The primers shown below were designed to amplify the polynucleotide for the first polypeptide fragment from the 5' end of the polynucleotide in plasmid pAG114 encoding the *A. aculeatus* GH3A beta-glucosidase.

Forward primer (610232):

5'-ATACACAACTGGATTTACATGAAGCTCAGTTGGCTTGAGGC-3' (SEQ ID NO: 11)

Reverse primer (69937):

5'-AAGAGCACCCGTGTTCTTCAGTAGAACAGTACTGTCTGC-3' (SEQ ID NO: 12)

Primer 610232 was designed to prime 18 bp upstream of the unique *Nco* I restriction enzyme site on plasmid pAILo2 and the 23 nucleotides corresponding to amino acids 1-8 of the *A. aculeatus* GH3A beta-glucosidase. Primer 69937 was designed to contain a 24 bp region corresponding to amino acids 397-404 of the *A. aculeatus* GH3A beta-glucosidase in addition to a 15 bp region corresponding to amino acids 406-410 of the *A. fumigatus* GH3A beta-glucosidase.

The primers shown below were designed to amplify the polynucleotide for the second polypeptide fragment from plasmid pDFng116 encoding the *A. fumigatus* GH3A beta-glucosidase.

Forward primer (69938):

5'-ACTGTTCTACTGAAGAACACGGGTGCTCTTCCTTTGACC-3' (SEQ ID NO: 13)

Reverse primer (69939):

5'-GCCCGGGTTGACGCGGCCATAGAGCACGTCGACCAGGGA-3' (SEQ ID NO: 14)

Primer 69938 was designed to contain a 15 bp region encoding amino acids 399-404 of the *A. aculeatus* GH3A beta-glucosidase in addition to a 24 bp region encoding amino acids 406-410 of the *A. fumigatus* GH3A beta-glucosidase. Primer 69939 was designed to contain a 24 bp region encoding amino acids 582-589 of the *A. fumigatus* GH3A beta-glucosidase in addition to a 15 bp region encoding amino acids 585-589 of the *A. aculeatus* GH3A beta-glucosidase.

The primers shown below were designed to amplify the polynucleotide for the third polypeptide fragment from plasmid pAG114 encoding the *A. aculeatus* GH3A beta-glucosidase.

Forward primer (69940):

5'-GACGTGCTCTATGGCCGCGTCAACCCGGGCGCCAAATCT-3' (SEQ ID NO: 15)

Reverse primer (69941):

5'-GTCAGTCACCTCTAGTTAATTAATTATTGCACCTTCGGGAGCGCCGCGTG-3' (SEQ ID NO: 16)

Primer 69940 was designed to contain a 15 bp region encoding amino acids 585-589 of the *A. fumigatus* GH3A beta-glucosidase in addition to a 24 bp region corresponding to amino acids 589-596 of the *A. aculeatus* GH3A beta-glucosidase. Primer 69941 was designed to contain a 27 bp region corresponding to amino acids 852-860 of the *A. aculeatus* GH3A beta-glucosidase in addition to a 23 bp region which includes the *Pac* I restriction enzyme site and the 5' end of the AMG terminator from plasmid pAILo2.

| Chimeric section | Oligo pairs | DNA template |
|---|---|---|
| First fragment | 610232 + 69937 | pAG114 |

(continued)

| Chimeric section | Oligo pairs | DNA template |
|---|---|---|
| Second fragment | 69938 + 69939 | pDFng116 |
| Third fragment | 69940 + 69941 | pAG114 |

A total of 50 picomoles of each set of the primers above were used in an amplification reaction composed of 100 ng of pAG114 or pDFng116, 1X PHUSION® Buffer (New England Biolabs, Ipswich, MA, USA), 5 μl of a blend of dATP, dTTP, dGTP, and dCTP, each at 10 mM, and 1 unit of PHUSION® *Taq* DNA polymerase (New England Biolabs, Ipswich, MA, USA) in a final volume of 50 μl. The amplification reaction was performed in an EPPENDORF® MASTERCYCLER® 5333 (Eppendorf EG, Hamburg, Germany) programmed for 1 cycle at 98°C for 30 seconds; and 30 cycles each at 98°C for 10 seconds, 55°C for 30 seconds, and 72°C for 1 minute. After the 30 cycles, the reaction was heated for 7 minutes at 72°C. The heat block then went to a 10°C soak cycle.

The reaction products were isolated by 1.0% agarose gel electrophoresis using 40 mM Tris base, 20 mM sodium acetate, 1 mM disodium EDTA (TAE) buffer where a 1488 bp PCR product band for the first GH3A beta-glucosidase polypeptide fragment, a 645 bp PCR product band for the second GH3A beta-glucosidase polypeptide fragment, and a 907 bp PCR product band for the third GH3A beta-glucosidase polypeptide fragment were excised from the gels and extracted using a QIAQUICK® Gel Extraction Kit (QIAGEN Inc., Valencia, CA, USA).

Plasmid pAILo2 was gapped by digestion with *Pac* I and *Nco* I. The digestion was verified by fractionating an aliquot of the digestion by 0.8% agarose gel electrophoresis using TAE buffer where an expected fragment of 5617 bp (gapped) was obtained. The 5617 bp (gapped) fragment was excised from the gel and purified using a QIAQUICK® Gel Extraction Kit.

Multi-fragment PCR cloning was performed according to the method of Zhu *et al*., 2007, *supra.* The homologous ends of the 1488 bp PCR product for the first GH3A beta-glucosidase polypeptide fragment, the homologous ends of the 645 bp PCR product for the second GH3A beta-glucosidase polypeptide fragment, the homologous ends of the 907 bp PCR product for the third GH3A beta-glucosidase polypeptide fragment, and plasmid pAILo2, digested with *Pac* I and *Nco* I, were joined together using an IN-FUSION™ Advantage PCR Cloning Kit. A total of 25 ng of the 1488 bp PCR product, 25 ng of the 645 bp PCR product, 25 ng of the 907 bp PCR product, and 200 ng of plasmid pAILo2 (digested with *Pac* I and *Nco* I) were used in a reaction containing 2 μl of 5X IN-FUSION™ reaction buffer (Clontech Laboratories, Inc., Mountain View, CA, USA) and 1 μl of IN-FUSION™ enzyme (Clontech Laboratories, Inc., Mountain View, CA, USA) in a final volume of 10 μl. The reaction was incubated for 15 minutes at 37°C, followed by 15 minutes at 50°C, and then placed on ice. The reaction volume was increased to 50 μl with 10 mM Tris-0.1 mM EDTA pH 8 (TE) buffer and 3 μl of the reaction were used to transform *E. coli* XL10-GOLD® Ultracompetent Cells (Stratagene, La Jolla, CA, USA) according to the manufacturer's instructions. Transformants were selected on LB plates supplemented with 100 μg of ampicillin per ml. Plasmid DNA from several of the resulting *E. coli* transformants was prepared using a BIOROBOT® 9600 (QIAGEN Inc., Valencia, CA, USA).

One plasmid designated pTH247 comprising a polynucleotide encoding amino acids 1 to 404 of the *A. aculeatus* GH3A beta-glucosidase, amino acids 406 to 589 from *Aspergillus fumigatus* GH3A beta-glucosidase, and amino acids 589 to 860 of the *A. aculeatus* GH3A beta-glucosidase was identified and the full-length polynucleotide sequence was determined using a 3130xl Genetic Analyzer (Applied Biosystems, Foster City, CA, USA).

The DNA sequence and deduced amino acid sequence of the *Aspergillus fumigatus* GH3A beta-glucosidase and *Aspergillus aculeatus* GH3A beta-glucosidase chimeric polypeptide are shown in SEQ ID NO: 5 and SEQ ID NO: 6, respectively, which is referred to herein as "chimeric beta-glucosidase".

## Example 3: Construction of an *Aspergillus fumigatus* GH3A beta-glucosidase and *Aspergillus aculeatus* GH3A beta-glucosidase chimeric variant having beta-glucosidase activity

A variant of the *Aspergillus fumigatus* GH3A beta-glucosidase and *Aspergillus aculeatus* GH3A beta-glucosidase chimeric polypeptide ("chimeric beta-glucosidase") was constructed by performing site-directed mutagenesis on pTH247 using a QUIKCHANGE® Multi Site-Directed Mutagenesis Kit (Stratagene, La Jolla, CA, USA).

The resulting variant plasmid DNAs were prepared using a BIOROBOT® 9600. The variant plasmid constructs were sequenced using an Applied Biosystems 3130xl Genetic Analyzer to verify the changes.

A summary of the oligos used for the site-directed mutagenesis and the variant obtained are shown in Table 1.

TABLE 1

| Amino acid changes | Primer name | Sequences | Cloning Plasmid Name |
|---|---|---|---|
| N455E | 69885 | GGGTAGTGGTACTGCCGAGTTCCCTTACCTTGTCAC (SEQ ID NO: 17) | pTH265 |
| F511Y | 69886 | GCCGACTCTGGAGAGGGTTACATCAGTGTCGACGGCAAC (SEQ ID NO: 18) | |

The DNA sequence and deduced amino acid sequence of the variant of the chimeric beta-glucosidase are shown in SEQ ID NO: 7 and SEQ ID NO: 8, respectively, which is referred to herein as "chimeric beta-glucosidase variant".

**Example 4: Expression of the chimeric beta-glucosidase and chimeric beta-glucosidase variant**

*Aspergillus oryzae* JaL250 (WO 99/61651) protoplasts were prepared according to the method of Christensen et al., 1988, Bio/Technology 6: 1419-1422 and transformed with 5 μg of pTH247 ("chimeric beta-glucosidase") or pTH265 ("chimeric beta-glucosidase variant"). Each transformation yielded about 20-25 transformants. Ten transformants of pTH247 and twelve transformants of pTH265 were isolated to individual PDA plates. Once the cultures from each spore purified transformant were confluent and had sporulated, spore stocks were made by applying 5 ml of sterile filtered 0.01% TWEEN® 80 (diluted with glass distilled water) onto the center of each PDA plate and using a sterile spreader to scrape the spores into solution. Eight μl of each spore sock were inoculated into 1 ml of MDU2BP medium in 24 well culture plates and incubated at 34°C stationary for 5 days. Broth samples were harvested at day 5 and analyzed for beta-glucosidase activity by diluting the broth samples 10-fold and 100-fold into 50 mM sodium acetate, 0.01% TWEEN® 20 pH 5.0 buffer. Twenty microliters of each diluted sample were mixed with 100 μl of a solution of 1 mM *p*-nitrophenyl beta-D-glucopyranoside (N7006, Sigma-Aldrich, St. Louis, MO, USA) in 50 mM sodium acetate, 0.01% TWEEN® 20 pH 5.0 buffer in a clear, flat-bottom 96-well plate (Corning, Inc., Oneonta, NY, USA) and incubated at ambient temperature for 20 minutes. After the 20 minute incubation, 50 μl of 1 M sodium carbonate were added to each reaction mixture in order to stop the reaction. The resulting absorbance at 405 nm was measured using a SPECTRAMAX® 340pc spectrophotometric plate reader (Molecular Devices, Sunnyvale, CA, USA) to determine the relative amounts of beta-glucosidase activity in each well.

Spore stocks from the highest producing transformants identified by measuring beta-glucosidase activity were used to inoculate a 2 liter shake flask containing 500 ml of MDU2BP medium. Shake flasks were incubated for 5 days at 34°C with agitation at 220 rpm. After the incubation, the broths were sterile filtered using a 0.22 μm polyethersulfone membrane (Millipore, Bedford, MA, USA) for purification. One *A. oryzae* transformant expressing pTH247 and identified from the activity assay was designated *A. oryzae* O4VHP. Another *A. oryzae* transformant expressing pTH265 and identified from the activity assay was designated *A. oryzae* O6HPA.

**Example 5: Purification of the chimeric beta-glucosidase and chimeric beta-glucosidase variant**

*A. oryzae* O4VHP broth supernatant containing the chimeric beta-glucosidase, obtained according to Example 4, was concentrated and desalted using an ECONOPAC® 10 DG column equilibrated with 50 mM sodium acetate pH 5.0, containing 100 mM NaCl.

*A. oryzae* O6HPA broth supernatant containing the chimeric beta-glucosidase variant, obtained according to Example 4, was concentrated and desalted using an ECONOPAC® 10 DG column (equilibrated with 20 mM Tris-HCl pH 8.0. A 3 ml volume of broth supernatant was loaded onto the column and then eluted with a 4 ml volume of 20 mM Tris-HCl pH 8.0. The desalted sample was purified using a 20 ml MONO Q™ column (HR 16/10, GE Healthcare, Piscataway, NJ, USA) equilibrated with 20 mM Tris-HCl pH 8.0. A 16 ml volume of the desalted material was loaded onto the MONO Q™ column and then washed with 5 column volumes of 20 mM Tris-HCl pH 8.0. The chimeric beta-glucosidase variant was eluted using a 20 column volume gradient from 0.0 to 0.40 M NaCl in 20 mM Tris-HCl pH 8.0. Fractions of 6 ml were collected, and fractions 25 through 33 were pooled. Purified samples were submitted to SDS-PAGE analysis. SDS-PAGE on a 8-16% stain free Tris-HCl gel (Bio-Rad Laboratories, Inc., Hercules, CA, USA) with imaging using a CRITERION® Stain Free Imager (Bio-Rad Laboratories, Inc., Hercules, CA, USA) showed the protein to be greater than 95% pure.

**Example 6: Pretreatment of corn stover**

Corn stover was pretreated at the U.S. Department of Energy National Renewable Energy Laboratory (NREL) using 1.4% (w/v) sulfuric acid for 2 minutes at 190°C. The water-insoluble solids in the pretreated corn stover contained 57.5% cellulose, 4.6% hemicelluloses, and 28.4% lignin. Cellulose and hemicellulose were determined by a two-stage sulfuric acid hydrolysis with subsequent analysis of sugars by high performance liquid chromatography using NREL Standard Analytical Procedure #002. Lignin was determined gravimetrically after hydrolyzing the cellulose and hemicellulose fractions with sulfuric acid using NREL Standard Analytical Procedure #003. The pretreated corn stover was washed repeatedly using reverse osmosis water followed by decanting off the supernatant fraction, until the measured pH was greater than 4.0. The washed pretreated corn stover (initial dry weight 32.35% TS) was subsequently milled prior to use in a Cosmos ICMG 40 wet multi-utility grinder (EssEmm Corporation, Tamil Nadu, India). The dry weight of the milled, water-washed pretreated corn stover was 7.4% TS.

**Example 7: Determination of the specific activity of the chimeric beta-glucosidase and chimeric beta-glucosidase variant**

The purified chimeric beta-glucosidase and the purified chimeric beta-glucosidase variant obtained according to Example 5 were evaluated for their ability to enhance the hydrolysis of PCS by a *Trichoderma reesei* cellulolytic protein preparation composed of CELLUCLAST® 1.5L (Novozymes A/S, Bagsvaerd, Denmark) supplemented with 10% addition of *Thermoascus aurantiacus* GH61A polypeptide having cellulolytic enhancing activity (obtained as described in WO 2005/074656) and 2% addition of SHEARZYME® 2X (Novozymes A/S, Bagsvaerd, Denmark), hereinafter designated "*Trichoderma reesei* cellulolytic protein composition". Purified wild-type *Aspergillus fumigatus* GH3A beta-glucosidase and wild-type *Aspergillus aculeatus* GH3A beta-glucosidase (Example 1) were run as controls.

The hydrolysis of PCS was conducted using 2.2 ml deep-well plates (Axygen, Union City, CA, USA) in a total reaction volume of 1.0 ml. The hydrolysis was performed with 50 mg of washed, ground, sieved PCS (Example 4) per ml of 50 mM sodium acetate pH 5.0 buffer containing 1 mM manganese sulfate, 40 g of glucose per ml, and a fixed protein loading of 2.24 mg of the *T. reesei* cellulolytic protein composition per gram of cellulose and between 0 to 10% addition (by protein) to the base CELLUCLAST 1.5L enzyme loading with the purified *Aspergillus fumigatus* Family 3A beta-glucosidase (2.24 mg of the *T. reesei* cellulolytic protein composition per g of cellulose and between 0 and 0.2 mg of *Aspergillus fumigatus* Family 3A beta-glucosidase or variant thereof per g of cellulose). Hydrolysis assays were performed in triplicate for 72 hours at 50°C. Following hydrolysis, samples were filtered with a 0.45 μm MULTISCREEN® 96-well filter plate (Millipore, Bedford, MA, USA) and filtrates analyzed for sugar content as described below. When not used immediately, filtered sugary aliquots were frozen at -20°C.

Sugar concentrations of samples diluted in 0.005 M $H_2SO_4$ were measured after elution from a 4.6 x 250 mm AMINEX® HPX-87H column (Bio-Rad Laboratories, Inc., Hercules, CA, USA) by 0.005 M $H_2SO_4$ with 0.05% w/w benzoic acid at a flow rate of 0.6 ml per minute at 65°C with quantitation by integration of glucose and cellobiose signals from refractive index detection (CHEMSTATION®, AGILENT® 1100 HPLC, Agilent Technologies, Santa Clara, CA, USA) calibrated by pure sugar samples. The resultant equivalents were used to calculate the percentage of cellulose conversion for each reaction. Glucose released was calculated by subtracting glucose levels measured in the PCS alone from those measured in PCS with enzyme mixture.

The results demonstrated that the chimeric beta-glucosidase (Figure 5) and the chimeric beta-glucosidase variant (Figure 6) have a higher specific activity than the wild-type *Aspergillus fumigatus* GH3A beta-glucosidase and the wild-type *Aspergillus aculeatus* GH3A beta-glucosidase (releases more glucose at a given enzyme concentration than the *Aspergillus fumigatus* GH3A beta-glucosidase and the *Aspergillus aculeatus* GH3A beta-glucosidase at the same concentration under the specified conditions).

The present disclosure is further described by the following numbered paragraphs:

[1] An isolated chimeric polypeptide having beta-glucosidase activity, comprising: (a) a first polypeptide fragment at the N-terminal end of the chimeric polypeptide selected from the group consisting of (i) a polypeptide fragment having at least 60% identity to amino acids 20 to 404 of SEQ ID NO: 2; (ii) a polypeptide fragment encoded by a polynucleotide that hybridizes under at least low stringency conditions with nucleotides 131 to 1455 of SEQ ID NO: 1 or the cDNA sequence thereof, or the full-length complement thereof; (iii) a polypeptide fragment encoded by a polynucleotide having at least 60% identity to nucleotides 131 to 1455 of SEQ ID NO: 1 or the cDNA sequence thereof; and (iv) a polypeptide fragment comprising or consisting of amino acids 20 to 404 of SEQ ID NO: 2; (b) a second polypeptide fragment at the C-terminal end of the first polypeptide fragment selected from the group consisting of (i) a polypeptide fragment having at least 60% identity to amino acids 406 to 589 of SEQ ID NO: 4; (ii) a polypeptide fragment encoded by a polynucleotide that hybridizes under at least low stringency conditions with nucleotides 1570 to 2184 of SEQ ID NO: 3 or the cDNA sequence thereof, or the full-length complement thereof; (iii) a polypeptide fragment encoded by a polynucleotide having at least 60% identity to nucleotides 1570 to 2184 of SEQ ID NO: 3 or the cDNA sequence thereof; and (iv) a polypeptide fragment comprising or consisting of amino acids 406 to 589 of SEQ ID NO: 4; and (c) a third polypeptide fragment at the C-terminal end of the second polypeptide fragment selected from the group consisting of (i) a polypeptide fragment having at least 60% identity to amino acids 589 to 860 of SEQ ID NO: 2; (ii) a polypeptide fragment encoded by a polynucleotide that hybridizes under at least low stringency conditions with nucleotides 2072 to 2937 of SEQ ID NO: 1 or the cDNA sequence thereof, or the full-length complement thereof; (iii) a polypeptide fragment encoded by a polynucleotide having at least 60% identity to nucleotides 2072 to 2937 of SEQ ID NO: 1 or the cDNA sequence thereof; and (iv) a polypeptide fragment comprising or consisting of amino acids 589 to 860 of SEQ ID NO: 2.

[2] The chimeric polypeptide of paragraph 1, wherein the first polypeptide fragment has at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 95%, at least 96%, at least 97%, at least 98%, at least 99%, or 100% sequence identity to amino acids 20 to 404 of SEQ ID NO: 2.

[3] The chimeric polypeptide of paragraph 1, wherein the first polypeptide fragment is encoded by a polynucleotide that hybridizes under at least low stringency conditions, at least medium stringency conditions, at least medium-high stringency conditions, at least high stringency conditions, or at least very high stringency conditions with nucleotides 131 to 1455 of SEQ ID NO: 1 or the cDNA sequence thereof, or the full-length complement thereof.

[4] The chimeric polypeptide of paragraph 1, wherein the first polypeptide fragment is encoded by a polynucleotide having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 95%, at least 96%, at least 97%, at least 98%, at least 99%, or 100% sequence identity to nucleotides 131 to 1455 of SEQ ID NO: 1 or the cDNA sequence thereof.

[5] The chimeric polypeptide of paragraph 1, wherein the first polypeptide fragment comprises or consists of amino acids 20 to 404 of SEQ ID NO: 2.

[6] The chimeric polypeptide of paragraph 1, wherein the first polypeptide fragment is encoded by nucleotides 131 to 1455 of SEQ ID NO: 1 or the cDNA sequence thereof.

[7] The chimeric polypeptide of paragraph 1, wherein the second polypeptide fragment has at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 95%, at least 96%, at least 97%, at least 98%, at least 99%, or 100% sequence identity to amino acids 406 to 589 of SEQ ID NO: 4.

[8] The chimeric polypeptide of paragraph 1, wherein the second polypeptide fragment is encoded by a polynucleotide that hybridizes under at least low stringency conditions, at least medium stringency conditions, at least medium-high stringency conditions, at least high stringency conditions, or at least very high stringency conditions with nucleotides 1570 to 2184 of SEQ ID NO: 3 or the cDNA sequence thereof, or the full-length complement thereof.

[9] The chimeric polypeptide of paragraph 1, wherein the second polypeptide fragment is encoded by a polynucleotide having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 95%, at least 96%, at least 97%, at least 98%, at least 99%, or 100% sequence identity to nucleotides 1570 to 2184 of SEQ ID NO: 3 or the cDNA sequence thereof.

[10] The chimeric polypeptide of paragraph 1, wherein the second polypeptide fragment comprises or consists of amino acids 406 to 589 of SEQ ID NO: 4.

[11] The chimeric polypeptide of paragraph 1, wherein the second polypeptide fragment is encoded by nucleotides 1570 to 2184 of SEQ ID NO: 3 or the cDNA sequence thereof.

[12] The chimeric polypeptide of paragraph 1, wherein the third polypeptide fragment has at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 95%, at least 96%, at least 97%, at least 98%, at least 99%, or 100% sequence identity to amino acids 589 to 860 of SEQ ID NO: 2.

[13] The chimeric polypeptide of paragraph 1, wherein the third polypeptide fragment is encoded by a polynucleotide that hybridizes under at least low stringency conditions, at least medium stringency conditions, at least medium-high stringency conditions, at least high stringency conditions, or at least very high stringency conditions with nucleotides 2072 to 2937 of SEQ ID NO: 1 or the cDNA sequence thereof, or the full-length complement thereof.

[14] The chimeric polypeptide of paragraph 1, wherein the third polypeptide fragment is encoded by a polynucleotide having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 95%, at least 96%, at least 97%, at least 98%, at least 99%, or 100% sequence identity to nucleotides 2072 to 2937 of SEQ ID NO: 1 or the cDNA sequence thereof.

[15] The chimeric polypeptide of paragraph 1, wherein the third polypeptide fragment comprises or consists of amino acids 589 to 860 of SEQ ID NO: 2.

[16] The chimeric polypeptide of paragraph 1, wherein the third polypeptide fragment is encoded by nucleotides 2072 to 2937 of SEQ ID NO: 1 or the cDNA sequence thereof.

[17] The chimeric polypeptide of paragraph 1, which comprises or consists of amino acids 20 to 404 of SEQ ID NO: 2 as the first polypeptide fragment at the N-terminal end of the chimeric polypeptide, amino acids 406 to 589 of SEQ ID NO: 4 as the second polypeptide fragment at the C-terminal end of the first polypeptide fragment, and amino acids 589 to 860 of SEQ ID NO: 2 as the third polypeptide fragment at the C-terminal end of the second polypeptide fragment.

[18] The chimeric polypeptide of paragraph 1, which comprises or consists of the mature polypeptide of SEQ ID NO: 6.

[19] The chimeric polypeptide of any of paragraphs 1-18, further comprising a substitution at one or both positions corresponding to positions 455 and 511 of the mature polypeptide of SEQ ID NO: 6, wherein the variant has beta-glucosidase activity.

[20] The chimeric polypeptide of paragraph 19, wherein the parent beta-glucosidase is (a) a polypeptide having at least 60% sequence identity to the mature polypeptide of SEQ ID NO: 6; (b) a polypeptide encoded by a polynucleotide that hybridizes under at least low stringency conditions with (i) the mature polypeptide coding sequence of SEQ ID NO: 5, (ii) the cDNA sequence contained in the mature polypeptide coding sequence of SEQ ID NO: 5, or (iii) the full-length complement of (i) or (ii); (c) a polypeptide encoded by a polynucleotide having at least 60% identity to the mature polypeptide coding sequence of SEQ ID NO: 5 or the cDNA sequence thereof; or (d) a fragment of the mature polypeptide of SEQ ID NO: 6, which has beta-glucosidase activity.

[21] The chimeric polypeptide of paragraph 20, wherein the parent beta-glucosidase has at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% sequence identity to the mature polypeptide of SEQ ID NO: 6.

[22] The chimeric polypeptide of paragraph 20, wherein the parent beta-glucosidase is encoded by a polynucleotide that hybridizes under at least low stringency conditions, at least medium stringency conditions, at least medium-high stringency conditions, at least high stringency conditions, or at least very high stringency conditions with (i) the mature polypeptide coding sequence of SEQ ID NO: 5 or the cDNA sequence thereof or (ii) the full-length complement of (i).

[23] The chimeric polypeptide of paragraph 20, wherein the parent beta-glucosidase is encoded by a polynucleotide having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% sequence identity to the mature polypeptide coding sequence of SEQ ID NO: 5, or the cDNA sequence thereof.

[24] The chimeric polypeptide of paragraph 20, wherein the parent beta-glucosidase comprises or consists of the mature polypeptide of SEQ ID NO: 6.

[25] The chimeric polypeptide of paragraph 20, wherein the parent beta-glucosidase is a fragment of the mature polypeptide of SEQ ID NO: 6, wherein the fragment has beta-glucosidase activity.

[26] The chimeric polypeptide of paragraph 20, which has at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, but less than 100%, sequence identity to the amino acid sequence of the parent beta-glucosidase.

[27] The chimeric polypeptide of any of paragraphs 19-26, which has at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, but less than 100%, sequence identity to the mature polypeptide of SEQ ID NO: 6.

[28] The chimeric polypeptide of any of paragraphs 19-27, wherein the variant consists of 720 to 857 amino acids, e.g., 720 to 739, 740 to 759, 760 to 779, 780 to 799, 800 to 819, and 820 to 841 amino acids.

[29] The chimeric polypeptide of any of paragraphs 19-28, wherein the number of substitutions is 1 or 2 substitutions.

[30] The chimeric polypeptide of any of paragraphs 19-29, which comprises a substitution at a position corresponding to position 455.

[31] The chimeric polypeptide of paragraph 30, wherein the substitution is Glu.

[32] The chimeric polypeptide of any of paragraphs 19-31, which comprises a substitution at a position corresponding to position 511.

[33] The chimeric polypeptide of paragraph 32, wherein the substitution is Tyr

[34] The chimeric polypeptide of any of paragraphs 19-33, which comprises a substitution at each position corresponding to positions 455 and 511.

[35] The chimeric polypeptide of any of paragraphs 19-34, which comprises one or both of the substitutions N455E and F511Y.

[36] The chimeric polypeptide of any of paragraphs 19-35, which comprises the substitutions N455E and F511Y.

[37] The chimeric polypeptide of any of paragraphs 1-36, which further comprises a signal peptide at the N-terminal end of the first polypeptide fragment.

[38] The chimeric polypeptide of paragraph 37, wherein the signal peptide is the signal peptide of SEQ ID NO: 2 or SEQ ID NO: 4.

[39] The chimeric polypeptide of paragraph 37, wherein the signal peptide is amino acids 1 to 19 of SEQ ID NO: 2 or amino acids 1 to 19 of SEQ ID NO: 4.

[40] An isolated polynucleotide encoding the chimeric polypeptide of any of paragraphs 1-39.

[41] A nucleic acid construct comprising the polynucleotide of paragraph 40.

[42] An expression vector comprising the polynucleotide of paragraph 40.

[43] A host cell comprising the polynucleotide of paragraph 40.

[44] A method of producing a chimeric polypeptide having beta-glucosidase activity, comprising: cultivating the host cell of paragraph 43 under conditions suitable for the expression of the chimeric polypeptide.

[45] The method of paragraph 44, further comprising recovering the chimeric polypeptide.

[46] A transgenic plant, plant part or plant cell transformed with the polynucleotide of paragraph 40.

[47] A method of producing the chimeric polypeptide of any of paragraphs 1-39, comprising: (a) cultivating a transgenic plant or a plant cell comprising a polynucleotide encoding the chimeric polypeptide under conditions conducive for production of the chimeric polypeptide.

[48] The method of paragraph 47, further comprising recovering the chimeric polypeptide.

[49] A method for degrading or converting a cellulosic material, comprising: treating the cellulosic material with an enzyme composition in the presence of the chimeric polypeptide of any of paragraphs 1-39.

[50] The method of paragraph 49, wherein the cellulosic material is pretreated.

[51] The method of paragraph 49 or 50, further comprising recovering the degraded cellulosic material.

[52] The method of any of paragraphs 49-51, wherein the enzyme composition comprises one or more (e.g., several) enzymes selected from the group consisting of a cellulase, a polypeptide having cellulolytic enhancing activity, a hemicellulase, an esterase, an expansin, a laccase, a ligninolytic enzyme, a pectinase, a peroxidase, a protease, and a swollenin.

[53] The method of paragraph 52, wherein the cellulase is one or more (e.g., several) enzymes selected from the group consisting of an endoglucanase, a cellobiohydrolase, and a beta-glucosidase.

[54] The method of paragraph 52, wherein the hemicellulase is one or more (e.g., several) enzymes selected from the group consisting of a xylanase, an acetyxylan esterase, a feruloyl esterase, an arabinofuranosidase, a xylosidase, and a glucuronidase.

[55] The method of any of paragraphs 49-54, wherein the degraded cellulosic material is a sugar.

[56] The method of paragraph 55, wherein the sugar is selected from the group consisting of glucose, xylose, mannose, galactose, and arabinose.

[57] A method for producing a fermentation product, comprising: (a) saccharifying a cellulosic material with an enzyme composition in the presence of the chimeric polypeptide of any of paragraphs 1-39; (b) fermenting the saccharified cellulosic material with one or more (e.g., several) fermenting microorganisms to produce the fermentation product; and (c) recovering the fermentation product from the fermentation.

[58] The method of paragraph 57, wherein the cellulosic material is pretreated.

[59] The method of paragraph 57 or 58, wherein the enzyme composition comprises one or more (e.g., several) enzymes selected from the group consisting of a cellulase, a polypeptide having cellulolytic enhancing activity, a hemicellulase, an esterase, an expansin, a laccase, a ligninolytic enzyme, a pectinase, a peroxidase, a protease, and a swollenin.

[60] The method of paragraph 59, wherein the cellulase is one or more (e.g., several) enzymes selected from the group consisting of an endoglucanase, a cellobiohydrolase, and a beta-glucosidase.

[61] The method of paragraph 59, wherein the hemicellulase is one or more (e.g., several) enzymes selected from the group consisting of a xylanase, an acetyxylan esterase, a feruloyl esterase, an arabinofuranosidase, a xylosidase, and a glucuronidase.

[62] The method of any of paragraphs 57-61, wherein steps (a) and (b) are performed simultaneously in a simultaneous saccharification and fermentation.

[63] The method of any of paragraphs 57-62, wherein the fermentation product is an alcohol, an organic acid, a ketone, an amino acid, or a gas.

[64] A method of fermenting a cellulosic material, comprising: fermenting the cellulosic material with one or more (e.g., several) fermenting microorganisms, wherein the cellulosic material is saccharified with an enzyme composition in the presence of the chimeric polypeptide of any of paragraphs 1-39.

[65] The method of paragraph 64, wherein the cellulosic material is pretreated before saccharification.
[66] The method of paragraph 64 or 65, wherein the enzyme composition comprises one or more (e.g., several) enzymes selected from the group consisting of a cellulase, a polypeptide having cellulolytic enhancing activity, a hemicellulase, an esterase, an expansin, a laccase, a ligninolytic enzyme, a pectinase, a peroxidase, a protease, and a swollenin.
[67] The method of paragraph 66, wherein the cellulase is one or more (e.g., several) enzymes selected from the group consisting of an endoglucanase, a cellobiohydrolase, and a beta-glucosidase.
[68] The method of paragraph 66, wherein the hemicellulase is one or more (e.g., several) enzymes selected from the group consisting of a xylanase, an acetyxylan esterase, a feruloyl esterase, an arabinofuranosidase, a xylosidase, and a glucuronidase.
[69] The method of any of paragraphs 64-68, wherein the fermenting of the cellulosic material produces a fermentation product.
[70] The method of paragraph 69, further comprising recovering the fermentation product from the fermentation.
[71] The method of any of paragraphs 69 or 70, wherein the fermentation product is an alcohol, an organic acid, a ketone, an amino acid, or a gas.
[72] A whole broth formulation or cell culture composition comprising the chimeric polypeptide of any of paragraphs 1-39.

## Claims

1. An isolated chimeric polypeptide having beta-glucosidase activity, comprising:

   (a) a first polypeptide fragment at the N-terminal end of the chimeric polypeptide selected from the group consisting of (i) a polypeptide fragment having at least 90% identity to amino acids 20 to 404 of SEQ ID NO: 2; and (ii) a polypeptide fragment comprising or consisting of amino acids 20 to 404 of SEQ ID NO: 2;
   (b) a second polypeptide fragment at the C-terminal end of the first polypeptide fragment selected from the group consisting of (i) a polypeptide fragment having at least 90% identity to amino acids 406 to 589 of SEQ ID NO: 4; and (ii) a polypeptide fragment comprising or consisting of amino acids 406 to 589 of SEQ ID NO: 4; and
   (c) a third polypeptide fragment at the C-terminal end of the second polypeptide fragment selected from the group consisting of (i) a polypeptide fragment having at least 90% identity to amino acids 589 to 860 of SEQ ID NO: 2; and (ii) a polypeptide fragment comprising or consisting of amino acids 589 to 860 of SEQ ID NO: 2,

   wherein said chimeric polypeptide has increased specific activity compared to both the mature polypeptide of SEQ ID NO: 2 and the mature polypeptide of SEQ ID NO: 4.

2. The chimeric polypeptide of claim 1, which comprises or consists of amino acids 20 to 404 of SEQ ID NO: 2 as the first polypeptide fragment at the N-terminal end of the chimeric polypeptide, amino acids 406 to 589 of SEQ ID NO: 4 as the second polypeptide fragment at the C-terminal end of the first polypeptide fragment, and amino acids 589 to 860 of SEQ ID NO: 2 as the third polypeptide fragment at the C-terminal end of the second polypeptide fragment.

3. The chimeric polypeptide of claim 1, which comprises or consists of the mature polypeptide of SEQ ID NO: 6.

4. The chimeric polypeptide of claim 1, which comprises or consists of amino acids 20 to 860 of SEQ ID NO: 6.

5. The chimeric polypeptide of any of claims 1-4, further comprising a substitution at one or both positions corresponding to positions 455 and 511 of the mature polypeptide of SEQ ID NO: 6, wherein the variant has beta-glucosidase activity.

6. The chimeric polypeptide of claim 5, wherein the parent beta-glucosidase is

   (a) a polypeptide having at least 97% sequence identity to the mature polypeptide of SEQ ID NO: 6 and having has beta-glucosidase activity;

(b) a polypeptide comprising or consisting of the mature polypeptide of SEQ ID NO: 6; or
(c) a fragment of the mature polypeptide of SEQ ID NO: 6, which has beta-glucosidase activity.

**7.** The chimeric polypeptide of claim 5 or 6, which comprises one or both of the substitutions N455E and F511Y.

**8.** The chimeric polypeptide of any of claims 1-7, which further comprises a signal peptide at the N-terminal end of the first polypeptide fragment.

**9.** The chimeric polypeptide of claim 8, wherein the signal peptide is the signal peptide of SEQ ID NO: 2 or SEQ ID NO: 4.

**10.** An isolated polynucleotide encoding the chimeric polypeptide of any of claims 1-9.

**11.** A method of producing a chimeric polypeptide having beta-glucosidase activity, comprising:

(a) cultivating a host cell comprising the polynucleotide of claim 10 under conditions suitable for the expression of the chimeric polypeptide; and optionally
(b) recovering the chimeric polypeptide.

**12.** A method for degrading or converting a cellulosic material, comprising treating the cellulosic material with an enzyme composition in the presence of the chimeric polypeptide of any of claims 1-9.

**13.** The method of claim 12, further comprising recovering the degraded cellulosic material.

**14.** A method for producing a fermentation product, comprising:

(a) saccharifying a cellulosic material with an enzyme composition in the presence of the chimeric polypeptide of any of claims 1-9;
(b) fermenting the saccharified cellulosic material with one or more (e.g., several) fermenting microorganisms to produce the fermentation product; and
(c) recovering the fermentation product from the fermentation.

**15.** A method of fermenting a cellulosic material, comprising fermenting the cellulosic material with one or more (*e.g.*, several) fermenting microorganisms, wherein the cellulosic material is saccharified with an enzyme composition in the presence of the chimeric polypeptide of any of claims 1-9.

**16.** The method of claim 15, wherein the fermenting of the cellulosic material produces a fermentation product.

**17.** The method of claim 16, further comprising recovering the fermentation product from the fermentation.

**18.** A whole broth formulation or cell culture composition comprising the chimeric polypeptide of any of claims 1-9.

## Patentansprüche

**1.** Isoliertes chimäres Polypeptid mit beta-Glucosidaseaktivität, umfassend:

(a) ein erstes Polypeptidfragment am N-terminalen Ende des chimären Polypeptids, ausgewählt aus der Gruppe bestehend aus (i) einem Polypeptidfragment mit mindestens 90% Identität zu Aminosäuren 20 bis 404 von SEQ ID NO: 2; und (ii) einem Polypeptidfragment, das Aminosäuren 20 bis 404 von SEQ ID NO: 2 umfasst oder daraus besteht;
(b) ein zweites Polypeptidfragment am C-terminalen Ende des ersten Polypeptidfragments, ausgewählt aus der Gruppe bestehend aus (i) einem Polypeptidfragment mit mindestens 90% Identität zu Aminosäuren 406 bis 589 von SEQ ID NO: 4; und (ii) einem Polypeptidfragment, das Aminosäuren 406 bis 589 von SEQ ID NO: 4 umfasst oder daraus besteht; und
(c) ein drittes Polypeptidfragment am C-terminalen Ende des zweiten Polypeptidfragments, ausgewählt aus der Gruppe bestehend aus (i) einem Polypeptidfragment mit mindestens 90% Identität zu Aminosäuren 589 bis 860 von SEQ ID NO: 2; und (ii) einem Polypeptidfragment, das Aminosäuren 589 bis 860 von SEQ ID NO: 2 umfasst oder daraus besteht,

wobei das chimäre Polypeptid erhöhte spezifische Aktivität verglichen zu sowohl dem reifen Polypeptid von SEQ ID NO: 2 als auch dem reifen Polypeptid von SEQ ID NO: 4 aufweist.

2. Chimäres Polypeptid nach Anspruch 1, das Aminosäuren 20 bis 404 von SEQ ID NO: 2 als das erste Polypeptidfragment am N-terminalen Ende des chimären Polypeptids, Aminosäuren 406 bis 589 von SEQ ID NO: 4 als das zweite Polypeptidfragment am C-terminalen Ende des ersten Polypeptidfragments, und Aminosäuren 589 bis 860 von SEQ ID NO: 2 als das dritte Polypeptidfragment am C-terminalen Ende des zweiten Polypeptidfragments umfasst oder daraus besteht.

3. Chimäres Polypeptid nach Anspruch 1, das das reife Polypeptid von SEQ ID NO: 6 umfasst oder daraus besteht.

4. Chimäres Polypeptid nach Anspruch 1, das Aminosäuren 20 bis 860 von SEQ ID NO: 6 umfasst oder daraus besteht.

5. Chimäres Polypeptid nach einem beliebigen der Ansprüche 1-4, weiterhin umfassend eine Substitution an einer oder beiden Positionen entsprechend Positionen 455 und 511 des reifen Polypeptids von SEQ ID NO: 6, wobei die Variante beta-Glucosidaseaktivität aufweist.

6. Chimäres Polypeptid nach Anspruch 5, wobei die parentale beta-Glucosidase

(a) ein Polypeptid mit mindestens 97% Sequenzidentität zum reifen Polypeptid von SEQ ID NO: 6 und mit beta-Glucosidaseaktivität;
(b) ein Polypeptid, das das reife Polypeptid von SEQ ID NO: 6 umfasst oder daraus besteht; oder
(c) ein Fragment des reifen Polypeptids von SEQ ID NO: 6, das beta-Glucosidaseaktivität aufweist, ist.

7. Chimäres Polypeptid nach Anspruch 5 oder 6, das eine oder beide der Substitutionen N455E und F511Y umfasst.

8. Chimäres Polypeptid nach einem beliebigen der Ansprüche 1-7, das weiterhin ein Signalpeptid am N-terminalen Ende des ersten Polypeptidfragments umfasst.

9. Chimäres Polypeptid nach Anspruch 8, wobei das Signalpeptid das Signalpeptid von SEQ ID NO: 2 oder SEQ ID NO: 4 ist.

10. Isoliertes Polynukleotid, das das chimäre Polypeptid gemäß einem beliebigen der Ansprüche 1-9 kodiert.

11. Verfahren zum Herstellen eines chimären Polypeptids mit beta-Glucosidaseaktivität, umfassend:

(a) Kultivieren einer Wirtszelle, die das Polynukleotid gemäß Anspruch 10 umfasst, unter Bedingungen, die für die Expression des chimären Polypeptids geeignet sind; und gegebenenfalls
(b) Gewinnen des chimären Polypeptids.

12. Verfahren zum Abbauen oder Umwandeln eines cellulosischen Materials, umfassend Behandeln des cellulosischen Materials mit einer Enzymzusammensetzung in der Gegenwart des chimären Polypeptids gemäß einem beliebigen der Ansprüche 1-9.

13. Verfahren nach Anspruch 12, weiterhin umfassend Gewinnen des abgebauten cellulosischen Materials.

14. Verfahren zum Herstellen eines Fermentationsprodukts, umfassend:

(a) Verzuckern eines cellulosischen Materials mit einer Enzymzusammensetzung in der Gegenwart des chimären Polypeptids gemäß einem beliebigen der Ansprüche 1-9;
(b) Fermentieren des verzuckerten cellulosischen Materials mit einem oder mehreren (z.B. einigen) fermentierenden Mikroorganismen, um das Fermentationsprodukt herzustellen; und
(c) Gewinnen des Fermentationsprodukts aus der Fermentation.

15. Verfahren zum Fermentieren eines cellulosischen Materials, umfassend Fermentieren des cellulosischen Materials mit einem oder mehreren (z.B. einigen) fermentierenden Mikroorganismen, wobei das cellulosische Material mit einer Enzymzusammensetzung in der Gegenwart des chimären Polypeptids gemäß einem beliebigen der Ansprüche 1-9 verzuckert wird.

**16.** Verfahren nach Anspruch 15, wobei das Fermentieren des cellulosischen Materials ein Fermentationsprodukt herstellt.

**17.** Verfahren nach Anspruch 16, weiterhin umfassend Gewinnen des Fermentationsprodukts aus der Fermentation.

**18.** Gesamtbrühenformulierung oder Zellkulturzusammensetzung, umfassend das chimäre Polypeptid gemäß einem beliebigen der Ansprüche 1-9.

**Revendications**

**1.** Polypeptide chimérique isolé doté d'une activité bêta-glucosidase, comprenant :

(a) un premier fragment polypeptidique à l'extrémité N-terminale du polypeptide chimérique choisi dans le groupe constitué de (i) un fragment polypeptidique présentant au moins 90 % d'identité avec les acides aminés 20 à 404 de SEQ ID NO : 2 ; et (ii) un fragment polypeptidique comprenant ou constitué des acides aminés 20 à 404 de SEQ ID NO : 2 ;
(b) un deuxième fragment polypeptidique à l'extrémité C-terminale du premier fragment polypeptidique choisi dans le groupe constitué de (i) un fragment polypeptidique présentant au moins 90 % d'identité avec les acides aminés 406 à 589 de SEQ ID NO : 4 ; et (ii) un fragment polypeptidique comprenant ou constitué des acides aminés 406 à 589 de SEQ ID NO : 4 ; et
(c) un troisième fragment polypeptidique à l'extrémité C-terminale du deuxième fragment polypeptidique choisi dans le groupe constitué de (i) un fragment polypeptidique présentant au moins 90 % d'identité avec les acides aminés 589 à 860 de SEQ ID NO : 2 ; et (ii) un fragment polypeptidique comprenant ou constitué des acides aminés 589 à 860 de SEQ ID NO : 2,

où ledit polypeptide chimérique présente une activité spécifique accrue comparativement à la fois au polypeptide mature de SEQ ID NO : 2 et au polypeptide mature de SEQ ID NO : 4.

**2.** Polypeptide chimérique selon la revendication 1, qui comprend ou est constitué des acides aminés 20 à 404 de SEQ ID NO : 2 en tant que premier fragment polypeptidique à l'extrémité N-terminale du polypeptide chimérique, des acides aminés 406 à 589 de SEQ ID NO : 4 en tant que deuxième fragment polypeptidique à l'extrémité C-terminale du premier fragment polypeptidique, et des acides aminés 589 à 860 de SEQ ID NO : 2 en tant que troisième fragment polypeptidique à l'extrémité C-terminale du deuxième fragment polypeptidique.

**3.** Polypeptide chimérique selon la revendication 1, qui comprend ou est constitué du polypeptide mature de SEQ ID NO : 6.

**4.** Polypeptide chimérique selon la revendication 1, qui comprend ou est constitué des acides aminés 20 à 860 de SEQ ID NO : 6.

**5.** Polypeptide chimérique selon l'une quelconque des revendications 1 à 4, comprenant en outre une substitution au niveau de l'une ou des deux positions correspondant aux positions 455 et 511 du polypeptide mature de SEQ ID NO : 6, où le variant est doté d'une activité bêta-glucosidase.

**6.** Polypeptide chimérique selon la revendication 5, où la bêta-glucosidase parente est

(a) un polypeptide présentant au moins 97 % d'identité de séquence avec le polypeptide mature de SEQ ID NO : 6 et étant doté d'une activité bêta-glucosidase ;
(b) un polypeptide comprenant ou constitué du polypeptide mature de SEQ ID NO : 6 ; ou
(c) un fragment du polypeptide mature de SEQ ID NO : 6, qui est doté d'une activité bêta-glucosidase.

**7.** Polypeptide chimérique selon la revendication 5 ou 6, qui comprend l'une ou les deux des substitutions N455E et F511Y.

**8.** Polypeptide chimérique selon l'une quelconque des revendications 1 à 7, qui comprend en outre un peptide signal à l'extrémité N-terminale du premier fragment polypeptidique.

9. Polypeptide chimérique selon la revendication 8, dans lequel le peptide signal est le peptide signal de SEQ ID NO : 2 ou SEQ ID NO : 4.

10. Polynucléotide isolé codant pour le polypeptide chimérique selon l'une quelconque des revendications 1 à 9.

11. Méthode de production d'un polypeptide chimérique doté d'une activité bêta-glucosidase, comprenant :

(a) la culture d'une cellule hôte comprenant le polynucléotide selon la revendication 10 dans des conditions appropriées pour l'expression du polypeptide chimérique ; et éventuellement
(b) la récupération du polypeptide chimérique.

12. Méthode de dégradation ou de conversion d'un matériau cellulosique, comprenant le traitement du matériau cellulosique avec une composition enzymatique en présence du polypeptide chimérique selon l'une quelconque des revendications 1 à 9.

13. Méthode selon la revendication 12, comprenant en outre la récupération du matériau cellulosique dégradé.

14. Méthode de production d'un produit de fermentation, comprenant :

(a) la saccharification d'un matériau cellulosique avec une composition enzymatique en présence du polypeptide chimérique selon l'une quelconque des revendications 1 à 9 ;
(b) la fermentation du matériau cellulosique saccharifié avec un ou plusieurs micro-organismes de fermentation pour produire le produit de fermentation ; et
(c) la récupération du produit de fermentation à partir de la fermentation.

15. Méthode de fermentation d'un matériau cellulosique, comprenant la fermentation du matériau cellulosique avec un ou plusieurs micro-organismes de fermentation, dans laquelle le matériau cellulosique est saccharifié avec une composition enzymatique en présence du polypeptide chimérique selon l'une quelconque des revendications 1 à 9.

16. Méthode selon la revendication 15, dans laquelle la fermentation du matériau cellulosique produit un produit de fermentation.

17. Méthode selon la revendication 16, comprenant en outre la récupération du produit de fermentation à partir de la fermentation.

18. Formulation de bouillon complet ou composition de culture cellulaire comprenant le polypeptide chimérique selon l'une quelconque des revendications 1 à 9.

```
atgaagctcagttggcttgaggcggctgccttgacggctgcttcagtcgtcagcgctgtatgttggctt 69
 M  K  L  S  W  L  E  A  A  A  L  T  A  A  S  V  V  S  A
tttttgacctcctcgctgcttctagatatttggtgtgaggctgacaattcgtgctctacaggatgaact 138
                                                             D  E  L
ggcgttctctcctcctttctacccctctccgtgggccaatggccagggagagtgggcggaagcctacca 207
  A  F  S  P  P  F  Y  P  S  P  W  A  N  G  Q  G  E  W  A  E  A  Y  Q
gcgtgcagtggccattgtatcccagatgactctggatgagaaggtcaacctgaccaccggaactgggta 276
  R  A  V  A  I  V  S  Q  M  T  L  D  E  K  V  N  L  T  T  G  T  G
atgacactacagctgctgcgagatgaatcgcctgctaacgagcttctagatgggagctggagaagtgcg 345
                                                    W  E  L  E  K  C
tcggtcagactggtggtgtcccaaggtaggacccccggataaaaacatgtgttcagttggctaaccgac 414
V  G  Q  T  G  G  V  P  R
gatcgctgtctagactgaacatcggtggcatgtgtcttcaggacagtcccttgggaattcgtgatagta 483
          L  N  I  G  G  M  C  L  Q  D  S  P  L  G  I  R  D
agtcttgatacaactggagctcggccgttgacactcttgctcacaatgtgttctgcaggtgactacaat 552
                                                          S  D  Y  N
tcggctttccctgctggtgtcaacgttgctgcgacatgggacaagaaccttgcttatctacgtggtcag 621
 S  A  F  P  A  G  V  N  V  A  A  T  W  D  K  N  L  A  Y  L  R  G  Q
gctatgggtcaagagttcagtgacaaaggaattgatgttcaattgggaccggccgcgggtcccctcggc 690
 A  M  G  Q  E  F  S  D  K  G  I  D  V  Q  L  G  P  A  A  G  P  L  G
aggagccctgatggaggtcgcaactgggaaggtttctctccagacccggctcttactggtgtgctcttt 759
 R  S  P  D  G  G  R  N  W  E  G  F  S  P  D  P  A  L  T  G  V  L  F
gcggagacgattaagggtattcaagacgctggtgtcgtggcgacagccaagcattacattctcaatgag 828
 A  E  T  I  K  G  I  Q  D  A  G  V  V  A  T  A  K  H  Y  I  L  N  E
caagagcatttccgccaggtcgcagaggctgcgggctacggattcaatatctccgacacgatcagctct 897
 Q  E  H  F  R  Q  V  A  E  A  A  G  Y  G  F  N  I  S  D  T  I  S  S
aacgttgatgacaagaccattcatgaaatgtacctctggcccttcgcggatgccgttcgcgccggcgtt 966
 N  V  D  D  K  T  I  H  E  M  Y  L  W  P  F  A  D  A  V  R  A  G  V
ggcgccatcatgtgttcctacaaccagatcaacaacagctacggttgccagaacagttacactctgaac 1035
 G  A  I  M  C  S  Y  N  Q  I  N  N  S  Y  G  C  Q  N  S  Y  T  L  N
aagcttctgaaggccgagctcggcttccagggctttgtgatgtctgactggggtgctcaccacagtggt 1104
 K  L  L  K  A  E  L  G  F  Q  G  F  V  M  S  D  W  G  A  H  H  S  G
gttggttctgctttggccggcttggatatgtcaatgcctggcgatatcaccttcgattctgccactagt 1173
 V  G  S  A  L  A  G  L  D  M  S  M  P  G  D  I  T  F  D  S  A  T  S
ttctggggtaccaacctgaccattgctgtgctcaacggtaccgtcccgcagtggcgcgttgacgacatg 1242
 F  W  G  T  N  L  T  I  A  V  L  N  G  T  V  P  Q  W  R  V  D  D  M
gctgtccgtatcatggctgcctactacaaggttggccgcgaccgcctgtaccagccgcctaacttcagc 1311
 A  V  R  I  M  A  A  Y  Y  K  V  G  R  D  R  L  Y  Q  P  P  N  F  S
tcctggactcgcgatgaatacggcttcaagtatttctacccccaggaagggccctatgagaaggtcaat 1380
 S  W  T  R  D  E  Y  G  F  K  Y  F  Y  P  Q  E  G  P  Y  E  K  V  N
cactttgtcaatgtgcagcgcaaccacagcgaggttattcgcaagttgggagcagacagtactgttcta 1449
 H  F  V  N  V  Q  R  N  H  S  E  V  I  R  K  L  G  A  D  S  T  V  L
ctgaagaacaacaatgccctgccgctgaccggaaaggagcgcaaagttgcgatcctgggtgaagatgct 1518
 L  K  N  N  N  A  L  P  L  T  G  K  E  R  K  V  A  I  L  G  E  D  A
ggatccaactcgtacggtgccaatggctgctctgaccgtggctgtgacaacggtactcttgctatggct 1587
 G  S  N  S  Y  G  A  N  G  C  S  D  R  G  C  D  N  G  T  L  A  M  A
```

# Fig. 1A

```
tggggtagcggcactgccgaattcccatatctcgtgacccctgagcaggctattcaagccgaggtgctc 1656
 W  G  S  G  T  A  E  F  P  Y  L  V  T  P  E  Q  A  I  Q  A  E  V  L
aagcataagggcagcgtctacgccatcacggacaactgggcgctgagccaggtggagaccctcgctaaa 1725
 K  H  K  G  S  V  Y  A  I  T  D  N  W  A  L  S  Q  V  E  T  L  A  K
caagccaggtaagttctgtcgtccatacattggaggtatatgttttcagtgaactgacaagtgtctccg 1794
 Q  A  S
tagtgtctctcttgtatttgtcaactcggacgcgggagagggctatatctccgtggacggaaacgaggg 1863
    V  S  L  V  F  V  N  S  D  A  G  E  G  Y  I  S  V  D  G  N  E  G
cgaccgcaacaacctcaccctctggaagaacggcgacaacctcatcaaggctgctgcaaacaactgcaa 1932
  D  R  N  N  L  T  L  W  K  N  G  D  N  L  I  K  A  A  A  N  N  C  N
caacaccatcgttgtcatccactccgttggacctgttttggttgacgagtggtatgaccaccccaacgt 2001
  N  T  I  V  V  I  H  S  V  G  P  V  L  V  D  E  W  Y  D  H  P  N  V
tactgccatcctctgggcgggcttgccaggccaggagtctggcaactccttggctgacgtgctctacgg 2070
  T  A  I  L  W  A  G  L  P  G  Q  E  S  G  N  S  L  A  D  V  L  Y  G
ccgcgtcaacccgggcgccaaatctccattcacctggggcaagacgagggaggcgtacggggattacct 2139
  R  V  N  P  G  A  K  S  P  F  T  W  G  K  T  R  E  A  Y  G  D  Y  L
tgtccgtgagctcaacaacggcaacggagctccccaagatgatttctcggaaggtgttttcattgacta 2208
  V  R  E  L  N  N  G  N  G  A  P  Q  D  D  F  S  E  G  V  F  I  D  Y
ccgcggattcgacaagcgcaatgagaccccgatctacgagttcggacatggtctgagctacaccacttt 2277
  R  G  F  D  K  R  N  E  T  P  I  Y  E  F  G  H  G  L  S  Y  T  T  F
caactactctggccttcacatccaggttctcaacgcttcctccaacgctcaagtagccactgagactgg 2346
  N  Y  S  G  L  H  I  Q  V  L  N  A  S  S  N  A  Q  V  A  T  E  T  G
cgccgctcccaccttcggacaagtcggcaatgcctctgactacgtgtaccctgagggattgaccagaat 2415
  A  A  P  T  F  G  Q  V  G  N  A  S  D  Y  V  Y  P  E  G  L  T  R  I
cagcaagttcatctatccctggcttaattccacagacctgaaggcctcatctggcgacccgtactatgg 2484
  S  K  F  I  Y  P  W  L  N  S  T  D  L  K  A  S  S  G  D  P  Y  Y  G
agtcgacaccgcggagcacgtgcccgagggtgctactgatggctctccgcagcccgttctgcctgccgg 2553
  V  D  T  A  E  H  V  P  E  G  A  T  D  G  S  P  Q  P  V  L  P  A  G
tggtggcttcggtggtaacccgcgcctctacgatgagttgatccgtgtttcggtgacagtcaagaacac 2622
  G  G  F  G  G  N  P  R  L  Y  D  E  L  I  R  V  S  V  T  V  K  N  T
tggtcgtgttgccggtgatgctgtgcctcaattggtaattagatcctcgtgcagtattggttccagatg 2691
  G  R  V  A  G  D  A  V  P  Q  L
ctaaccgcttgctagtatgtttcccttggtggacccaatgagcccaaggttgtgttgcgcaaattcgac 2760
                Y  V  S  L  G  G  P  N  E  P  K  V  V  L  R  K  F  D
cgcctcaccctcaagccctccgaggagacggtgtggacgactaccctgacccgccgcgatctgtctaac 2829
 R  L  T  L  K  P  S  E  E  T  V  W  T  T  T  L  T  R  R  D  L  S  N
tgggacgttgcggctcaggactgggtcatcacttcttacccgaagaaggtccatgttggtagctcttcg 2898
 W  D  V  A  A  Q  D  W  V  I  T  S  Y  P  K  K  V  H  V  G  S  S  S
cgtcagctgccccttcacgcggcgctcccgaaggtgcaataa 2940
 R  Q  L  P  L  H  A  A  L  P  K  V  Q  .
```

# Fig. 1B

```
atgagattcggttggctcgaggtggccgctctgacggccgcttctgtagccaatgcccaggtttgtgat 69
 M  R  F  G  W  L  E  V  A  A  L  T  A  A  S  V  A  N  A  Q
gctttcccgtcattgtttcggatatagttgacaatagtcatggaaataatcaggaattggctttctctc 138
                                                      E  L  A  F  S
caccattctacccttcgccttgggctgatggccagggagagtgggcagatgcccatcgacgcgccgtcg 207
P  P  F  Y  P  S  P  W  A  D  G  Q  G  E  W  A  D  A  H  R  R  A  V
agatcgtttctcagatgacactggcggagaaggttaaccttacaacgggtactgggtgggttgcgactt 276
E  I  V  S  Q  M  T  L  A  E  K  V  N  L  T  T  G  T  G
ttttgttgacagtgagctttcttcactgaccatctacacagatgggaaatggaccgatgcgtcggtcaa 345
                                        W  E  M  D  R  C  V  G  Q
accggcagcgttcccaggtaagcttgcaattctgcaacaacgtgcaagtgtagttgctaaaacgcggtg 414
 T  G  S  V  P  R
gtgcagacttggtatcaactggggtctttgtggccaggattcccctttgggtatccgtttctgtgagct 483
      L  G  I  N  W  G  L  C  G  Q  D  S  P  L  G  I  R  F
atacccgcggagtctttcagtccttgtattatgtgctgatgattgtctctgtatagctgacctcaactc 552
                                                        S  D  L  N  S
cgccttccctgctggtactaatgtcgccgcgacatgggacaagacactcgcctaccttcgtggcaaggc 621
  A  F  P  A  G  T  N  V  A  A  T  W  D  K  T  L  A  Y  L  R  G  K  A
catgggtgaggaattcaacgacaagggcgtggacattttgctggggcctgctgctggtcctctcggcaa 690
  M  G  E  E  F  N  D  K  G  V  D  I  L  L  G  P  A  A  G  P  L  G  K
atacccggacggcggcagaatctgggaaggcttctctcctgatccggttctcactggtgtacttttcgc 759
  Y  P  D  G  G  R  I  W  E  G  F  S  P  D  P  V  L  T  G  V  L  F  A
cgaaactatcaagggtatccaagacgcgggtgtgattgctactgccaagcattacattctgaatgaaca 828
  E  T  I  K  G  I  Q  D  A  G  V  I  A  T  A  K  H  Y  I  L  N  E  Q
ggagcatttccgacaggttggcgaggcccagggatatggttacaacatcacggagacgatcagctccaa 897
  E  H  F  R  Q  V  G  E  A  Q  G  Y  G  Y  N  I  T  E  T  I  S  S  N
cgtggatgacaagaccatgcacgagttgtacctttggtgagtagttgacactgcaaatgaggaccttga 966
  V  D  D  K  T  M  H  E  L  Y  L  W
ttgatttgactgacctggaatgcaggccctttgcagatgctgtgcgcggtaagatttccgtagacttg 1035
                              P  F  A  D  A  V  R
acctcgcgacgaagaaatcgctgacgaaccatcgtagctggcgttggcgctgtcatgtgttcctacaat 1104
                                         A  G  V  G  A  V  M  C  S  Y  N
caaatcaacaacagctacggttgtcaaaacagtcaaactctcaacaagctcctcaaggctgagctgggc 1173
 Q  I  N  N  S  Y  G  C  Q  N  S  Q  T  L  N  K  L  L  K  A  E  L  G
ttccaaggcttcgtcatgagtgactggagcgctcaccacagcggtgtcggcgctgccctcgctggttg 1242
 F  Q  G  F  V  M  S  D  W  S  A  H  H  S  G  V  G  A  A  L  A  G  L
gatatgtcgatgcctggagacatttccttcgacgacggactctccttctggggcacgaacctaactgtc 1311
 D  M  S  M  P  G  D  I  S  F  D  D  G  L  S  F  W  G  T  N  L  T  V
agtgttcttaacggcaccgttccagcctggcgtgtcgatgacatggctgttcgtatcatgaccgcgtac 1380
 S  V  L  N  G  T  V  P  A  W  R  V  D  D  M  A  V  R  I  M  T  A  Y
tacaaggttggtcgtgaccgtcttcgtattccccctaacttcagctcctggacccgggatgagtacggc 1449
 Y  K  V  G  R  D  R  L  R  I  P  P  N  F  S  S  W  T  R  D  E  Y  G
tgggagcattctgctgtctccgagggagcctggaccaaggtgaacgacttcgtcaatgtgcagcgcagt 1518
 W  E  H  S  A  V  S  E  G  A  W  T  K  V  N  D  F  V  N  V  Q  R  S
```

# Fig. 2A

```
cactctcagatcatccgtgagattggtgccgctagtacagtgctcttgaagaacacgggtgctcttcct 1587
 H  S  Q  I  I  R  E  I  G  A  A  S  T  V  L  L  K  N  T  G  A  L  P
ttgaccggcaaggaggttaaagtgggtgttctcggtgaagacgctggttccaacccgtggggtgctaac 1656
 L  T  G  K  E  V  K  V  G  V  L  G  E  D  A  G  S  N  P  W  G  A  N
ggctgccccgaccgcggctgtgataacggcactcttgctatggcctggggtagtggtactgccaacttc 1725
 G  C  P  D  R  G  C  D  N  G  T  L  A  M  A  W  G  S  G  T  A  N  F
ccttaccttgtcacccccgagcaggctatccagcgagaggtcatcagcaacggcggcaatgtctttgct 1794
 P  Y  L  V  T  P  E  Q  A  I  Q  R  E  V  I  S  N  G  G  N  V  F  A
gtgactgataacggggctctcagccagatggcagatgttgcatctcaatccaggtgagtgcgggctctt 1863
 V  T  D  N  G  A  L  S  Q  M  A  D  V  A  S  Q  S  S
agaaaaagaacgttctctgaatgaagtttttaaccattgcgaacagcgtgtctttggtgtttgtcaac 1932
                                                 V  S  L  V  F  V  N
gccgactctggagagggtttcatcagtgtcgacggcaacgagggtgaccgcaaaaatctcactctgtgg 2001
 A  D  S  G  E  G  F  I  S  V  D  G  N  E  G  D  R  K  N  L  T  L  W
aagaacggcgaggccgtcattgacactgttgtcagccactgcaacaacacgattgtggttattcacagt 2070
 K  N  G  E  A  V  I  D  T  V  V  S  H  C  N  N  T  I  V  V  I  H  S
gttgggcccgtcttgatcgaccggtggtatgataaccccaacgtcactgccatcatctgggccggcttg 2139
 V  G  P  V  L  I  D  R  W  Y  D  N  P  N  V  T  A  I  I  W  A  G  L
cccggtcaggagagtggcaactccctggtcgacgtgctctatggccgcgtcaaccccagcgccaagacc 2208
 P  G  Q  E  S  G  N  S  L  V  D  V  L  Y  G  R  V  N  P  S  A  K  T
ccgttcacctggggcaagactcgggagtcttacggggctcccttgctcaccgagcctaacaatggcaat 2277
 P  F  T  W  G  K  T  R  E  S  Y  G  A  P  L  L  T  E  P  N  N  G  N
ggtgctccccaggatgatttcaacgagggcgtcttcattgactaccgtcactttgacaagcgcaatgag 2346
 G  A  P  Q  D  D  F  N  E  G  V  F  I  D  Y  R  H  F  D  K  R  N  E
acccccatttatgagtttggccatggcttgagctacaccacctttggttactctcaccttcgggttcag 2415
 T  P  I  Y  E  F  G  H  G  L  S  Y  T  T  F  G  Y  S  H  L  R  V  Q
gccctcaatagttcgagttcggcatatgtcccgactagcggagagaccaagcctgcgccaacctatggt 2484
 A  L  N  S  S  S  S  A  Y  V  P  T  S  G  E  T  K  P  A  P  T  Y  G
gagatcggtagtgccgccgactacctgtatcccgagggtctcaaaagaattaccaagtttatttaccct 2553
 E  I  G  S  A  A  D  Y  L  Y  P  E  G  L  K  R  I  T  K  F  I  Y  P
tggctcaactcgaccgacctcgaggattcttctgacgacccgaactacggctgggaggactcggagtac 2622
 W  L  N  S  T  D  L  E  D  S  S  D  D  P  N  Y  G  W  E  D  S  E  Y
attcccgaaggcgctagggatgggtctcctcaacccctcctgaaggctggcggcgctcctggtggtaac 2691
 I  P  E  G  A  R  D  G  S  P  Q  P  L  L  K  A  G  G  A  P  G  G  N
cctaccctttatcaggatcttgttagggtgtcggccaccataaccaacactggtaacgtcgccggttat 2760
 P  T  L  Y  Q  D  L  V  R  V  S  A  T  I  T  N  T  G  N  V  A  G  Y
gaagtccctcaattggtgagtgacccgcatgttccttgcgttgcaatttggctaactcgcttctagtat 2829
 E  V  P  Q  L                                                     Y
gtttcattgggcggaccgaacgagcctcgggtcgttctgcgcaagttcgaccgaatcttcctggctcct 2898
 V  S  L  G  G  P  N  E  P  R  V  V  L  R  K  F  D  R  I  F  L  A  P
ggggagcaaaaggtttggaccacgactcttaaccgtcgtgatctcgccaattgggatgtggaggctcag 2967
 G  E  Q  K  V  W  T  T  T  L  N  R  R  D  L  A  N  W  D  V  E  A  Q
gactgggtcatcacaaagtaccccaagaaagtgcacgtcggcagctcctcgcgtaagctgcctctgaga 3036
 D  W  V  I  T  K  Y  P  K  K  V  H  V  G  S  S  S  R  K  L  P  L  R
gcgcctctgccccgtgtctactag 3060
 A  P  L  P  R  V  Y  .
```

# Fig. 2B

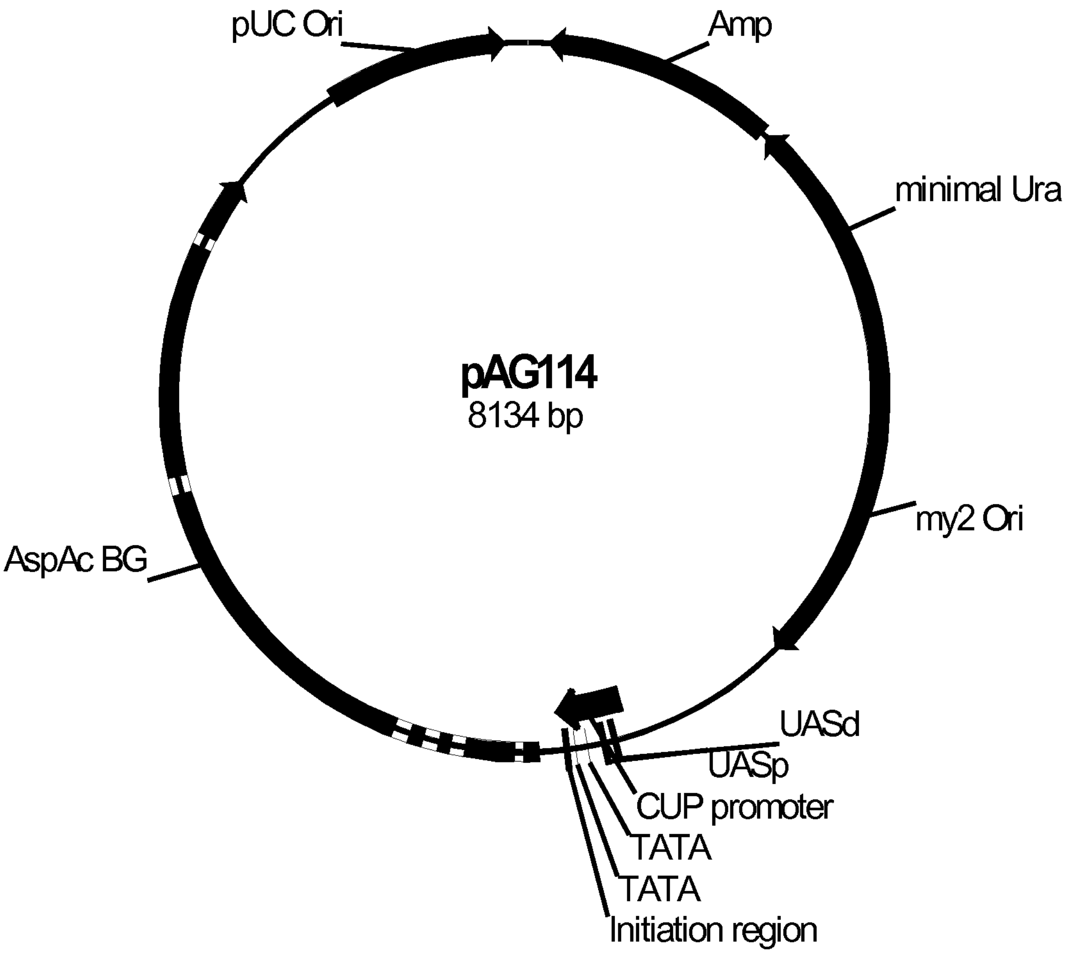
pUC Ori
Amp
minimal Ura
pAG114
8134 bp
my2 Ori
AspAc BG
UASd
UASp
CUP promoter
TATA
TATA
Initiation region

Fig. 3

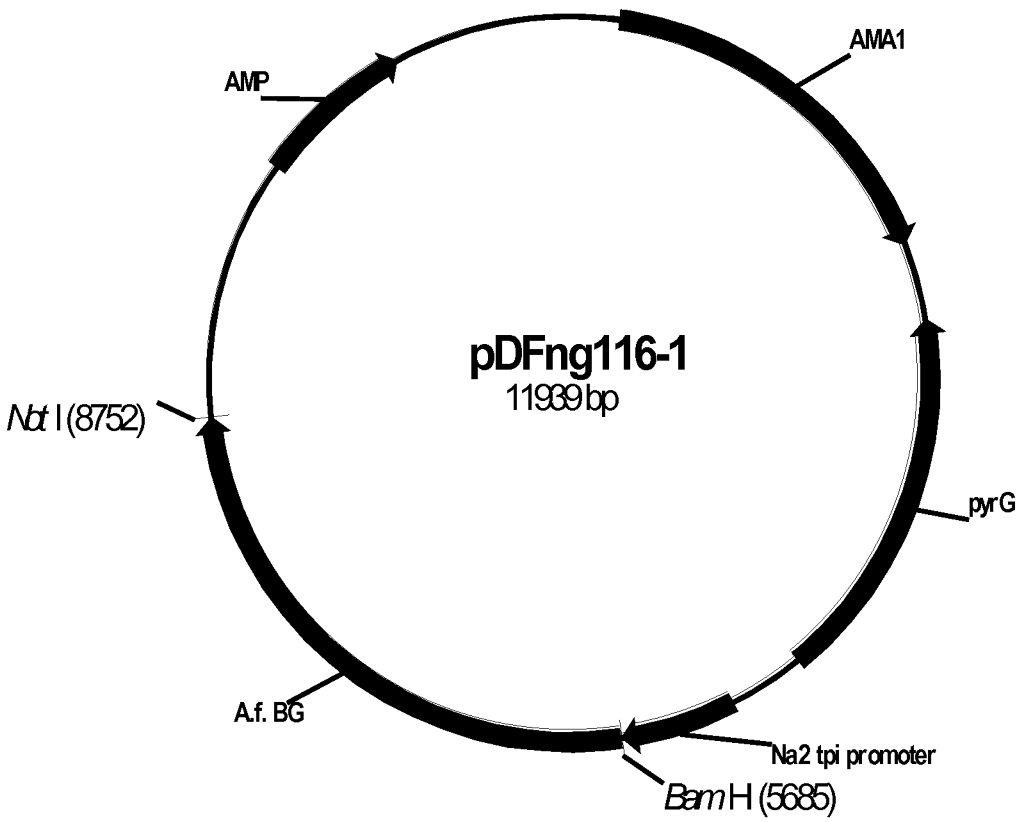
pDFng116-1
11939 bp
AMA1
AMP
pyrG
Not I (8752)
A.f. BG
Na2 tpi promoter
Bam H (5685)

Fig. 4

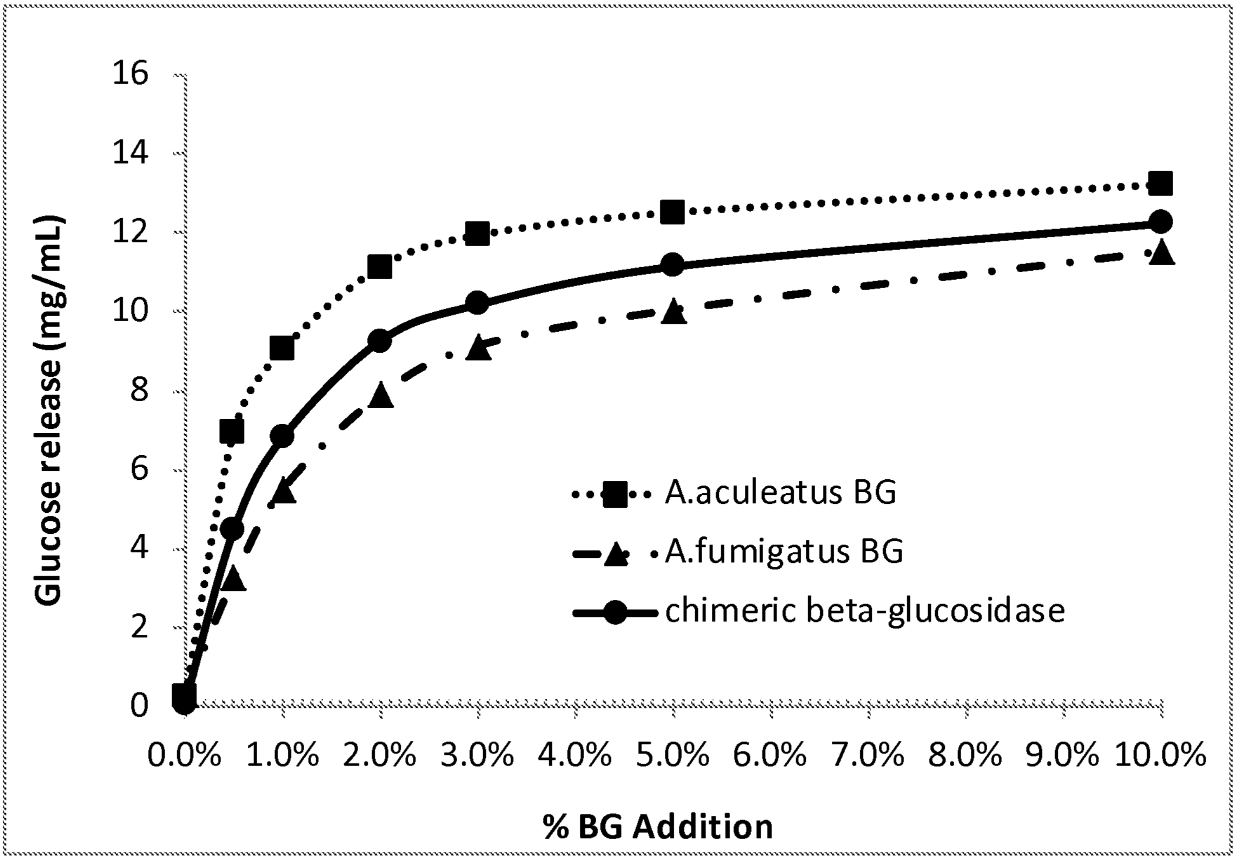
16
14
12
10
8
6
4
2
0
Glucose release (mg/mL)
0.0% 1.0% 2.0% 3.0% 4.0% 5.0% 6.0% 7.0% 8.0% 9.0% 10.0%
% BG Addition
A.aculeatus BG
A.fumigatus BG
chimeric beta-glucosidase

Fig. 5

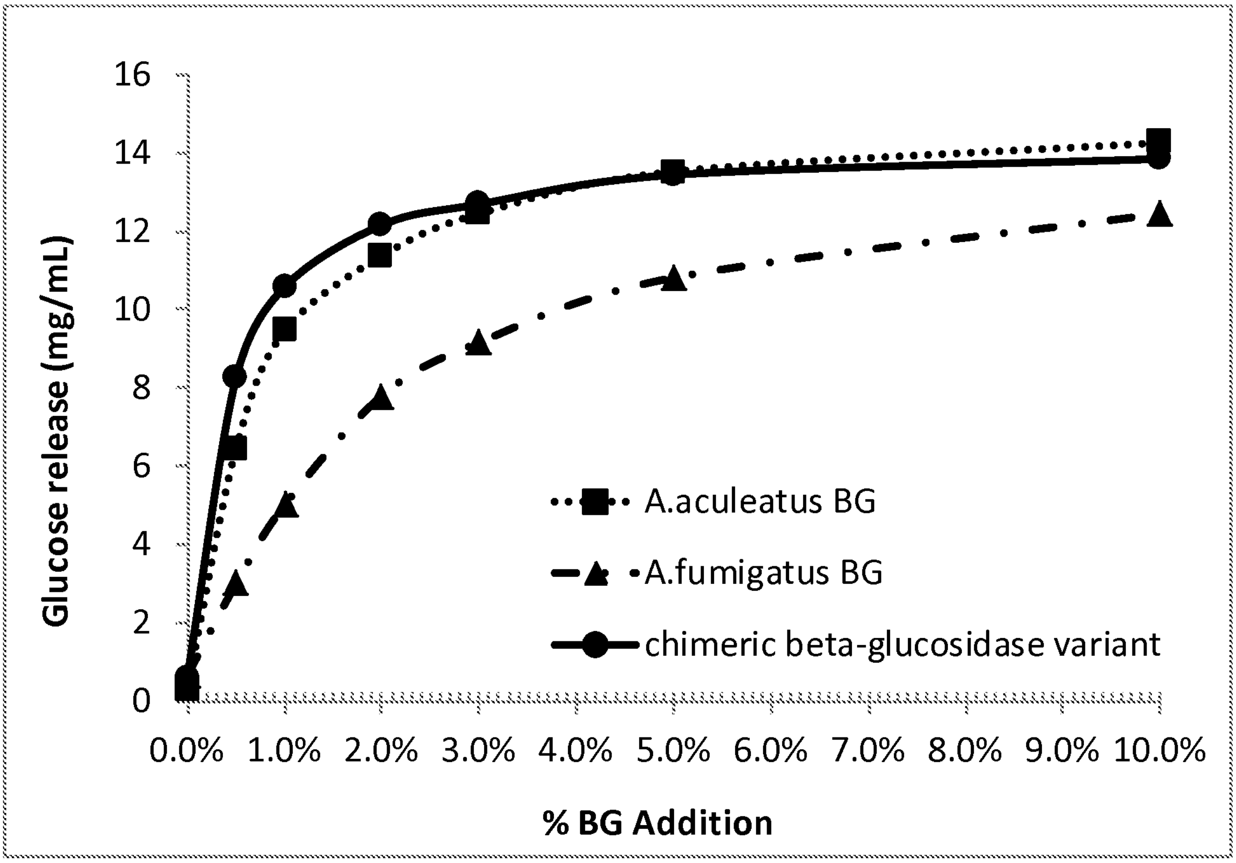
16
14
12
10
8
6
4
2
0
Glucose release (mg/mL)
0.0% 1.0% 2.0% 3.0% 4.0% 5.0% 6.0% 7.0% 8.0% 9.0% 10.0%
% BG Addition
A.aculeatus BG
A.fumigatus BG
chimeric beta-glucosidase variant

Fig. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 7244605 B
- WO 02095014 A
- WO 2002095014 A
- WO 9943835 A
- WO 9600787 A
- WO 0056900 A
- US 6011147 A
- WO 9425612 A
- WO 9533836 A
- WO 2010039889 A
- WO 0024883 A
- EP 238023 A
- WO 9015861 A
- WO 2010096673 A
- US 20020164730 A
- WO 2006110891 A
- WO 2006110899 A
- WO 2006110900 A
- WO 2006110901 A
- WO 2006032282 A
- WO 9117243 A
- WO 9117244 A
- WO 9105039 A
- WO 9315186 A
- US 5275944 A
- WO 9602551 A
- US 5536655 A
- WO 0070031 A
- WO 05093050 A
- WO 2011059740 A
- WO 2009042871 A
- WO 2010141325 A
- WO 2006074435 A
- WO 2010057086 A
- WO 2005047499 A
- WO 2007019442 A
- WO 2010088387 A
- WO 2011035029 A
- WO 2008057637 A
- WO 9813465 A
- WO 98015619 A
- WO 98015633 A
- WO 9906574 A
- WO 9910481 A
- WO 99025847 A
- WO 99031255 A
- WO 2002101078 A
- WO 2003027306 A
- WO 2003052054 A
- WO 2003052055 A
- WO 2003052056 A
- WO 2003052057 A
- WO 2003052118 A
- WO 2004016760 A
- WO 2004043980 A
- WO 2004048592 A
- WO 2005001065 A
- WO 2005028636 A
- WO 2005093050 A
- WO 2005093073 A
- WO 2006074005 A
- WO 2006117432 A
- WO 2007071818 A
- WO 2007071820 A
- WO 2008008070 A
- WO 2008008793 A
- US 5457046 A
- US 5648263 A
- US 5686593 A
- WO 2005074647 A
- WO 2008148131 A
- WO 2011035027 A
- WO 2005074656 A
- WO 2010065830 A
- WO 2007089290 A
- WO 2009085935 A
- WO 2009085859 A
- WO 2009085864 A
- WO 2009085868 A
- WO 2010138754 A
- WO 2011005867 A
- WO 2011039319 A
- WO 2011041397 A
- WO 2011041504 A
- WO 2008151043 A
- WO 9421785 A
- WO 2006078256 A
- WO 2011041405 A
- WO 2010126772 A
- WO 2009079210 A
- WO 2011057083 A
- WO 2010108918 A
- WO 2009073709 A
- WO 2005001036 A
- WO 2010014880 A
- WO 2009042846 A
- WO 2009076122 A
- WO 2009127729 A

- WO 2010053838 A
- WO 2010065448 A
- WO 2006114094 A
- WO 2009073383 A
- WO 2010014706 A
- WO 2009068565 A
- WO 2003062430 A
- WO 9114772 A
- US 6395966 B
- US 7151204 B
- WO 9961651 A
- WO 2011057140 A
- WO 2004099228 A
- US 61541456 B


**Non-patent literature cited in the description**


- **KAWAGUCHI et al.** *Gene,* 1996, vol. 173, 287-288
- **VRIES.** *J. Bacteriol.,* 1998, vol. 180, 243-249
- **VENTURI et al.** Extracellular beta-D-glucosidase from Chaetomium thermophilum var. coprophilum: production, purification and some biochemical properties. *J. Basic Microbiol.,* 2002, vol. 42, 55-66
- **TEERI.** Crystalline cellulose degradation: New insight into the function of cellobiohydrolases. *Trends in Biotechnology,* 1997, vol. 15, 160-167
- **TEERI et al.** Trichoderma reesei cellobiohydrolases: why so efficient on crystalline cellulose?. *Biochem. Soc. Trans.,* 1998, vol. 26, 173-178
- **LEVER et al.** *Anal. Biochem.,* 1972, vol. 47, 273-279
- **VAN TILBEURGH et al.** *FEBS Letters,* 1982, vol. 149, 152-156
- **VAN TILBEURGH ; CLAEYSSENS.** *FEBS Letters,* 1985, vol. 187, 283-288
- **TOMME et al.** *Eur. J. Biochem.,* 1988, vol. 170, 575-581
- **ZHANG et al.** Outlook for cellulase improvement: Screening and selection strategies. *Biotechnology Advances,* 2006, vol. 24, 452-481
- **GHOSE.** Measurement of cellulase activities. *Pure Appl. Chem.,* 1987, vol. 59, 257-68
- **WISELOGEL et al.** Handbook on Bioethanol. Taylor & Francis, 1995, 105-118
- **WYMAN.** *Bioresource Technology,* 1994, vol. 50, 3-16
- **LYND.** *Applied Biochemistry and Biotechnology,* 1990, vol. 24/25, 695-719
- Recent Progress in Bioconversion of Lignocellulosics. **MOSIER et al.** Advances in Biochemical Engineering/Biotechnology,. Springer-Verlag, 1999, vol. 65, 23-40
- **ZHANG et al.** *Biotechnology Advances,* 2006, vol. 24, 452-481
- **GHOSE.** *Pure and Appl. Chem.,* 1987, vol. 59, 257-268
- Recent Advances in Carbohydrate Bioengineering. The Royal Society of Chemistry, 3-12
- **HENRISSAT B.** A classification of glycosyl hydrolases based on amino-acid sequence similarities. *Biochem. J.,* 1991, vol. 280, 309-316
- **HENRISSAT B. ; BAIROCH A.** Updating the sequence-based classification of glycosyl hydrolases. *Biochem. J.,* 1996, vol. 316, 695-696
- **SHALLOM, D. ; SHOHAM, Y.** Microbial hemicellulases. *Current Opinion In Microbiology,* 2003, vol. 6 (3), 219-228
- **BISARIA.** *Pure & Appl. Chem.,* 1987, vol. 59, 1739-1752
- **NIELSEN et al.** *Protein Engineering,* 1997, vol. 10, 1-6
- **NEEDLEMAN ; WUNSCH.** *J. Mol. Biol.,* 1970, vol. 48, 443-453
- **RICE et al.** EMBOSS: The European Molecular Biology Open Software Suite. *Trends Genet.,* 2000, vol. 16, 276-277
- **RICE et al.** *EMBOSS: The European Molecular Biology Open Software Suite,* 2000
- **EBRINGEROVA et al.** *Adv. Polym. Sci.,* 2005, vol. 186, 1-67
- **BIELY ; PUCHARD.** Recent progress in the assays of xylanolytic enzymes. *Journal of the Science of Food and Agriculture,* 2006, vol. 86 (11), 1636-1647
- **SPANIKOVA ; BIELY.** Glucuronoyl esterase - Novel carbohydrate esterase produced by Schizophyllum commune. *FEBS Letters,* 2006, vol. 580 (19), 4597-4601
- **HERRMANN ; VRSANSKA ; JURICKOVA ; HIRSCH ; BIELY ; KUBICEK.** The beta-D-xylosidase of Trichoderma reesei is a multifunctional beta-D-xylan xylohydrolase. *Biochemical Journal,* 1997, vol. 321, 375-381
- **BAILEY ; BIELY ; POUTANEN.** Interlaboratory testing of methods for assay of xylanase activity. *Journal of Biotechnology,* 1992, vol. 23 (3), 257-270
- **LEVER.** A new reaction for colorimetric determination of carbohydrates. *Anal. Biochem,* 1972, vol. 47, 273-279
- **H. NEURATH ; R.L. HILL.** In, The Proteins. Academic Press, 1979
- **CUNNINGHAM ; WELLS.** *Science,* 1989, vol. 244, 1081-1085
- **HILTON et al.** *J. Biol. Chem.,* 1996, vol. 271, 4699-4708
- **VOS et al.** *Science,* 1992, vol. 255, 306-312
- **SMITH et al.** *J. Mol. Biol,* 1992, vol. 224, 899-904

- **WLODAVER et al.** *FEBS Lett.,* 1992, vol. 309, 59-64
- **AGAISSE ; LERECLUS.** *Molecular Microbiology,* 1994, vol. 13, 97-107
- **EGON et al.** *Gene,* 1988, vol. 69, 301-315
- **VILLA-KAMAROFF et al.** *Proc. Natl. Acad. Sci. USA,* 1978, vol. 75, 3727-3731
- **DEBOER et al.** *Proc. Natl. Acad. Sci. USA,* 1983, vol. 80, 21-25
- **GILBERT et al.** Useful proteins from recombinant bacteria. *Scientific American,* 1980, vol. 242, 74-94
- **ROMANOS et al.** *Yeast,* 1992, vol. 8, 423-488
- **HUE et al.** *Journal of Bacteriology,* 1995, vol. 177, 3465-3471
- **SHERMAN.** *Mol. Cellular Biol.,* 1995, vol. 15, 5983-5990
- **SIMONEN ; PALVA.** *Microbiological Reviews,* 1993, vol. 57, 109-137
- **GEMS et al.** *Gene,* 1991, vol. 98, 61-67
- **CULLEN et al.** *Nucleic Acids Res.,* 1987, vol. 15, 9163-9175
- **CHANG ; COHEN.** *Mol. Gen. Genet.,* 1979, vol. 168, 111-115
- **YOUNG ; SPIZIZEN.** *J. Bacteriol.,* 1961, vol. 81, 823-829
- **DUBNAU ; DAVIDOFF-ABELSON.** *J. Mol. Biol.,* 1971, vol. 56, 209-221
- **SHIGEKAWA ; DOWER.** *Biotechniques,* 1988, vol. 6, 742-751
- **KOEHLER ; THORNE.** *J. Bacteriol.,* 1987, vol. 169, 5271-5278
- **HANAHAN.** *J. Mol. Biol.,* 1983, vol. 166, 557-580
- **DOWER et al.** *Nucleic Acids Res.,* 1988, vol. 16, 6127-6145
- **GONG et al.** *Folia Microbiol.,* 2004, vol. 49, 399-405
- **MAZODIER et al.** *J. Bacteriol.,* 1989, vol. 171, 3583-3585
- **BURKE et al.** *Proc. Natl. Acad. Sci. USA,* 2001, vol. 98, 6289-6294
- **CHOI et al.** *J. Microbiol. Methods,* 2006, vol. 64, 391-397
- **SMETS.** *Appl. Environ. Microbiol.,* 2005, vol. 71, 51-57
- **PERRY ; KURAMITSU.** *Infect. Immun.,* 1981, vol. 32, 1295-1297
- **CATT ; JOLLICK.** *Microbios,* 1991, vol. 68, 189-207
- **BUCKLEY et al.** *Appl. Environ. Microbiol.,* 1999, vol. 65, 3800-3804
- **CLEWELL.** *Microbiol. Rev.,* 1981, vol. 45, 409-436
- Ainsworth and Bisby's Dictionary of The Fungi. **HAWKSWORTH et al.** CAB International. University Press, 1995
- Biology and Activities of Yeast. Soc. App. Bacteriol. Symposium Series. 1980
- **YELTON et al.** *Proc. Natl. Acad. Sci. USA,* 1984, vol. 81, 1470-1474
- **CHRISTENSEN et al.** *Bio/Technology,* 1988, vol. 6, 1419-1422
- **MALARDIER et al.** *Gene,* 1989, vol. 78, 147-156
- Guide to Yeast Genetics and Molecular Biology. **BECKER ; GUARENTE.** Methods in Enzymology. Academic Press, Inc, vol. 194, 182-187
- **ITO et al.** *J. Bacteriol.,* 1983, vol. 153, 163
- **HINNEN et al.** *Proc. Natl. Acad. Sci. USA,* 1978, vol. 75, 1920
- Protein Purification. VCH Publishers, 1989
- Cellulose bioconversion technology. **PHILIPPIDIS, G. P.** Handbook on Bioethanol: Production and Utilization. Taylor & Francis, 1996, 179-212
- **SHEEHAN, J. ; HIMMEL, M.** Enzymes, energy and the environment: A strategic perspective on the U.S. Department of Energy's research and development activities for bioethanol. *Biotechnol. Prog.,* 1999, vol. 15, 817-827
- **LYND et al.** Microbial cellulose utilization: Fundamentals and biotechnology. *Microbiol. Mol. Biol. Reviews,* 2002, vol. 66, 506-577
- **FERNANDA DE CASTILHOS CORAZZA ; FLÁVIO FARIA DE MORAES ; GISELLA MARIA ZANIN ; IVO NEITZEL.** Optimal control in fed-batch reactor for the cellobiose hydrolysis. *Acta Scientiarum. Technology,* 2003, vol. 25, 33-38
- **GUSAKOV, A. V. ; SINITSYN, A. P.** Kinetics of the enzymatic hydrolysis of cellulose: 1. A mathematical model for a batch reactor process. *Enz. Microb. Technol.,* 1985, vol. 7, 346-352
- **RYU ; LEE.** Bioconversion of waste cellulose by using an attrition bioreactor. *Biotechnol. Bioeng.,* 1983, vol. 25, 53-65
- **GUSAKOV et al.** Enhancement of enzymatic cellulose hydrolysis using a novel type of bioreactor with intensive stirring induced by electromagnetic field. *Appl. Biochem. Biotechnol.,* 1996, vol. 56, 141-153
- **CHANDRA et al.** Substrate pretreatment: The key to effective enzymatic hydrolysis of lignocellulosics?. *Adv. Biochem. Engin./Biotechnol.,* 2007, vol. 108, 67-93
- **GALBE ; ZACCHI.** Pretreatment of lignocellulosic materials for efficient bioethanol production. *Adv. Biochem. Engin./Biotechnol.,* 2007, vol. 108, 41-65
- **HENDRIKS ; ZEEMAN.** Pretreatments to enhance the digestibility of lignocellulosic biomass. *Bioresource Technol.,* 2009, vol. 100, 10-18
- **MOSIER et al.** Features of promising technologies for pretreatment of lignocellulosic biomass. *Bioresource Technol.,* 2005, vol. 96, 673-686

- **TAHERZADEH ; KARIMI.** Pretreatment of lignocellulosic wastes to improve ethanol and biogas production: A review. *Int. J. of Mol. Sci.,* 2008, vol. 9, 1621-1651
- **YANG ; WYMAN.** Pretreatment: the key to unlocking low-cost cellulosic ethanol. *Biofuels Bioproducts and Biorefining-Biofpr.,* 2008, vol. 2, 26-40
- **DUFF ; MURRAY.** *Bioresource Technology,* 1996, vol. 855, 1-33
- **GALBE ; ZACCHI.** *Appl. Microbiol. Biotechnol.,* 2002, vol. 59, 618-628
- **BALLESTEROS et al.** *Appl. Biochem. Biotechnol.,* 2006, vol. 129-132, 496-508
- **VARGA et al.** *Appl. Biochem. Biotechnol.,* 2004, vol. 113-116, 509-523
- **SASSNER et al.** *Enzyme Microb. Technol.,* 2006, vol. 39, 756-762
- **SCHELL et al.** *Bioresource Technol.,* 2004, vol. 91, 179-188
- **LEE et al.** *Adv. Biochem. Eng. Biotechnol.,* 1999, vol. 65, 93-115
- **WYMAN et al.** *Bioresource Technol.,* 2005, vol. 96, 1959-1966
- **MOSIER et al.** *Bioresource Technol.,* 2005, vol. 96, 673-686
- **SCHMIDT ; THOMSEN.** *Bioresource Technol.,* 1998, vol. 64, 139-151
- **PALONEN et al.** *Appl. Biochem. Biotechnol.,* 2004, vol. 117, 1-17
- **VARGA et al.** *Biotechnol. Bioeng.,* 2004, vol. 88, 567-574
- **MARTIN et al.** *J. Chem. Technol. Biotechnol.,* 2006, vol. 81, 1669-1677
- **GOLLAPALLI et al.** *Appl. Biochem. Biotechnol.,* 2002, vol. 98, 23-35
- **CHUNDAWAT et al.** *Biotechnol. Bioeng.,* 2007, vol. 96, 219-231
- **ALIZADEH et al.** *Appl. Biochem. Biotechnol.,* 2005, vol. 121, 1133-1141
- **TEYMOURI et al.** *Bioresource Technol.,* 2005, vol. 96, 2014-2018
- **PAN et al.** *Biotechnol. Bioeng.,* 2005, vol. 90, 473-481
- **PAN et al.** *Biotechnol. Bioeng.,* 2006, vol. 94, 851-861
- **KURABI et al.** *Appl. Biochem. Biotechnol.,* 2005, vol. 121, 219-230
- **SCHELL et al.** *Appl. Biochem. and Biotechnol.,* 2003, vol. 105 (108), 69-85
- **MOSIER et al.** *Bioresource Technology,* 2005, vol. 96, 673-686
- Pretreatment of biomass. **HSU, T.-A.** Handbook on Bioethanol: Production and Utilization. Taylor & Francis, 1996, 179-212
- Physicochemical and biological treatments for enzymatic/microbial conversion of cellulosic biomass. **GHOSH ; SINGH.** Adv. Appl. Microbiol. 1993, vol. 39, 295-333
- Pretreating lignocellulosic biomass: a review. **MCMILLAN, J. D.** Enzymatic Conversion of Biomass for Fuels Production. American Chemical Society, 1994
- Ethanol production from renewable resources. **GONG, C. S. ; CAO, N. J. ; DU, J. ; TSAO, G. T.** Advances in Biochemical Engineering/Biotechnology. Springer-Verlag Berlin Heidelberg, 1999, vol. 65, 207-241
- **OLSSON ; HAHN-HAGERDAL.** Fermentation of lignocellulosic hydrolysates for ethanol production. *Enz. Microb. Tech.,* 1996, vol. 18, 312-331
- **VALLANDER ; ERIKSSON.** Production of ethanol from lignocellulosic materials: State of the art. *Adv. Biochem. Eng./Biotechnol.,* 1990, vol. 42, 63-95
- **PENTTILA et al.** *Gene,* 1986, vol. 45, 253-263
- **SALOHEIMO et al.** *Gene,* 1988, vol. 63, 11-22
- **OKADA et al.** *Appl. Environ. Microbiol.,* 1988, vol. 64, 555-563
- **SALOHEIMO et al.** *Molecular Microbiology,* 1994, vol. 13, 219-228
- **OOI et al.** *Nucleic Acids Research,* vol. 18, 5884
- **SAKAMOTO et al.** *Current Genetics,* 1995, vol. 27, 435-439
- **SAARILAHTI et al.** *Gene,* 1990, vol. 90, 9-14
- **DAN et al.** *J. Biol. Chem.,* 2000, vol. 275, 4973-4980
- More Gene Manipulations in Fungi. Academic Press, 1991
- **BAILEY, J.E. ; OLLIS, D.F.** Biochemical Engineering Fundamentals. McGraw-Hill Book Company, 1986
- **LIN et al.** *Appl. Microbiol. Biotechnol.,* 2006, vol. 69, 627-642
- **CHEN ; HO.** Cloning and improving the expression of Pichia stipitis xylose reductase gene in Saccharomyces cerevisiae. *Appl. Biochem. Biotechnol.,* 1993, vol. 39-40, 135-147
- **HO et al.** Genetically engineered Saccharomyces yeast capable of effectively cofermenting glucose and xylose. *Appl. Environ. Microbiol.,* 1998, vol. 64, 1852-1859
- **KOTTER ; CIRIACY.** Xylose fermentation by Saccharomyces cerevisiae. *Appl. Microbiol. Biotechnol.,* 1993, vol. 38, 776-783
- **WALFRIDSSON et al.** Xylose-metabolizing Saccharomyces cerevisiae strains overexpressing the TKL1 and TAL1 genes encoding the pentose phosphate pathway enzymes transketolase and transaldolase. *Appl. Environ. Microbiol.,* 1995, vol. 61, 4184-4190
- **KUYPER et al.** Minimal metabolic engineering of Saccharomyces cerevisiae for efficient anaerobic xylose fermentation: a proof principle. *FEMS Yeast Research,* 2004, vol. 4, 655-664

- **BEALL et al.** Parametric studies of ethanol production from xylose and other sugars by recombinant Escherichia coli. *Biotech. Bioeng.,* 1991, vol. 38, 296-303
- **INGRAM et al.** Metabolic engineering of bacteria for ethanol production. *Biotechnol. Bioeng.,* 1998, vol. 58, 204-214
- **ZHANG et al.** Metabolic engineering of a pentose metabolism pathway in ethanologenic Zymomonas mobilis. *Science,* 1995, vol. 267, 240-243
- **DEANDA et al.** Development of an arabinose-fermenting Zymomonas mobilis strain by metabolic pathway engineering. *Appl. Environ. Microbiol.,* 1996, vol. 62, 4465-4470
- The Alcohol Textbook. Nottingham University Press, 1999
- **ALFENORE et al.** Improving ethanol production and viability of Saccharomyces cerevisiae by a vitamin feeding strategy during fed-batch process. Springer-Verlag, 2002
- **GONG et al.** Ethanol production from renewable resources, in Advances in Biochemical Engineering/Biotechnology. Springer-Verlag Berlin Heidelberg, 1999, vol. 65, 207-241
- **SILVEIRA ; JONAS.** The biotechnological production of sorbitol. *Appl. Microbiol. Biotechnol.,* 2002, vol. 59, 400-408
- **NIGAM ; SINGH.** Processes for fermentative production of xylitol - a sugar substitute. *Process Biochemistry,* 1995, vol. 30 (2), 117-124
- **EZEJI et al.** Production of acetone, butanol and ethanol by Clostridium beijerinckii BA101 and in situ recovery by gas stripping. *World Journal of Microbiology and Biotechnology,* 2003, vol. 19 (6), 595-603
- **RICHARD ; MARGARITIS.** Empirical modeling of batch fermentation kinetics for poly(glutamic acid) production and other microbial biopolymers. *Biotechnology and Bioengineering,* 2004, vol. 87 (4), 501-515
- **KATAOKA et al.** Studies on hydrogen production by continuous culture system of hydrogen-producing anaerobic bacteria. *Water Science and Technology,* 1997, vol. 36 (6-7), 41-47
- **GUNASEELAN.** *Biomass and Bioenerg,* 1997, vol. 13 (1-2), 83-114
- **CHEN ; LEE.** Membrane-mediated extractive fermentation for lactic acid production from cellulosic biomass. *Appl. Biochem. Biotechnol.,* 1997, vol. 63-65, 435-448
- **TAGUE et al.** *Plant Physiol.,* 1988, vol. 86, 506
- **FRANCK et al.** *Cell,* 1980, vol. 21, 285-294
- **CHRISTENSEN et al.** *Plant Mol. Biol.,* 1992, vol. 18, 675-689
- **ZHANG et al.** *Plant Cell,* 1991, vol. 3, 1155-1165
- **EDWARDS ; CORUZZI.** *Ann. Rev. Genet.,* 1990, vol. 24, 275-303
- **ITO et al.** *Plant Mol. Biol.,* 1994, vol. 24, 863-878
- **WU et al.** *Plant Cell Physiol.,* 1998, vol. 39, 885-889
- **CONRAD et al.** *J. Plant Physiol.,* 1998, vol. 152, 708-711
- **CHEN et al.** *Plant Cell Physiol.,* 1998, vol. 39, 935-941
- **KYOZUKA et al.** *Plant Physiol.,* 1993, vol. 102, 991-1000
- **MITRA ; HIGGINS.** *Plant Mol. Biol.,* 1994, vol. 26, 85-93
- **KAGAYA et al.** *Mol. Gen. Genet.,* 1995, vol. 248, 668-674
- **XU et al.** *Plant Mol. Biol.,* 1993, vol. 22, 573-588
- **GASSER et al.** *Science,* 1990, vol. 244, 1293
- **POTRYKUS.** *Bio/Technology,* 1990, vol. 8, 535
- **SHIMAMOTO et al.** *Nature,* 1989, vol. 338, 274
- **HOOYKAS ; SCHILPEROORT.** *Plant Mol. Biol.,* 1992, vol. 19, 15-38
- **CHRISTOU.** *Plant J.,* 1992, vol. 2, 275-281
- **SHIMAMOTO.** *Curr. Opin. Biotechnol.,* 1994, vol. 5, 158-162
- **VASIL et al.** *Bio/Technology,* 1992, vol. 10, 667-674
- **OMIRULLEH et al.** *Plant Mol. Biol.,* 1993, vol. 21, 415-428
- **ZHU et al.** *BioTechniques,* 2007, vol. 43, 354-359